# FASCICULE DE BREVET EUROPEEN

(11) **EP 0 850 638 B1**
(45) Date de publication et mention de la délivrance du brevet: **01.09.1999**
(21) Numéro de dépôt: 97402863.1
(22) Date de dépôt: 27.11.1997
(51) Int. Cl.: A61K 7/13

(54) **Composition de teinture d'oxydation des fibres kératiniques et procédé de teinture mettant en oeuvre cette composition**
Zusammensetzung zur Oxidationsfärbung von keratinischen Fasern und Färbeverfahren mit dieser Zusammensetzung
Oxidative dye composition for keratinic fibres and dyeing process using this composition

(30) Priorité: 23.12.1996 FR 9615895
(43) Date de publication de la demande: 01.07.1998
(73) Titulaire: L'OREAL, 75008 Paris (FR)
(72) Inventeur: Rondeau, Christine, 78500 Sartrouville (FR); Cotteret, Jean, 78480 Verneuil Sur Seine (FR); De La Mettrie, Roland, 78110 Le Vesinet (FR)
(74) Mandataire: Miszputen, Laurent

(56) Documents cités:
- EP-A- 0 739 622
- US-A- 4 025 301

## Description

La présente invention a pour objet une composition prête à l'emploi pour la teinture d'oxydation des fibres kératiniques, en particulier des fibres kératiniques humaines telles que les cheveux, comprenant, dans un milieu approprié pour la teinture, au moins une base d'oxydation en association avec au moins un colorant direct cationique sélectionné et au moins un agent oxydant, ainsi que le procédé de teinture mettant en oeuvre cette composition. Elle concerne également un kit de coloration pour la préparation d'une telle composition prête à l'emploi.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant. Il a déjà été proposé, notamment dans le brevet US 4,025,301, des compositions pour la teinture renfermant un colorant direct cationique pyridinique en association avec un colorant d'oxydation et un agent oxydant.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La présente invention vise à proposer de nouvelles compositions pour la coloration d'oxydation des fibres kératiniques et en particulier des fibres kératiniques humaines telles que les cheveux qui permettent d'aboutir à des colorations riches en reflets tout en présentant de bonne propriétés de ténacité.

Ainsi, la demanderesse vient en effet de découvrir qu'il est possible d'obtenir de nouvelles teintures à la fois riches en reflets et tenaces en associant :
- au moins une base d'oxydation,
- au moins un colorant direct cationique de formules (I) et/ou (II) et/ou (III) et/ou (III') ci-après définies, et
- au moins un agent oxydant.

L'invention a donc pour premier objet une composition prête à l'emploi pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation
- au moins un colorant direct cationique choisi parmi :
   a) les composés de formule (I) suivante : 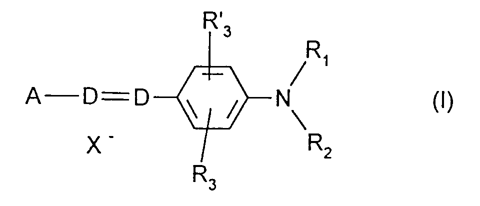 dans laquelle :
      - D représente un atome d'azote ou le groupement -CH,
      - R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      - R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
      - X ⁻ représente un anion de préférence choisi parmi le chlorure, le méthyl sulfate et l'acétate,
      - A représente un groupement choisi par les structures A1 à A19 suivantes : 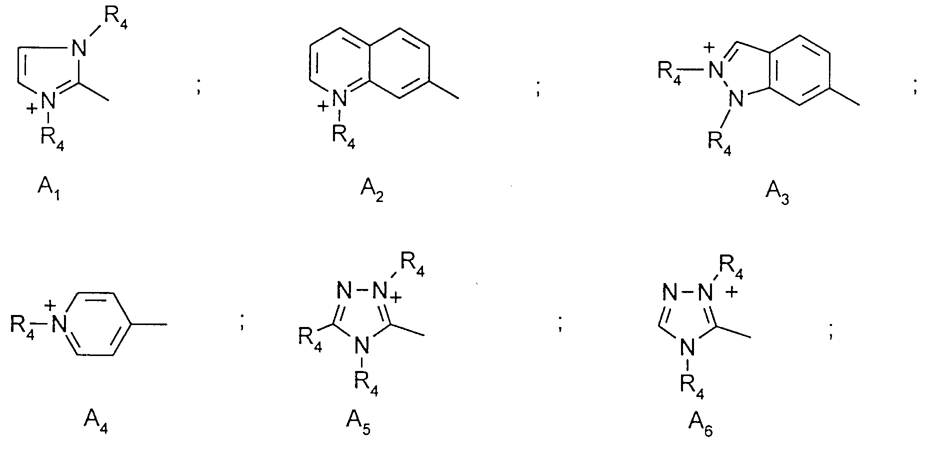 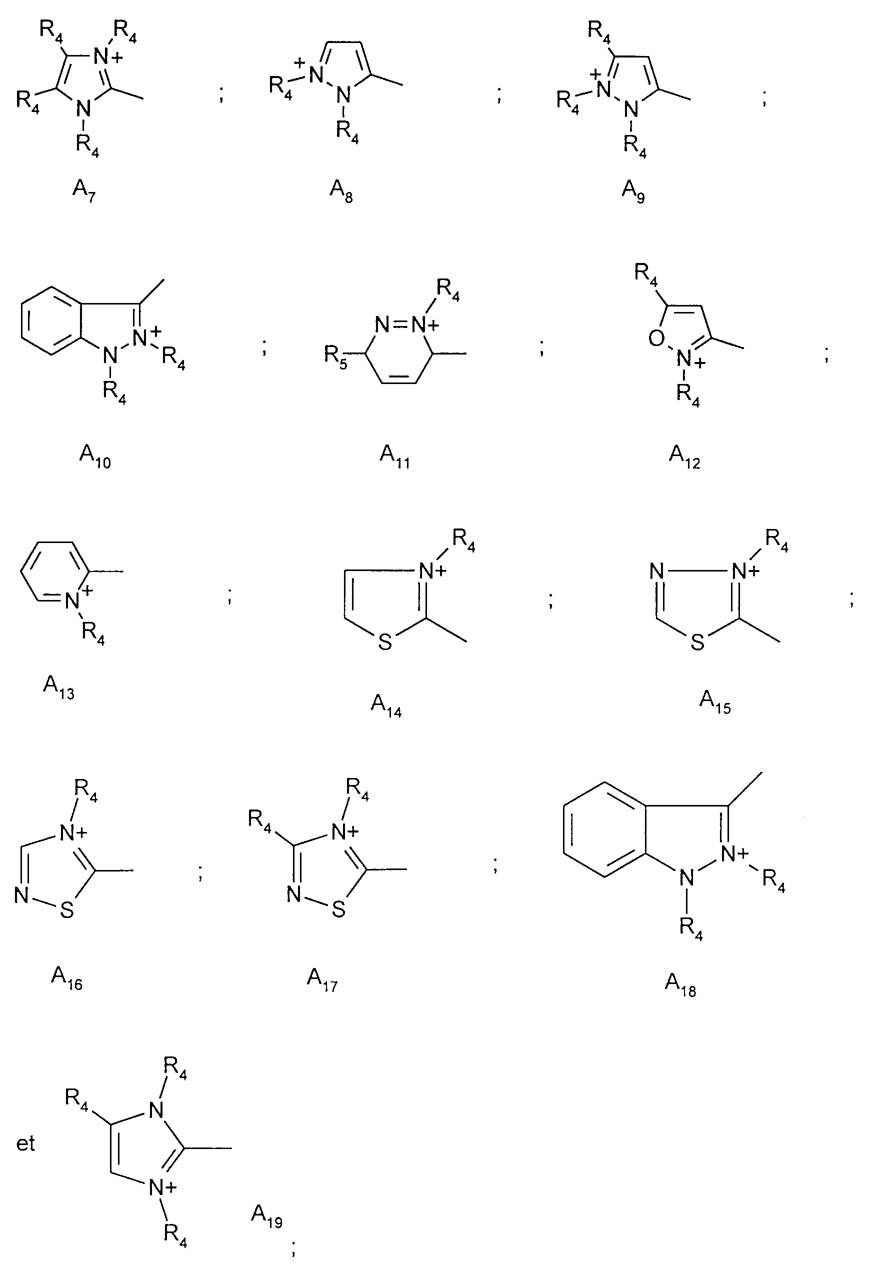
         dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
   b) les composés de formule (II) suivante : 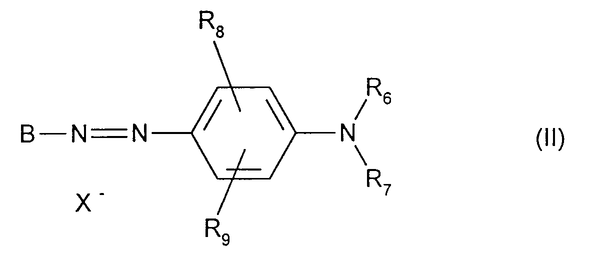 dans laquelle :
      - R₆ représente un atome d'hydrogène ou un radical alkyle en C₁-C₄,
      - R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
      - R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      - X ⁻ représente un anion de préférence choisi parmi le chlorure, le méthyl sulfate et l'acétate,
      - B représente un groupement choisi par les structures B1 à B6 suivantes : 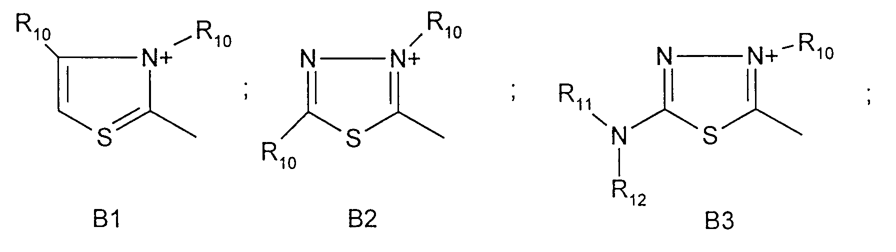 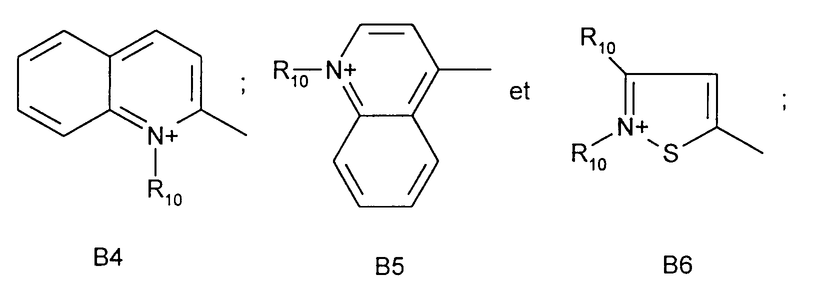
         dans lesquelles R₁₀ représente un radical alkyle en C₁-C₄, R₁₁ et R₁₂, identiques ou différents, représentent un atome d'hydrogène ou un radical alkyle en C₁-C₄ ;
   c) le composé de formule (III'1) suivante : 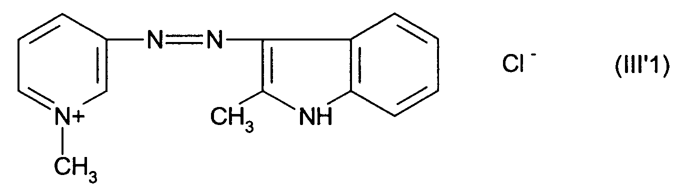 et les composés de formules (III) et (III') suivantes : 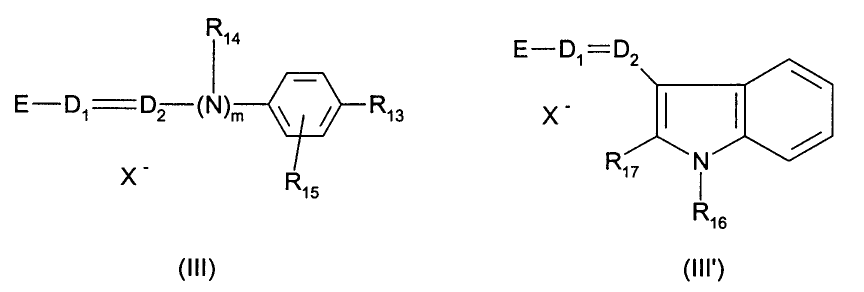 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₁₃ représente un groupement amino non substitué, alors D₁ et D₂ représentent simultanément un groupement -CH et m = 0,
      X ⁻ représente un anion de préférence choisi parmi le chlorure, le méthyl sulfate et l'acétate,
      E représente un groupement choisi par les structures E1 à E7 suivantes : 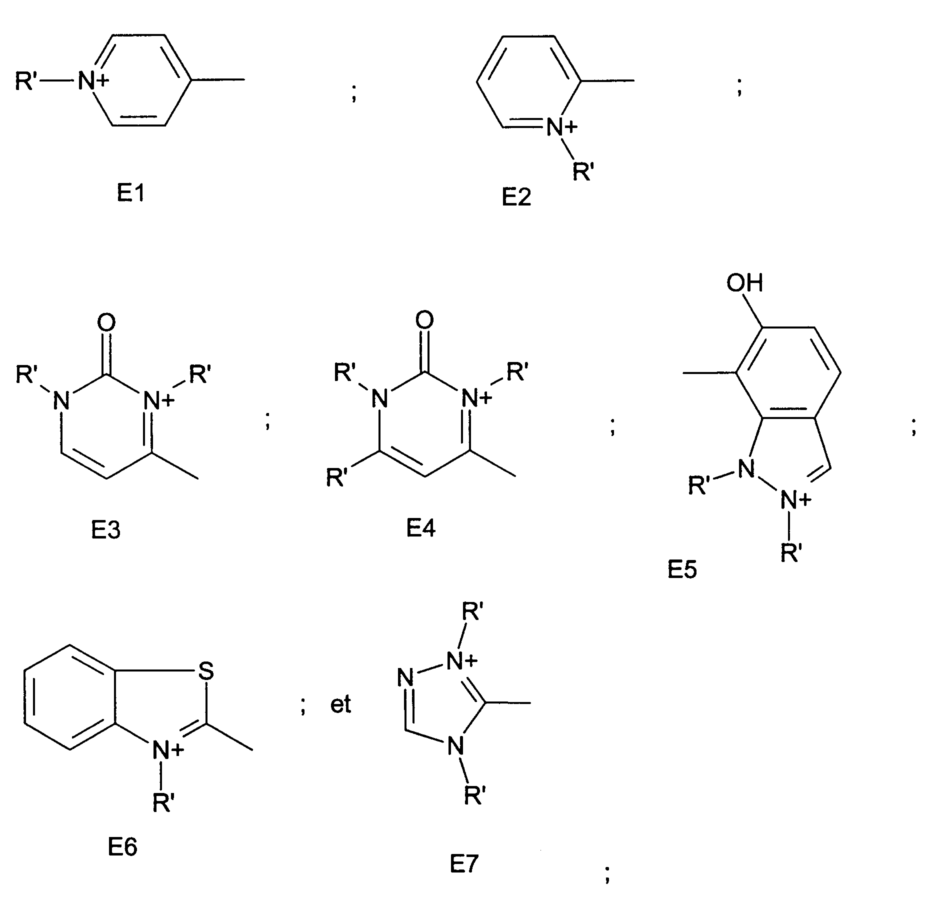
      dans lesquelles R' représente un radical alkyle en C₁-C₄ ;
      lorsque m = 0 et que D₁ représente un atome d'azote, alors E peut également désigner un groupement de structure E8 suivante : 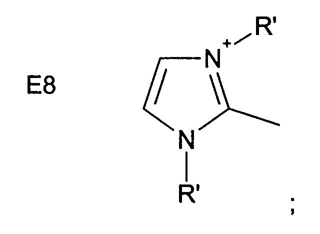
      dans laquelle R' représente un radical alkyle en C₁-C₄ ; et
- au moins un agent oxydant.

Les compositions tinctoriales prêtes à l'emploi conformes à l'invention permettent d'aboutir à des colorations dans des nuances lumineuses présentant une bonne résistance aux différents traitements que peuvent subir les cheveux et en particulier vis-à-vis des shampooings.

L'invention a également pour objet un procédé de teinture d'oxydation des fibres kératiniques mettant en oeuvre cette composition tinctoriale prête à l'emploi.

La ou les bases d'oxydation pouvant être utilisées dans les compositions tinctoriales prêtes à l'emploi conformes à l'invention sont de préférence choisies parmi les paraphénylènediamines, les bis-phénylalkylènediamines, les paraaminophénols, les ortho aminophénols et les bases d'oxydation hétérocycliques.

Parmi les paraphénylènediamines utilisables à titre de base d'oxydation dans les composition tinctoriales prêtes à l'emploi conformes à l'invention, on peut notamment citer les composés de formule (IV) suivante et leurs sels d'addition avec un acide : 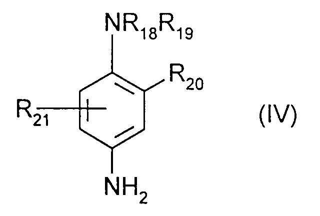 dans laquelle :
R₁₈ représente un atome d'hydrogène, un radical alkyle en C₁-C₄, monohydroxyalkyle en C₁-C₄, polyhydroxyalkyle en C₂-C₄, phényle, 4'-aminophényle ou alcoxy(C₁-C₄)alkyle en C₁-C₄,
R₁₉ représente un atome d'hydrogène, un radical alkyle en C₁-C₄, monohydroxyalkyle en C₁-C₄ ou polyhydroxyalkyle en C₂-C₄,
R₂₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
R₂₁ représente un atome d'hydrogène ou un radical alkyle en C₁-C₄.

Parmi les paraphénylènediamines de formule (IV) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

Parmi les paraphénylènediamines de formule (IV) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Parmi les bis-phénylalkylènediamines utilisables à titre de bases d'oxydation dans les compositions tinctoriales prêtes à l'emploi conformes à l'invention, on peut notamment citer les composés de formule (V) suivante, et leurs sels d'addition avec un acide : 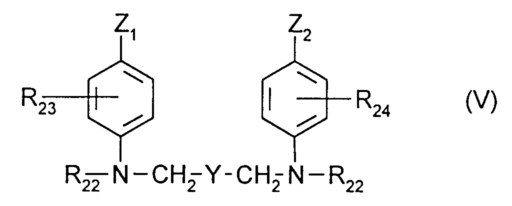 dans laquelle :
Z₁ et Z₂, identiques ou différents, représentent un radical hydroxyle ou NHR₂₅ dans lequel R₂₅ représente un atome d'hydrogène ou un radical alkyle en C₁-C₄,
R₂₂ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₂₃ et R₂₄, identiques ou différents, représentent un atome d'hydrogène ou d'halogène ou un radical alkyle en C₁-C₄,
Y représente un radical pris dans le groupe constitué par les radicaux suivants :
   -(CH₂)ₙ-; -(CH₂)ₘ-O-(CH₂)ₘ; -(CH₂)ₘ-CHOH-(CH₂)ₘ et 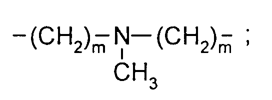
dans lesquels n est un nombre entier compris entre 0 et 8 inclusivement et m est un nombre entier compris entre 0 et 4 inclusivement.

Parmi les bis-phénylalkylènediamines de formules (V) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (V), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans les compositions tinctoriales prêtes à l'emploi conformes à l'invention, on peut notamment citer les composés de formule (VI) suivante, et leurs sels d'addition avec un acide : 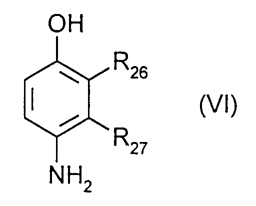 dans laquelle :
R₂₆ représente un atome d'hydrogène ou de fluor, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R₂₇ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₂₆ ou R₂₇ représente un atome d'hydrogène.

Parmi les para-aminophénols de formule (VI) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales prêtes à l'emploi conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales prêtes à l'emploi conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, le 4,5-diamino 1-(4'-chlorobenzyl) pyrazole, et leurs sels d'addition avec un acide.

Les sels d'addition avec un acide utilisables dans le cadre des compositions tinctoriales de l'invention sont notamment choisis parmi les chlorhydrates, les bromhydrates, les sulfates et les tartrates.

Les colorants directs cationiques de formules (I), (II), (III) et (III') utilisables dans les compositions tinctoriales prêtes à l'emploi conformes à l'invention, sont des composés connus et sont décrits par exemple dans les demandes de brevets WO 95/01772, WO 95/15144 et EP-A-0 714 954.

Parmi les colorants directs cationiques de formule (I) utilisables dans les compositions tinctoriales prêtes à l'emploi conformes à l'invention, on peut plus particulièrement citer les composés répondant aux structures (I1) à (I52) suivantes :

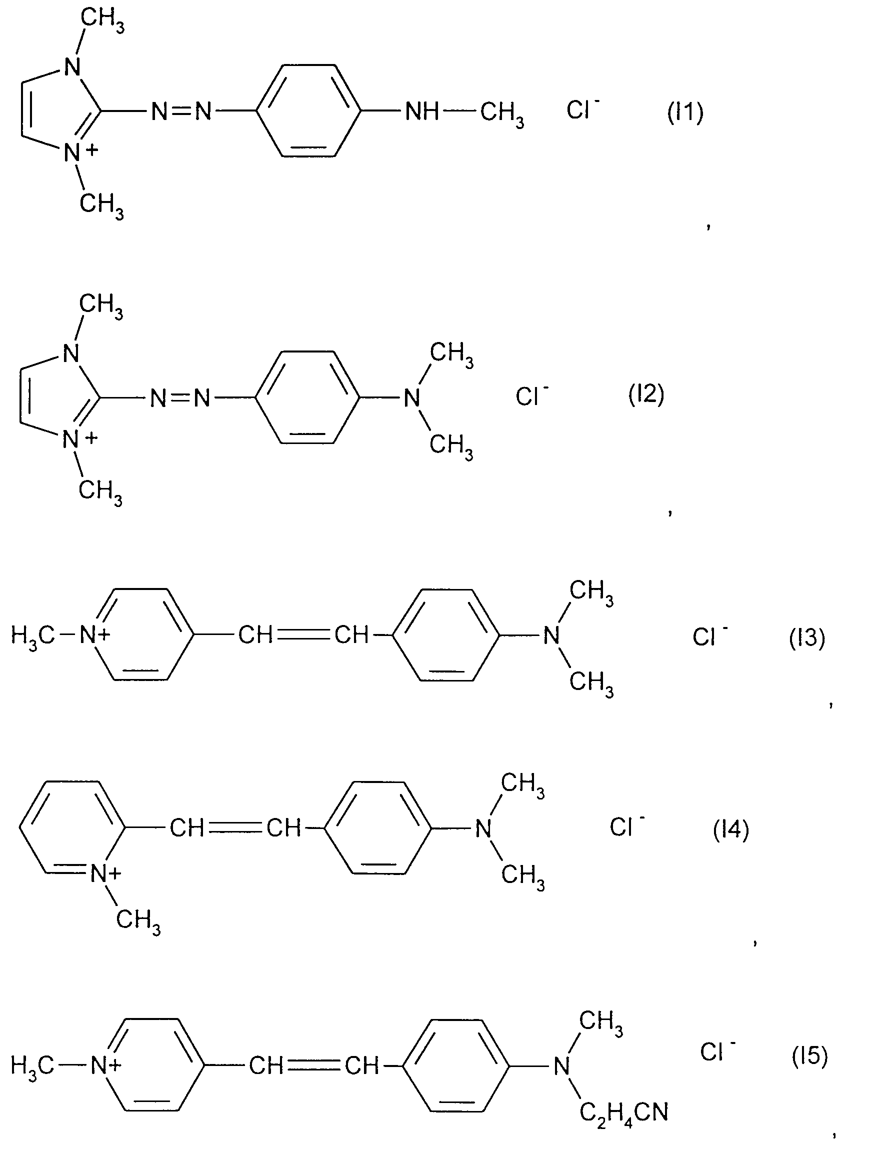

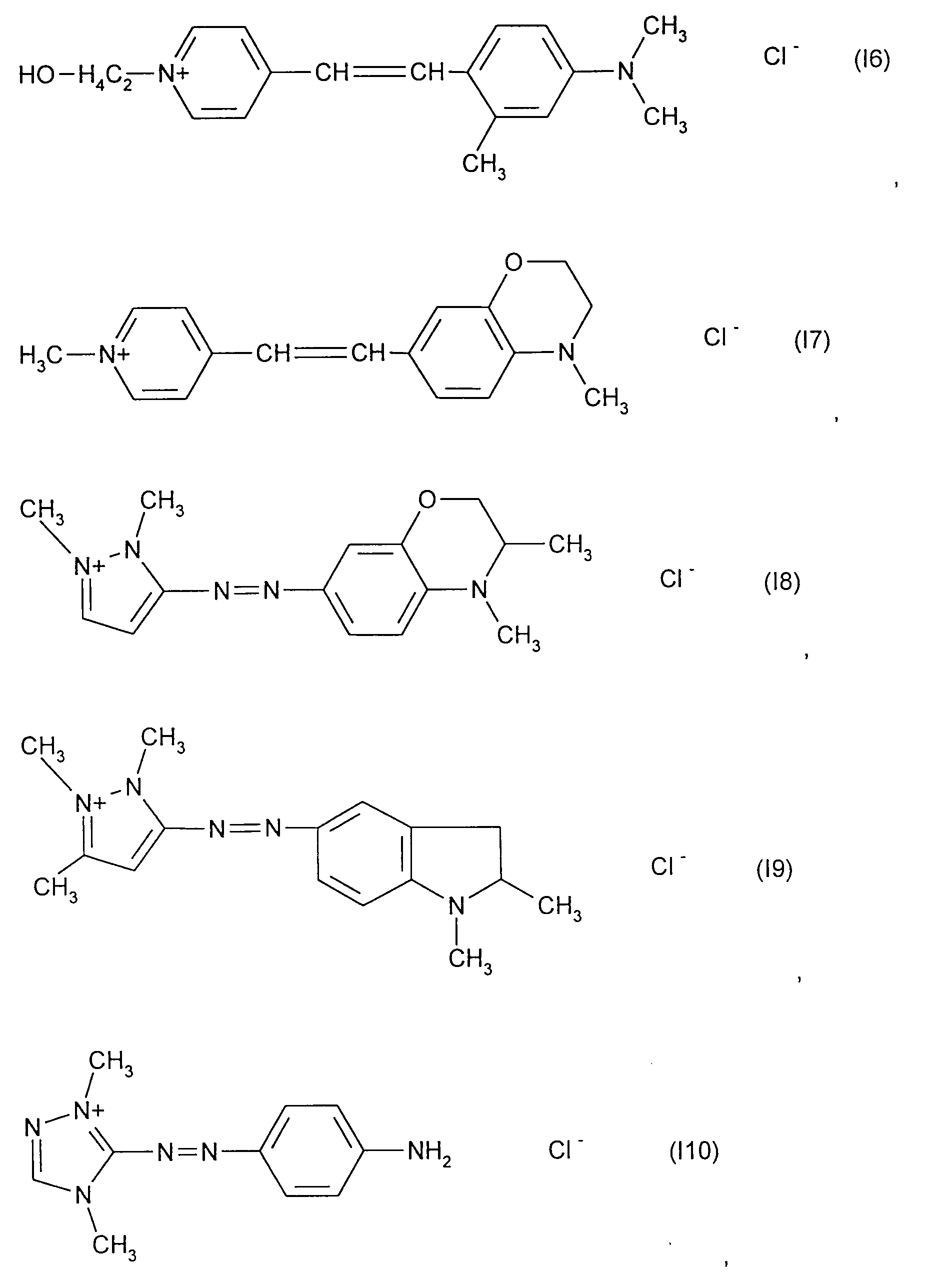

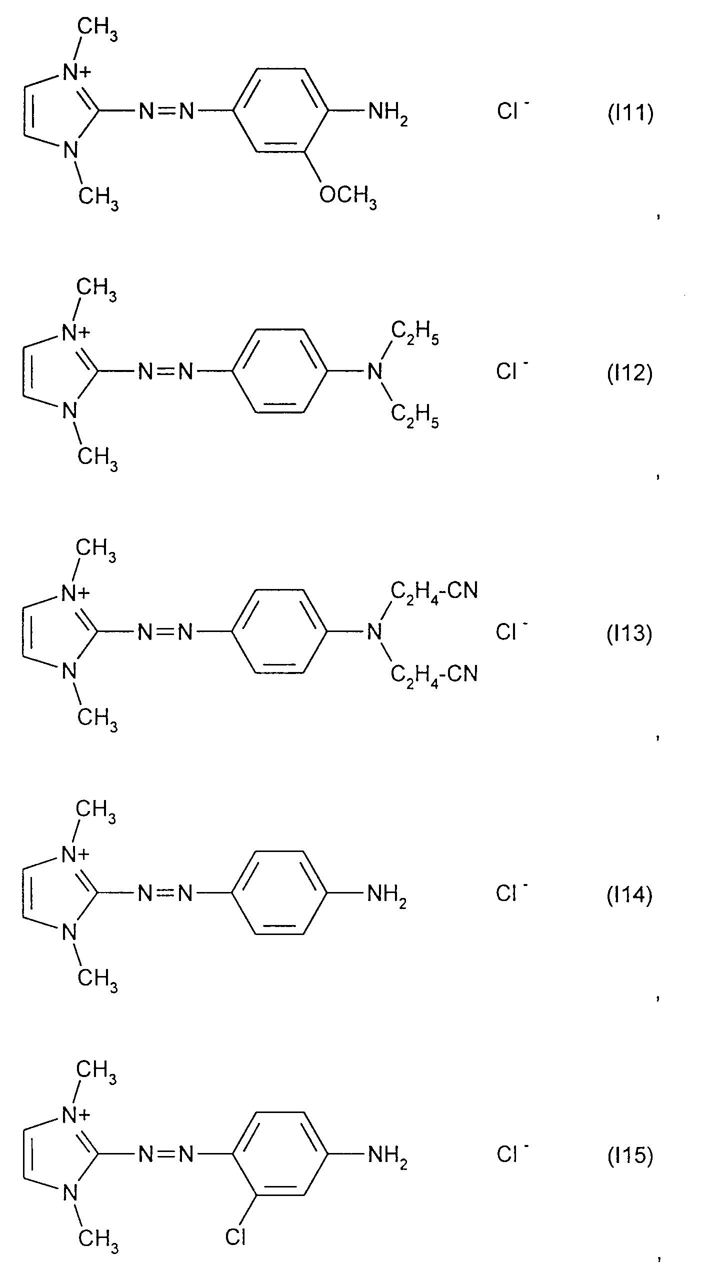

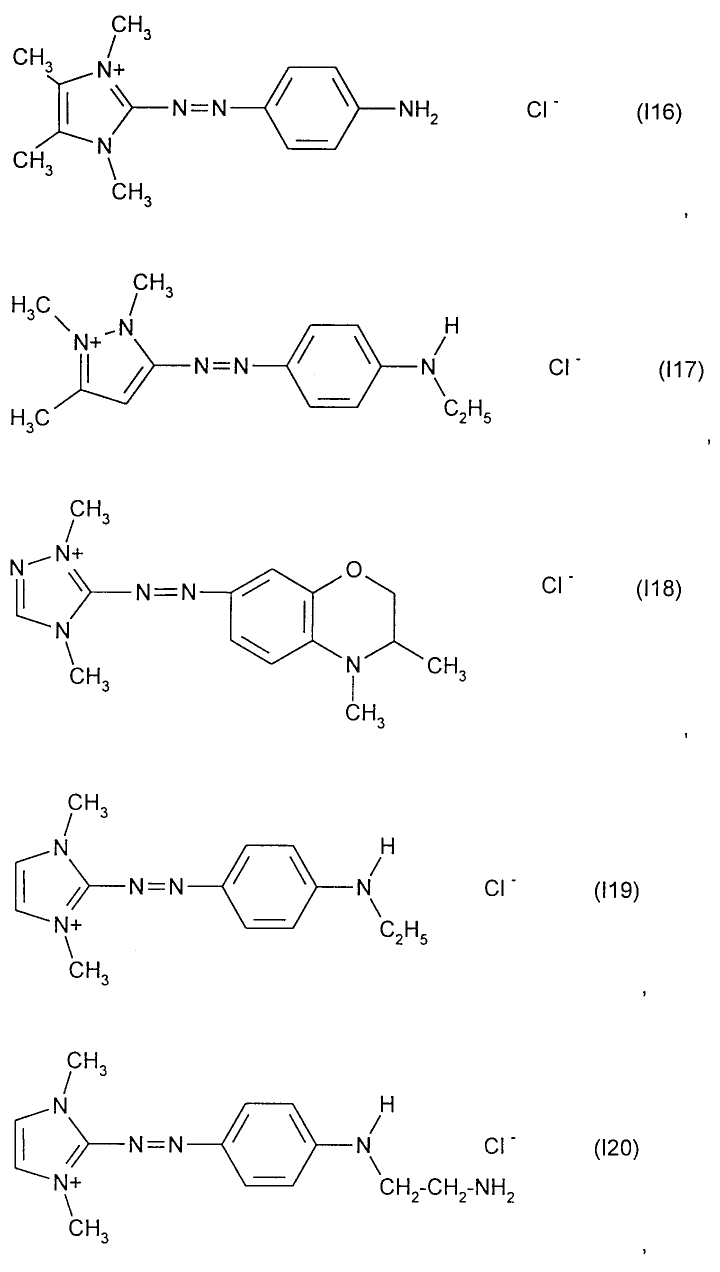

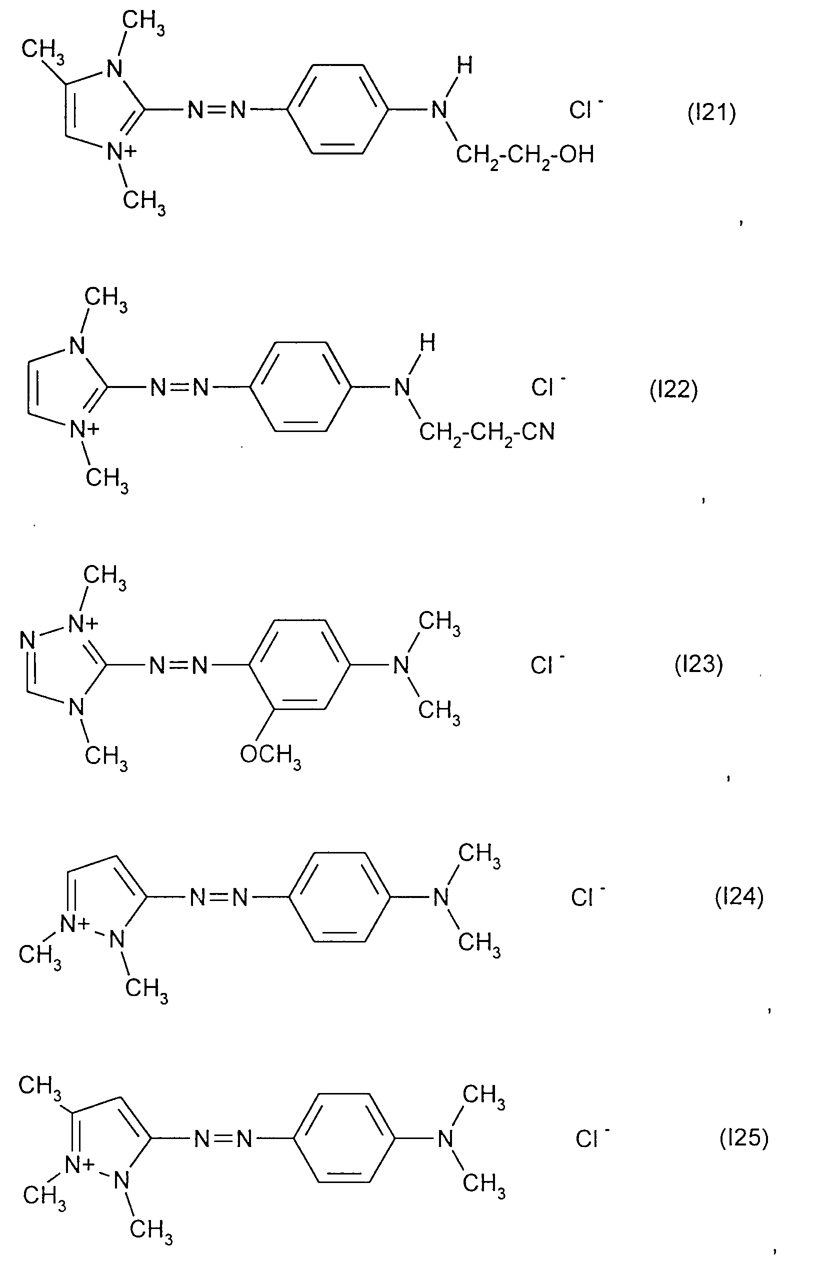

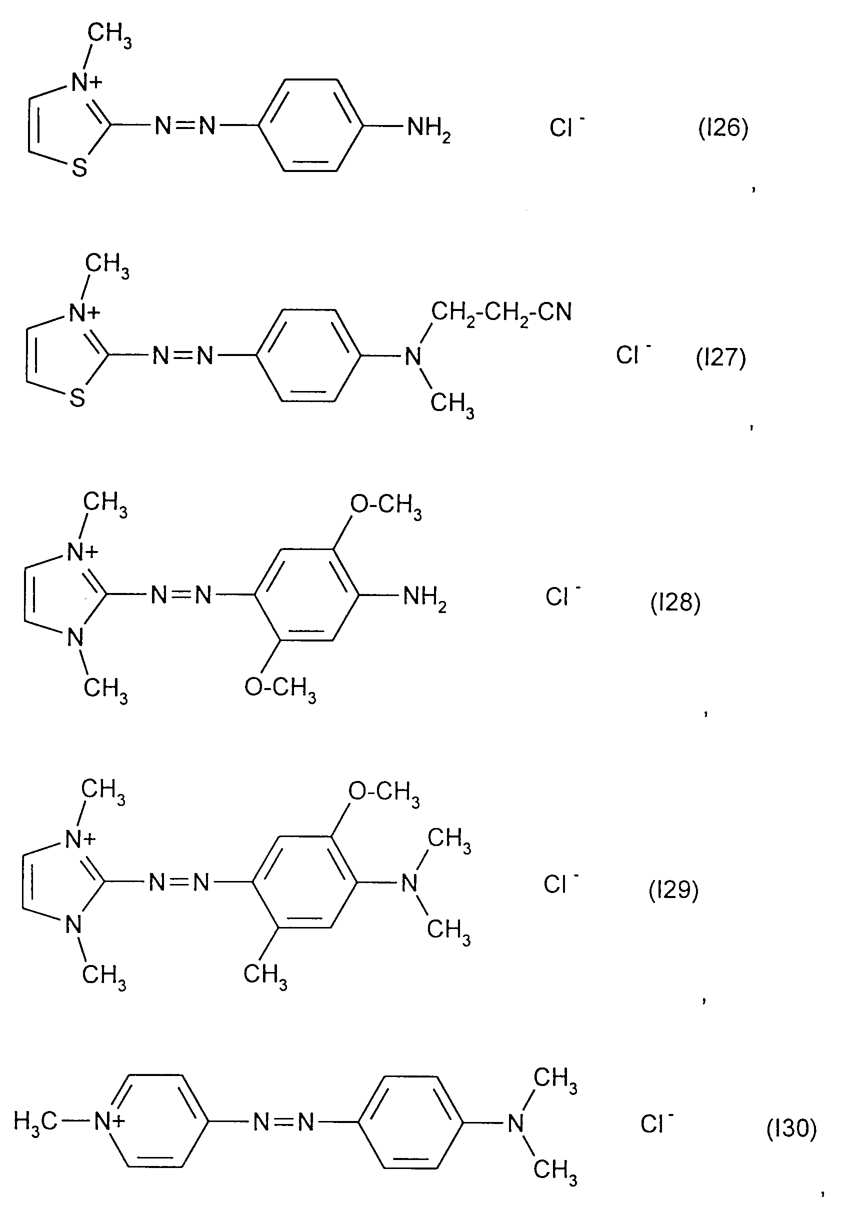

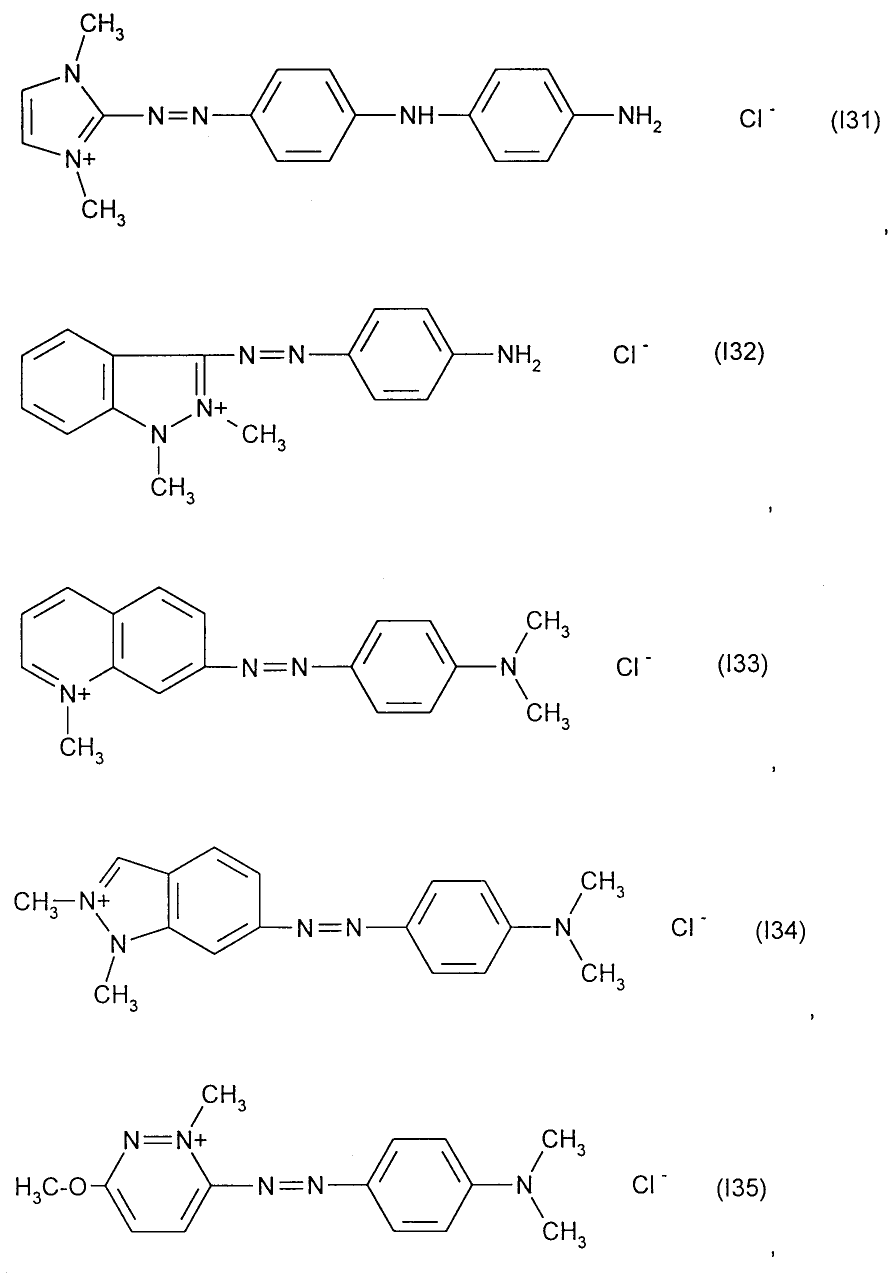

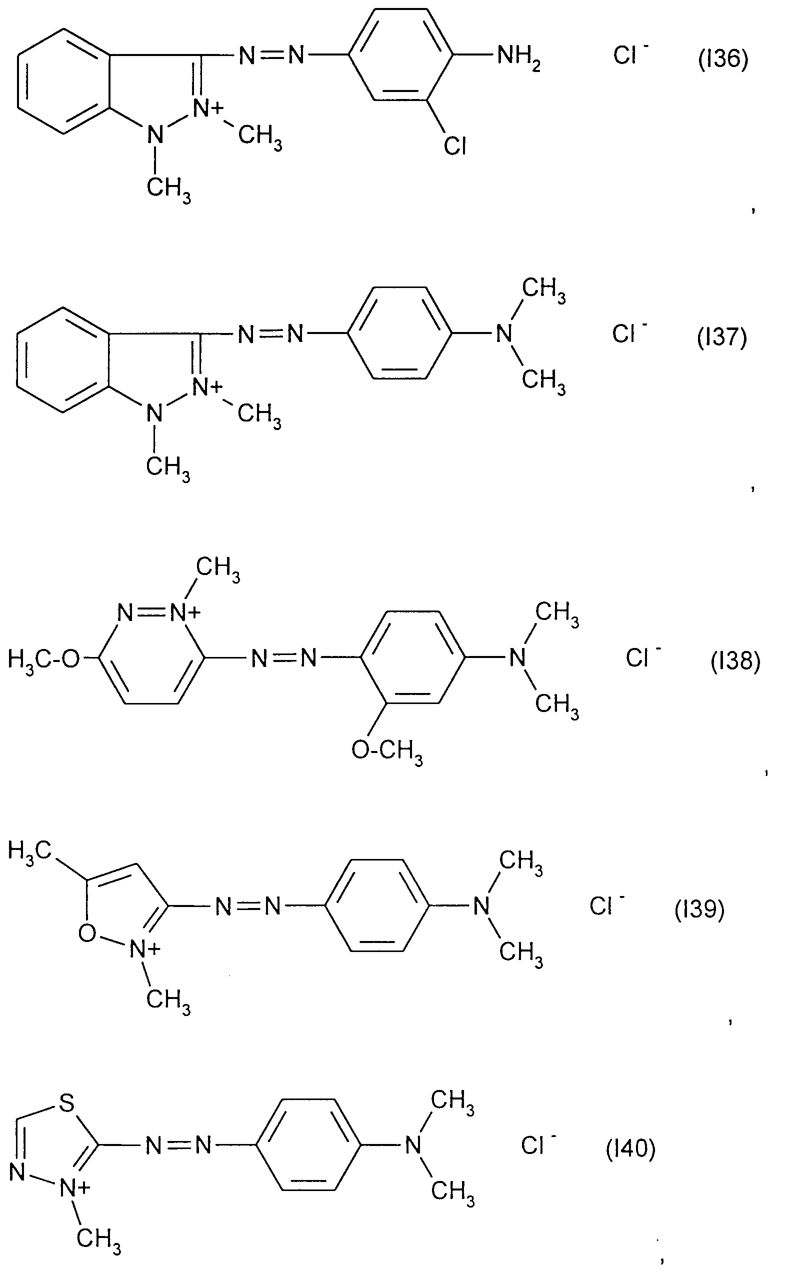

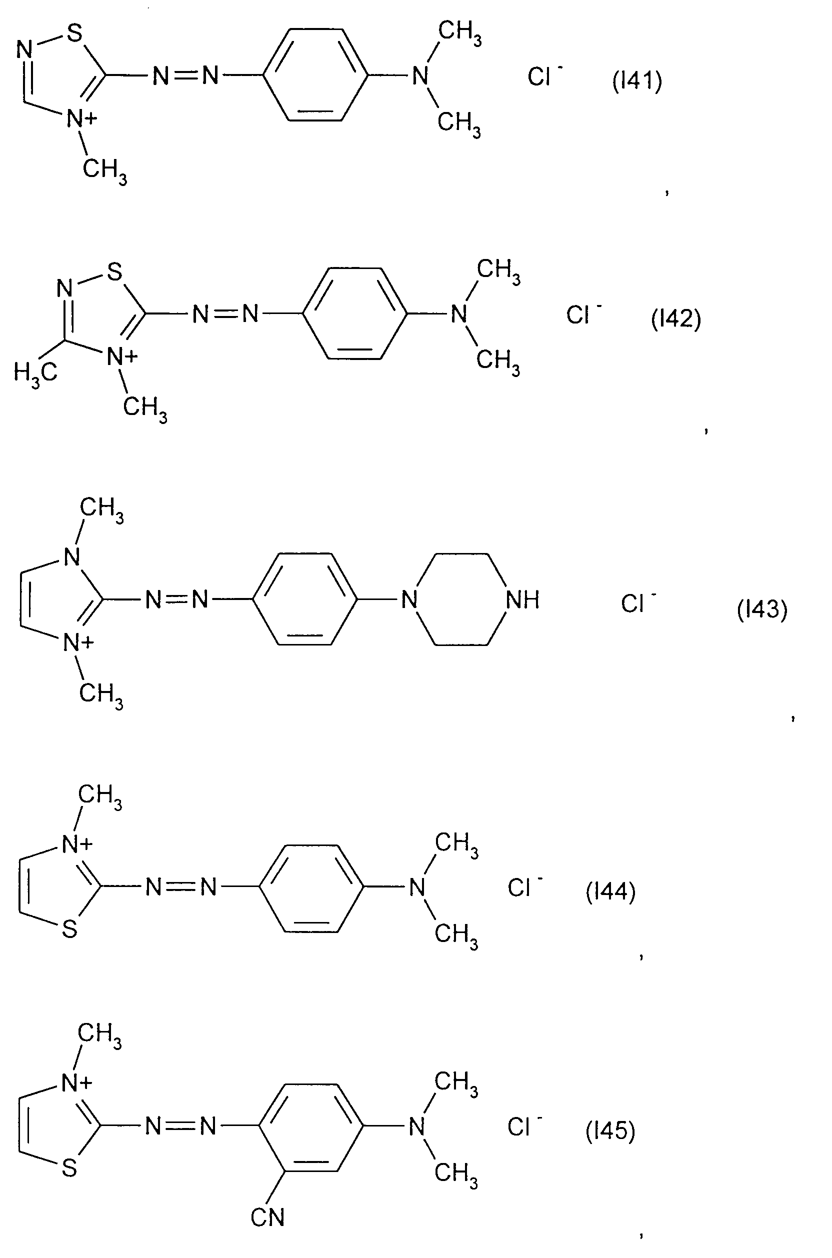

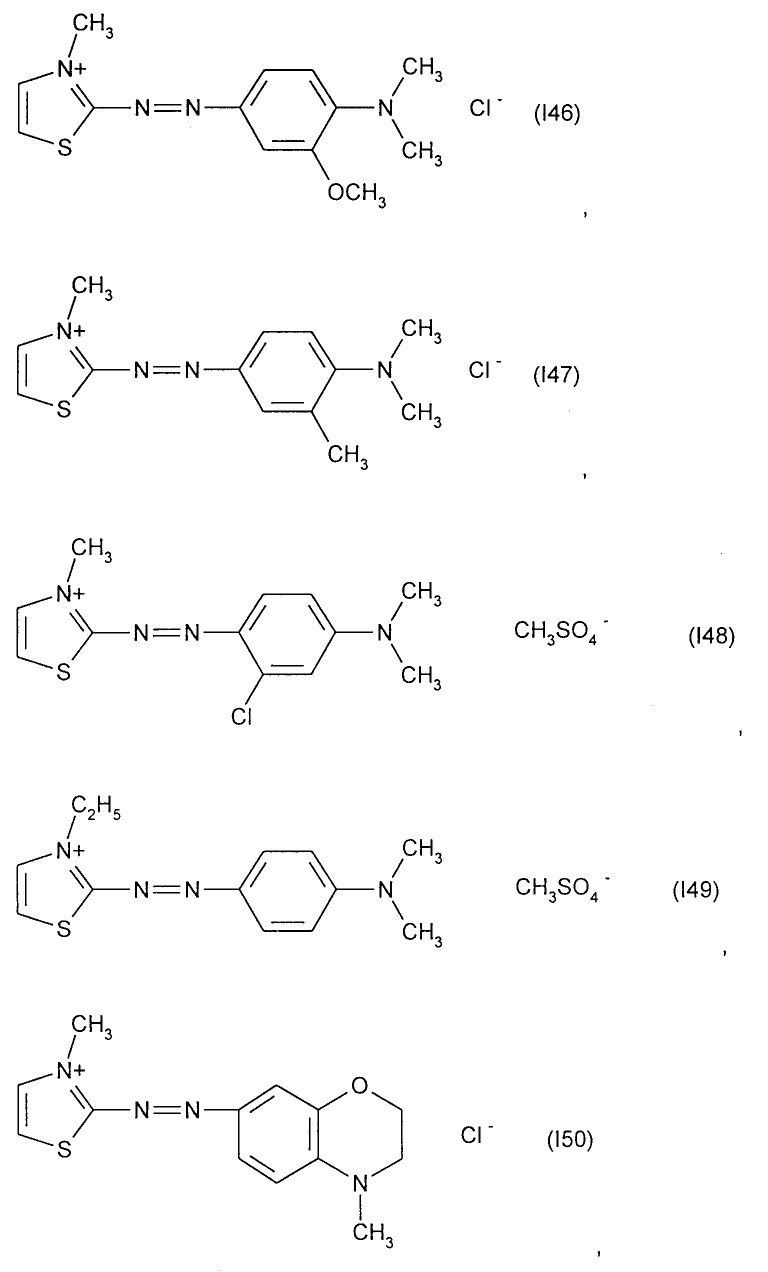

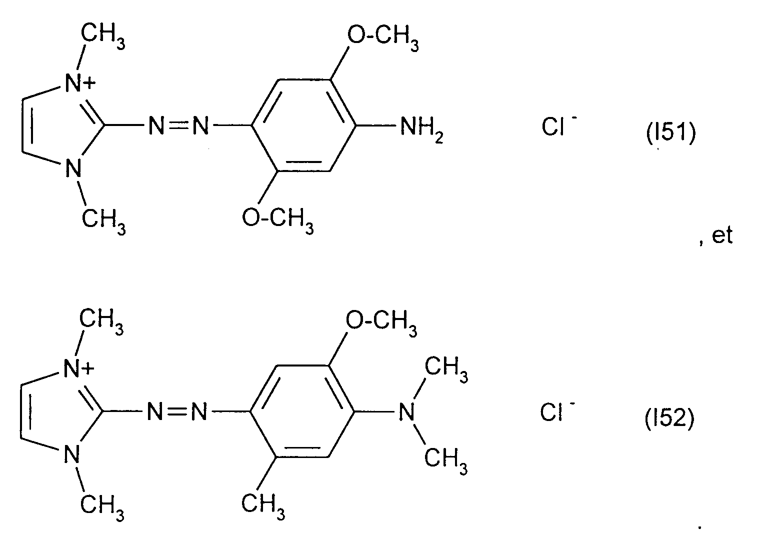

Parmi les composés de structures (I1) à (I52) décrits ci-dessus, on préfère tout particulièrement les composés répondant aux structures (I1), (I2), (I14) et (I31).

Parmi les colorants directs cationiques de formule (II) utilisables dans les compositions tinctoriales prêtes à l'emploi conformes à l'invention, on peut plus particulièrement citer les composés répondant aux structures (II1) à (II12) suivantes :

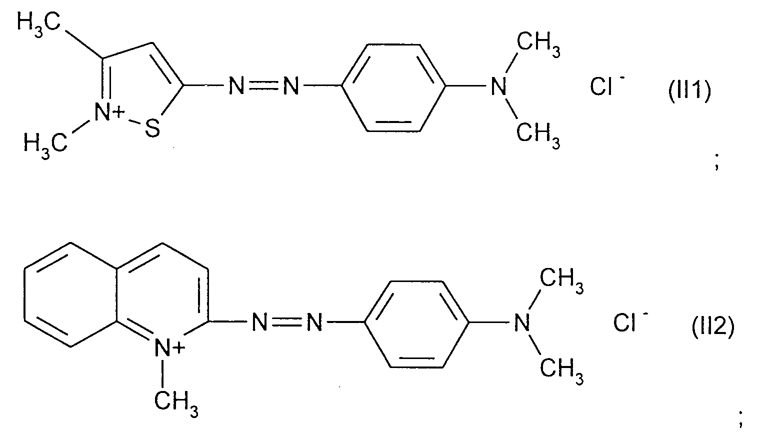

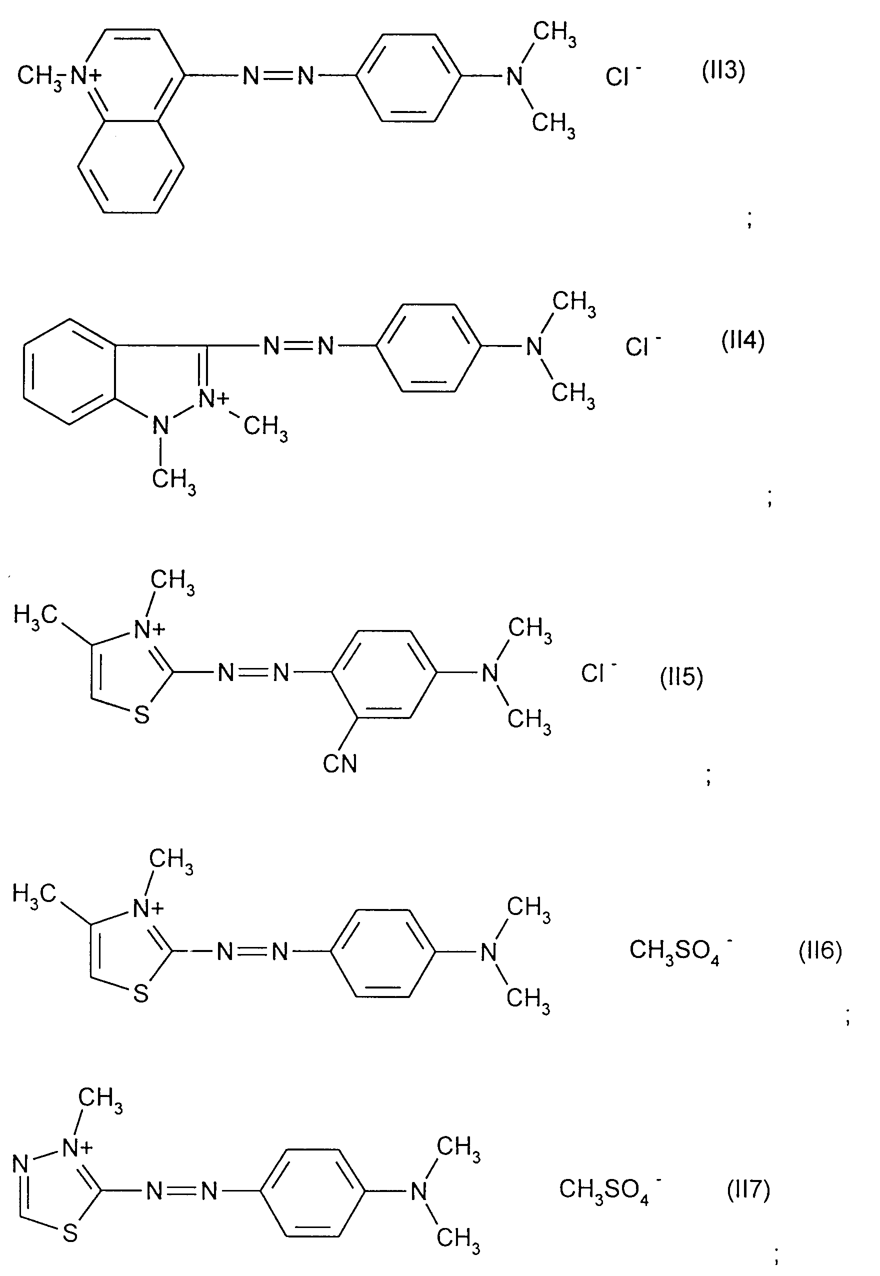

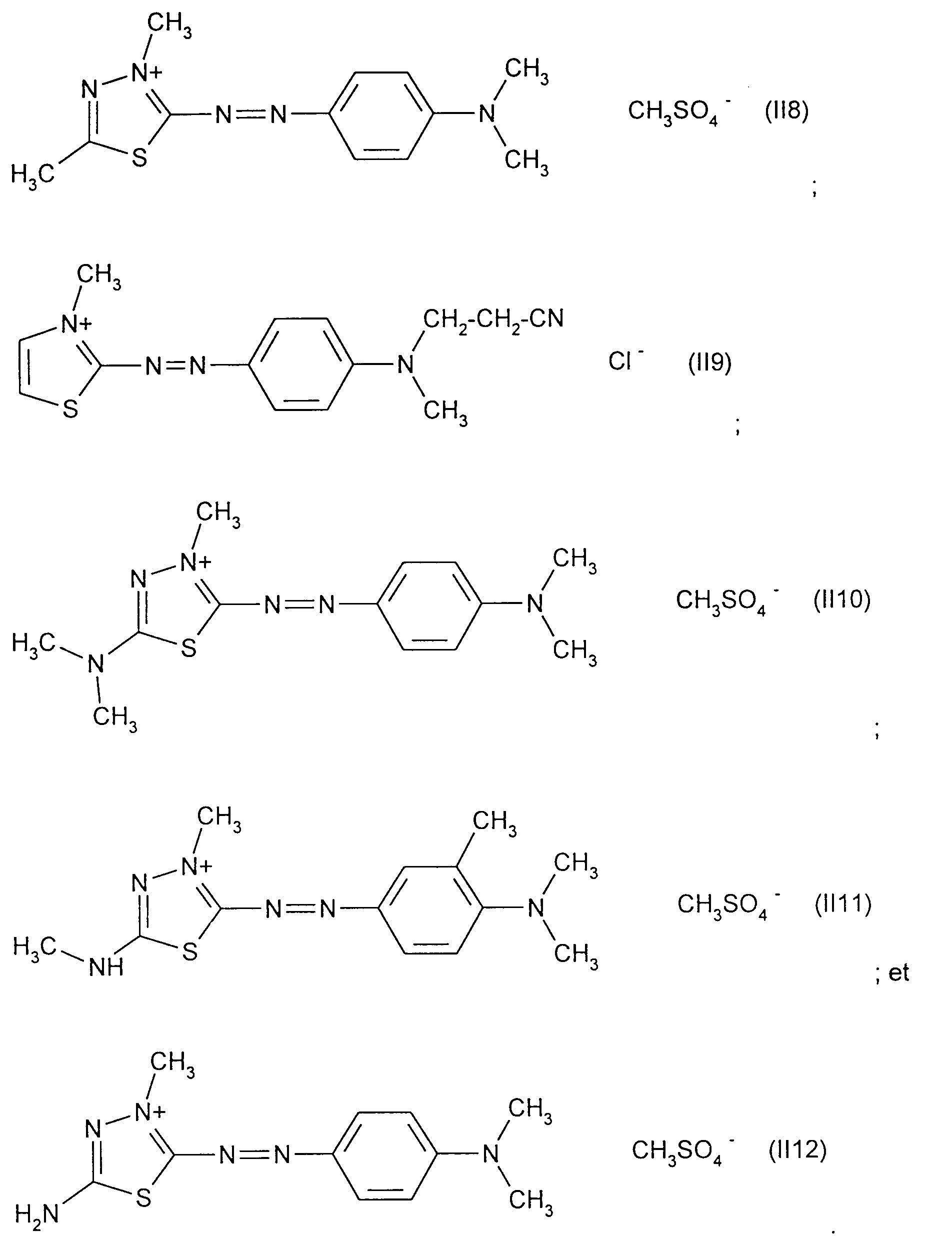

Parmi les colorants directs cationiques de formule (III), utilisables dans les compositions tinctoriales prêtes à l'emploi conformes à l'invention, on peut plus particulièrement citer les composés répondant aux structures (III1) à (III18) suivantes :

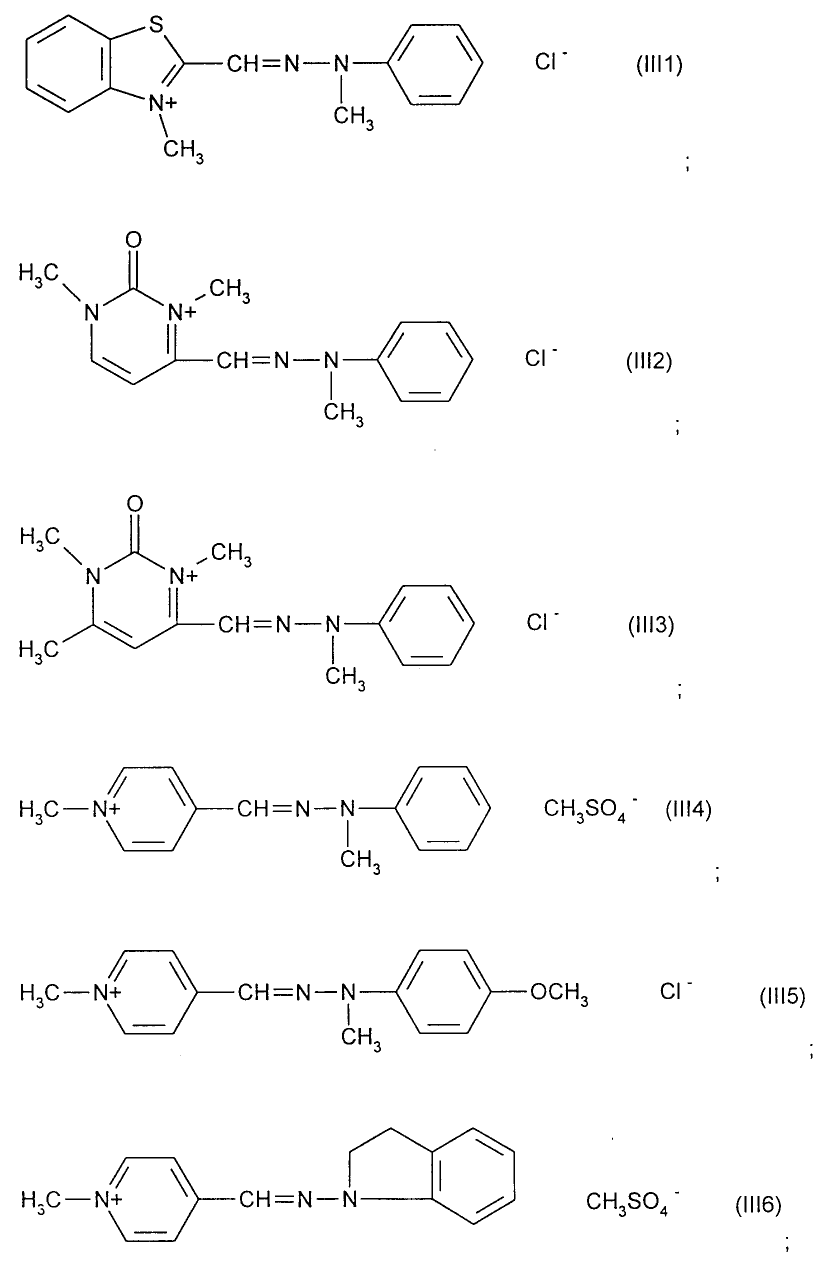

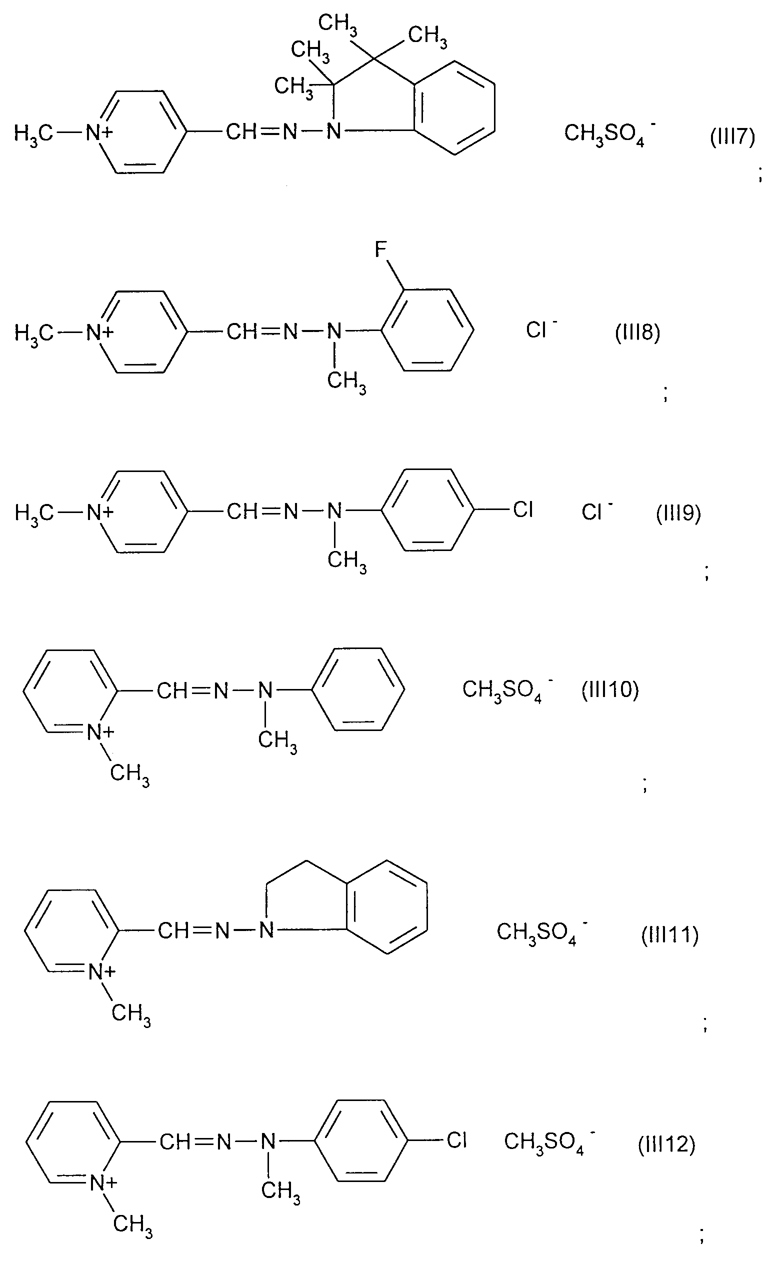

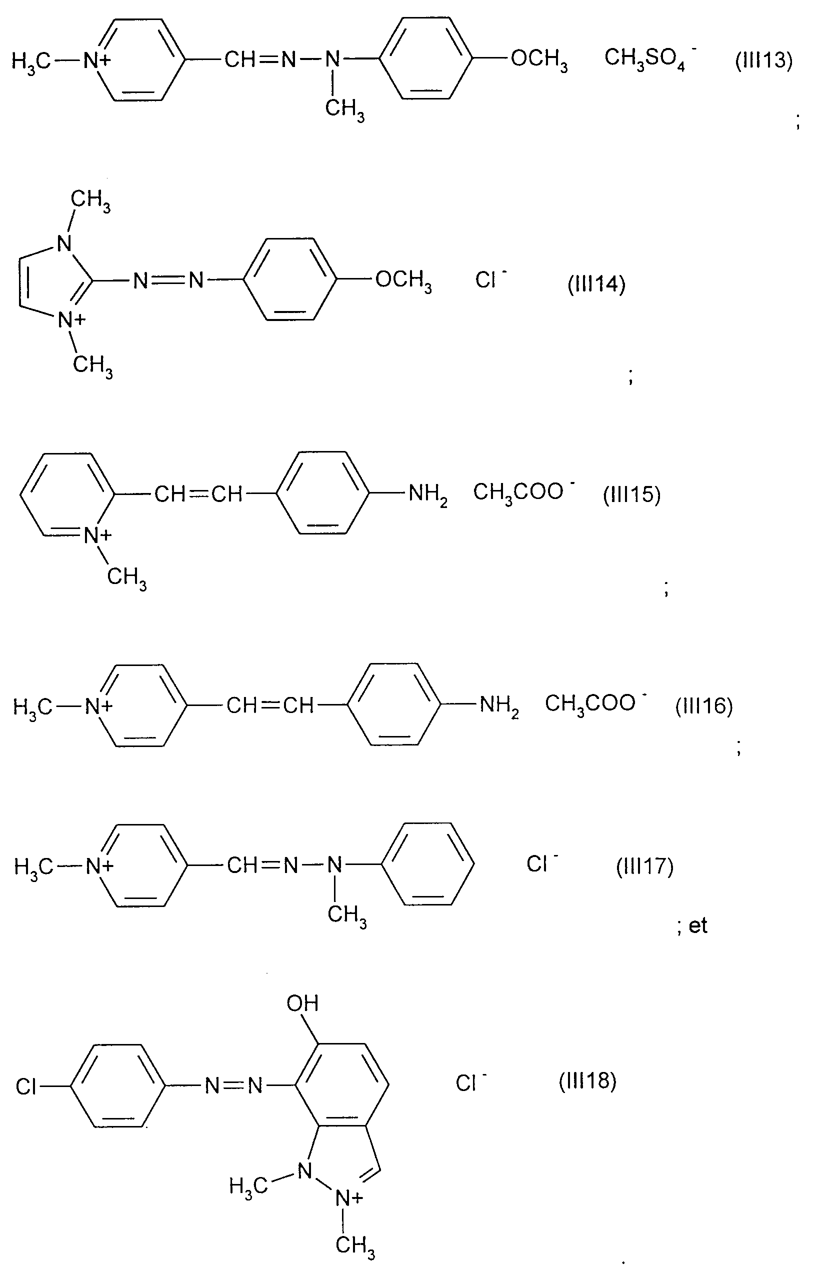

Parmi les composés particuliers de structures (III1) à (III18) décrits ci-dessus, on préfère tout particulièrement les composés répondant aux structures (III4), (III5) et (III13).

La ou les bases d'oxydation représentent de préférence de 0,0001 à 10 % en poids environ du poids total de la composition tinctoriale prête à l'emploi et encore plus préférentiellement de 0,001 à 5 % en poids environ de ce poids.

Le ou les colorants directs cationiques de formules (I) et/ou (II) et/ou (III) et/ou (III') conformes à l'invention, représentent de préférence de 0,001 à 10 % en poids environ du poids total de la composition tinctoriale prête à l'emploi et encore plus préférentiellement de 0,05 à 2 % en poids environ de ce poids.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 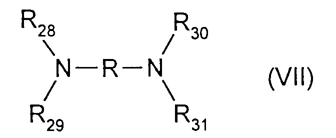
dans laquelle R est un reste propylène éventuellement substitué par un groupement hydroxyle ou un radical alkyle en C₁-C₄ ; R₂₈, R₂₉, R₃₀ et R₃₁, identiques ou différents, représentent un atome d'hydrogène, un radical alkyle en C₁-C₄ ou hydroxyalkyle en C₁-C₄.

Parmi les colorants directs cationiques de formule (III'), utilisables dans les compositions tinctoriales prêtes à l'emploi conformes à l'invention, on peut plus particulièrement citer les composés répondant aux structures (III'2) et (III'3) suivantes :

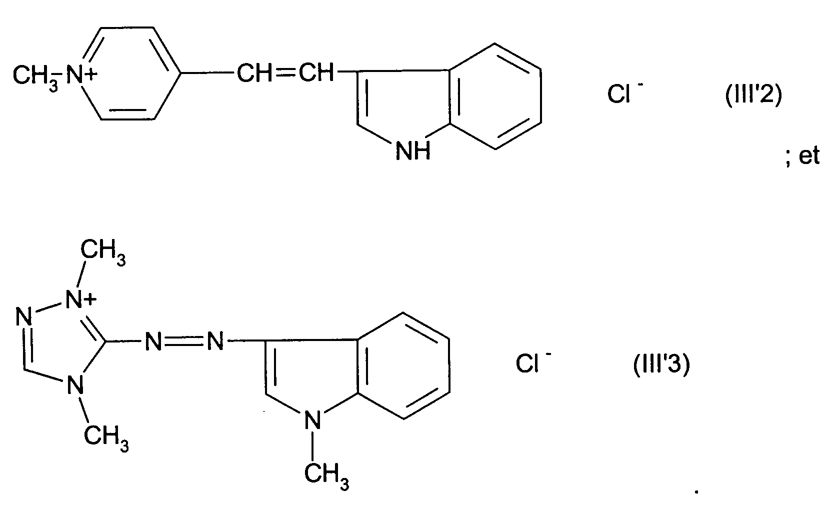

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La ou les bases d'oxydation représentent de préférence de 0,0001 à 10 % en poids environ du poids total de la composition tinctoriale prête à l'emploi et encore plus préférentiellement de 0,001 à 5 % en poids environ de ce poids.

La composition tinctoriale prête à l'emploi conforme à l'invention, peut également contenir, en plus de la ou des bases d'oxydation ci-dessus et en plus du ou des colorants cationiques de formules (I) et/ou (II) et/ou (III) et/ou (III') ci-dessus, au moins un coupleur choisi parmi les coupleurs habituellement utilisés pour le teinture d'oxydation des fibres kératiniques.

Lorsqu'ils sont utilisés, le ou les coupleurs représentent de préférence de 0,0001 à 5 % en poids environ du poids total de la composition tinctoriale prête à l'emploi et encore plus préférentiellement de0,005 à 3 % en poids environ de ce poids.

La composition tinctoriale conforme à l'invention peut encore contenir, en plus des colorants définis ci-dessus, d'autres colorants directs, notamment pour modifier les nuances ou les enrichir en reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxy-éthanol, le propylèneglycol, le monométhyléther de propylèneglycol, le mono-éthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales prêtes à l'emploi conformes à l'invention peuvent également renfermer divers adjuvants utilisés classiquement dans les compositions pour la teinture des cheveux, tels que des agents tensio-actifs anioniques, cationiques, non-ioniques, amphotères, ou leurs mélanges, des polymères anioniques, cationiques, non-ioniques, amphotères, ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Bien entendu, l'homme de l'art veillera à choisir le ou les éventuels composés complémentaires mentionnés ci-avant de manière telle que les propriétés avantageuses attachées intrinsèquement à la composition tinctoriale prête à l'emploi conforme à l'invention ne soient pas, ou substantiellement pas, altérées par la ou les adjonctions envisagées.

Les compositions tinctoriales prêtes à l'emploi conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres la composition tinctoriale prête à l'emploi telle que définie précédemment, et on laisse poser pendant 3 à 40 minutes environ, de préférence 5 à 30 minutes environ, après quoi on rince, on lave éventuellement au shampooing, on rince à nouveau et on sèche.

Selon une première forme de réalisation préférée, le procédé comporte une étape préliminaire consistant à stocker sous forme séparée, d'une part, une composition (A) comprenant, dans un milieu approprié pour la teinture, au moins une base d'oxydation et au moins un colorant direct cationique choisi parmi les composés de formules (I), (II), (III) et (III') telles que définies précédemment et, d'autre part, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

Selon une deuxième forme de réalisation préférée, le procédé comporte une étape préliminaire consistant à stocker sous forme séparée, d'une part, une composition (A) comprenant, dans un milieu approprié pour la teinture au moins une base d'oxydation ; d'autre part une composition (A') comprenant, dans un milieu approprié pour la teinture, au moins un colorant direct cationique choisi parmi les composés de formules (I), (II), (III) et (III') telles que définies précédemment, et enfin, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

La composition (A') utilisée selon cette deuxième variante du procédé conforme à l'invention, peut éventuellement se présenter sous forme de poudre, le ou les colorants directs cationiques de formules (I), (II), (III) et (III') conformes à l'invention constituant alors à lui (eux) seuls la totalité de ladite composition (A') ou étant éventuellement dispersé(s) dans un excipient pulvérulent organique et/ou minéral.

Lorsqu'il est présent dans la composition A', l'excipient organique peut être d'origine synthétique ou végétale et est choisi notamment parmi les polymères synthétiques réticulés et non réticulés, les polysaccharides comme les celluloses et les amidons modifiés ou non ainsi que les produits naturels les renfermant tels que la sciure de bois et les gommes végétales (guar, caroube, xanthane, etc...).

Lorsqu'il est présent dans la composition (A'), l'excipient minéral peut être constitué par des oxydes métalliques tels que les oxydes de titane, les oxydes d'aluminium, le kaolin, le talc, les silicates, le mica et les silices.

Un excipient avantageusement préféré selon l'invention est la sciure de bois.

La composition (A') en poudre peut encore renfermer des liants ou des produits d'enrobage dans une quantité ne dépassant pas de préférence 3% en poids environ du poids total de ladite composition (A').

Ces liants sont de préférence choisis parmi les huiles et les corps gras liquides d'origine minérale, synthétique, animale ou végétale.

La composition (A') peut éventuellement encore contenir d'autres adjuvants, à l'état de poudre, en particulier des tensio-actifs de toute nature, des agents de conditionnement du cheveu comme par exemple des polymères cationiques, etc...

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus, un second compartiment éventuel renferme la composition (A') telle que définie ci-dessus lorsqu'elle est présente et un troisième compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1

On a préparé la composition 1 (A), conforme à l'invention, suivante (teneurs en grammes) :

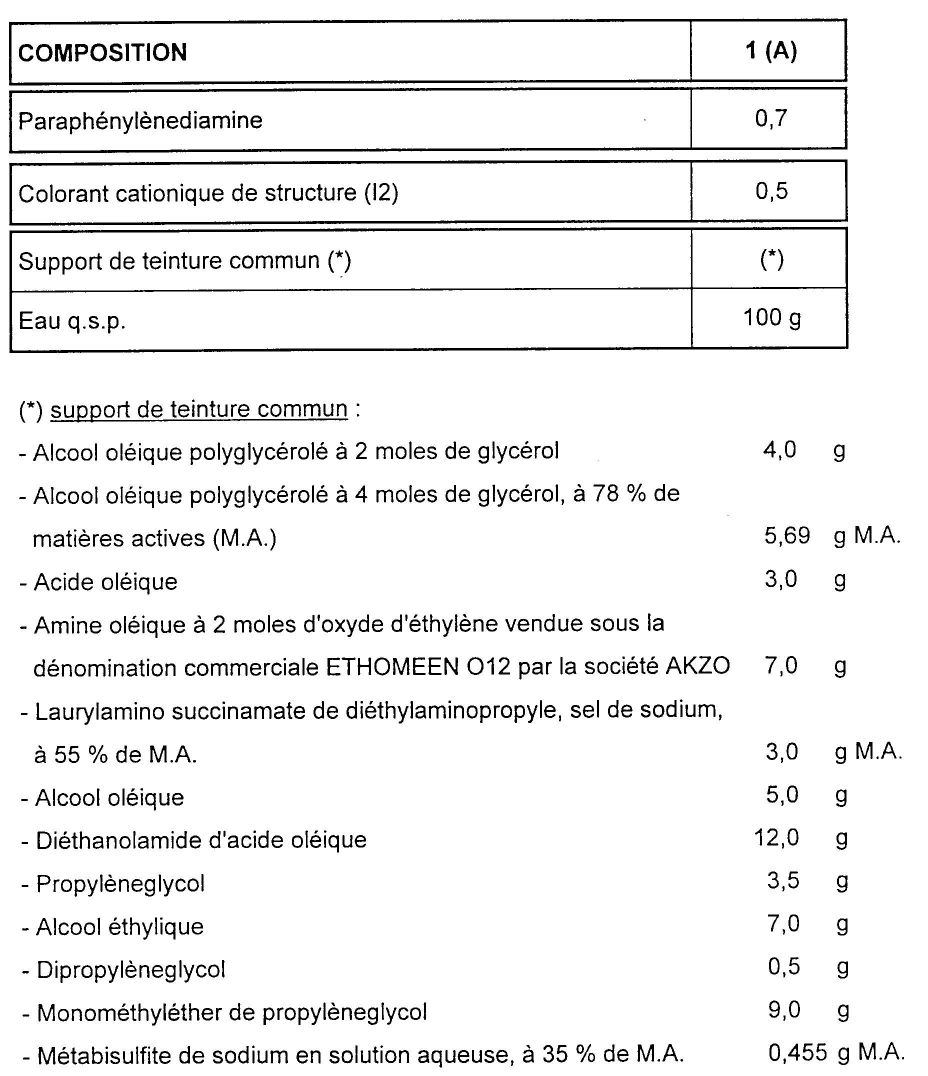

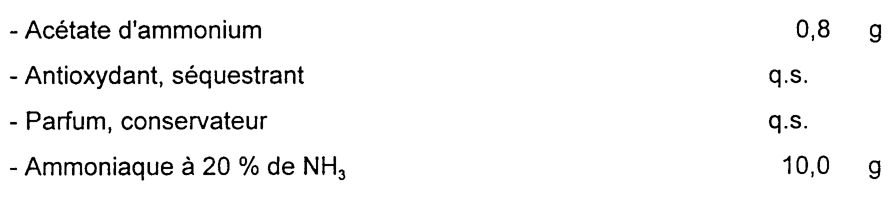

Au moment de l'emploi, on a mélangé la composition 1 (A) avec une quantité égale d'une composition (B) constituée par une solution de peroxyde d'hydrogène à 20 volumes (6 % en poids).

La composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les cheveux ont été teints dans une nuance rouge intense résistant bien aux shampooings ultérieurs.

Selon une variante de l'invention, le colorant direct cationique de structure (I2) est introduit au moment de l'emploi dans la composition 1 (A).

### EXEMPLE 2

### On a préparé la composition 2 (A) suivante :

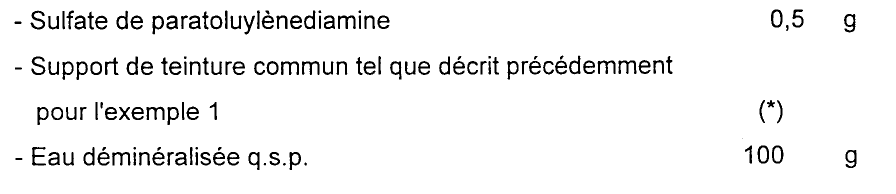

### On a préparé la composition 2 (A') suivante :

| | |
|---|---|
| - Colorant cationique de structure (I14) | 4 g |
| - Polyammonium quaternaire vendu sous la dénomination commerciale CELQUAT SC-240 par la société National Starch | 10 g |
| - Sciure de bois q.s.p. | 100 g |

Au moment de l'emploi, on a mélangé une partie en poids de la composition 2 (A) ci-dessus avec 0,1 partie en poids de la composition 2 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance cuivré intense résistant bien aux shampooings ultérieurs.

## Revendications

1. Composition prête à l'emploi pour la teinture d'oxydation des fibres kératiniques et en particulier des fibres kératiniques humaines telles que les cheveux caractérisée par le fait qu'elle comprend, dans un milieu approprié pour la teinture :
- au moins une base d'oxydation
- au moins un colorant direct cationique choisi parmi :
a) les composés de formule (I) suivante : 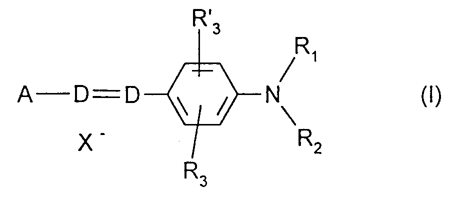 dans laquelle :
- D représente un atome d'azote ou le groupement -CH,
- R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
- R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
- X ⁻ représente un anion de préférence choisi parmi le chlorure, le méthyl sulfate et l'acétate,
- A représente un groupement choisi par les structures A1 à A19 suivantes : 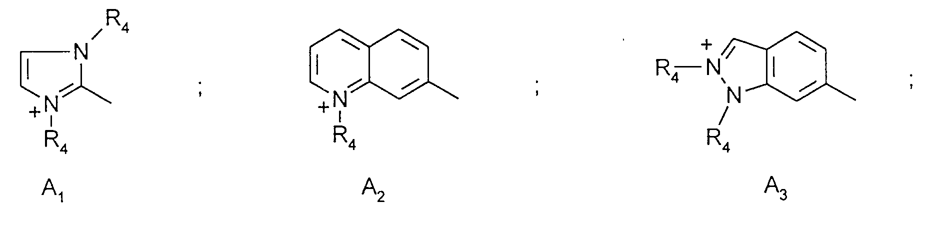 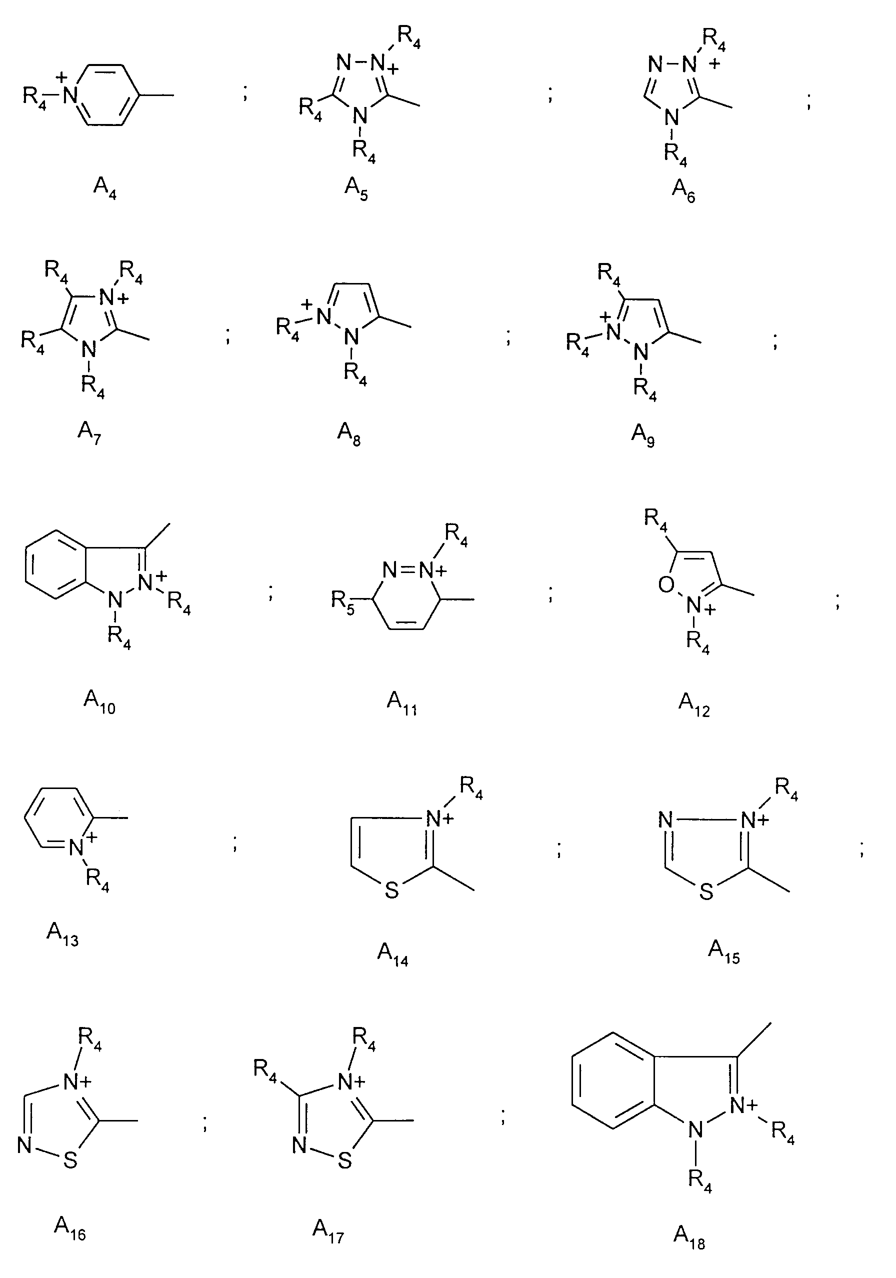 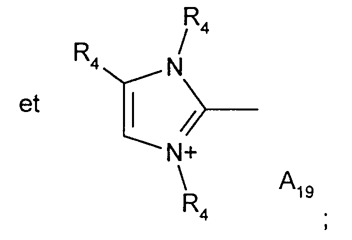
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
b) les composés de formule (II) suivante : 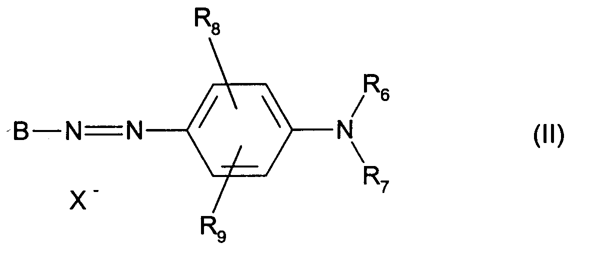 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X ⁻ représente un anion de préférence choisi parmi le chlorure, le méthyl sulfate et l'acétate,
B représente un groupement choisi par les structures B1 à B6 suivantes : 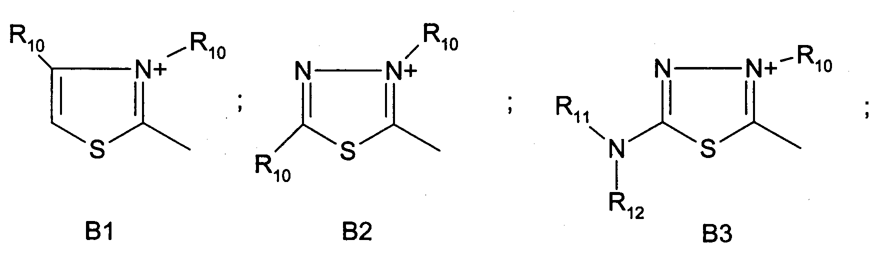 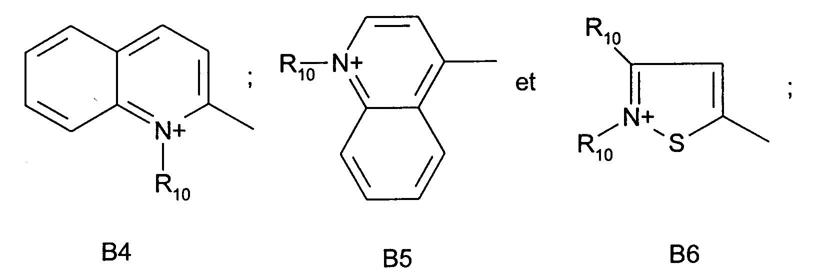 dans lesquelles R₁₀ représente un radical alkyle en C₁-C₄, R₁₁ et R₁₂, identiques ou différents, représentent un atome d'hydrogène ou un radical alkyle en C₁-C₄ ;
c) le composé de formule (III'1) suivante : 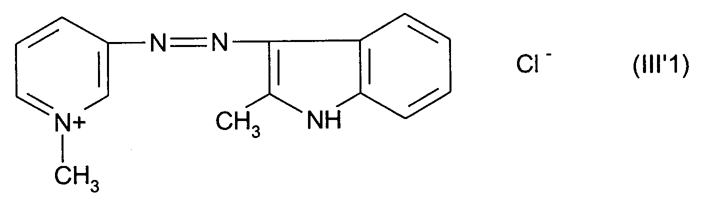 et les composés de formules (III) et (III') suivantes : 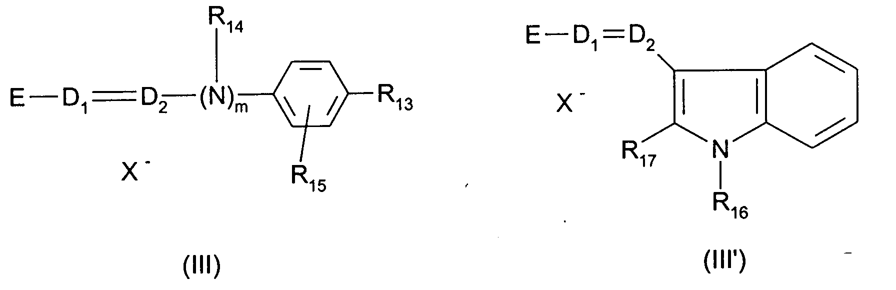 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X ⁻ représente un anion de préférence choisi parmi le chlorure, le méthyl sulfate et l'acétate,
E représente un groupement choisi par les structures E1 à E7 suivantes : 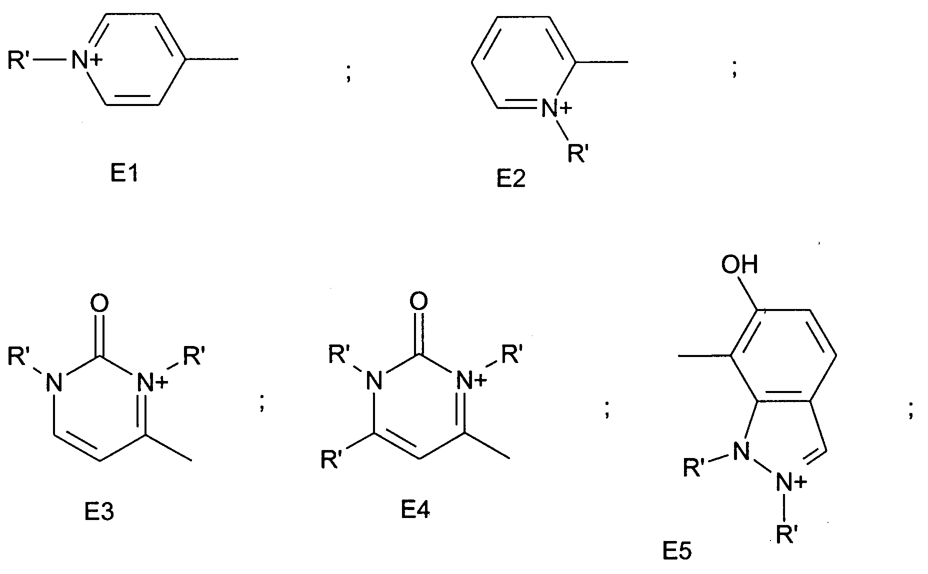 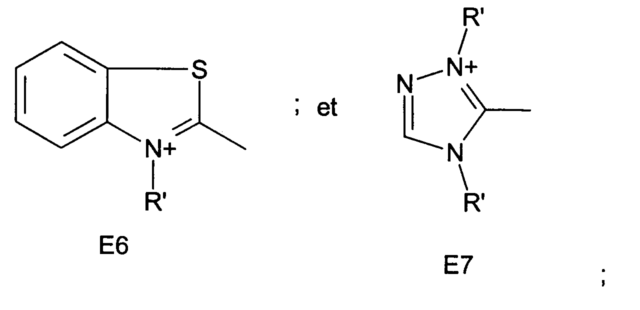 dans lesquelles R' représente un radical alkyle en C₁-C₄ ;
lorsque m = 0 et que D₁ représente un atome d'azote, alors E peut également désigner un groupement de structure E8 suivante : 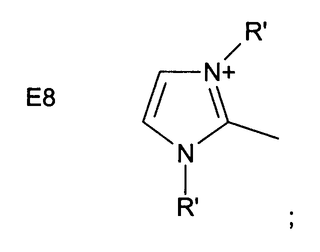 dans laquelle R' représente un radical alkyle en C₁-C₄ ; et
au moins un agent oxydant.

2. Composition selon la revendication 1, caractérisée par le fait que la ou les bases d'oxydation sont choisies parmi les paraphénylènediamines, les bis-phénylalkylènediamines, les para-aminophénols, les ortho aminophénols et les bases d'oxydation hétérocycliques.

3. Composition selon la revendication 2, caractérisée par le fait que les paraphénylènediamines sont choisies parmi les composés de formule (IV) suivante et leurs sels d'addition avec un acide : 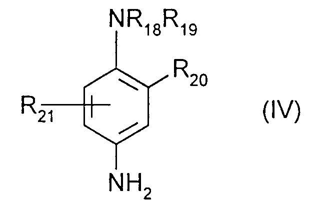 dans laquelle :
R₁₈ représente un atome d'hydrogène, un radical alkyle en C₁-C₄, monohydroxyalkyle en C₁-C₄, polyhydroxyalkyle en C₂-C₄, phényle, 4'-aminophényle ou alcoxy(C₁-C₄)alkyle en C₁-C₄,
R₁₉ représente un atome d'hydrogène, un radical alkyle en C₁-C₄, monohydroxyalkyle en C₁-C₄ ou polyhydroxyalkyle en C₂-C₄,
R₂₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
R₂₁ représente un atome d'hydrogène ou un radical alkyle en C₁-C₄.

4. Composition selon la revendication 3, caractérisée par le fait que les paraphénylènediamines de formule (IV) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

5. Composition selon la revendication 2, caractérisée par le fait que les bis-phénylalkylènediamines sont choisies parmi les composés de formule (V) suivante, et leurs sels d'addition avec un acide : 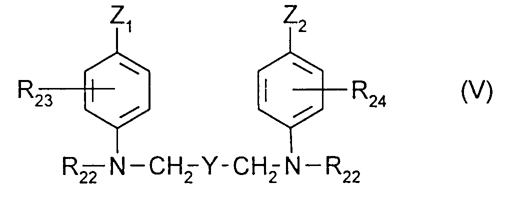 dans laquelle :
- Z₁ et Z₂, identiques ou différents, représentent un radical hydroxyle ou NHR₂₅ dans lequel R₂₅ représente un atome d'hydrogène ou un radical alkyle en C₁-C₄,
- R₂₂ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
- R₂₃ et R₂₄, identiques ou différents, représentent un atome d'hydrogène ou d'halogène ou un radical alkyle en C₁-C₄,
- Y représente un radical pris dans le groupe constitué par les radicaux suivants :
-(CH₂)ₙ-; -(CH₂)ₘ-O-(CH₂)ₘ; -(CH₂)ₘ-CHOH-(CH₂)ₘ et 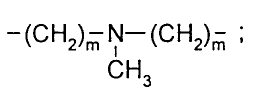
dans lesquels n est un nombre entier compris entre 0 et 8 inclusivement et m est un nombre entier compris entre 0 et 4 inclusivement.

6. Composition selon la revendication 5, caractérisée par le fait que les bis-phénylalkylènediamines de formules (V) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

7. Composition selon la revendication 2, caractérisée par le fait que les para-aminophénols sont choisis parmi les composés de formule (VI) suivante, et leurs sels d'addition avec un acide : 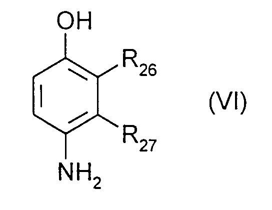 dans laquelle :
R₂₆ représente un atome d'hydrogène ou de fluor, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R₂₇ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₂₆ ou R₂₇ représente un atome d'hydrogène.

8. Composition selon la revendication 7, caractérisée par le fait que les para-aminophénols de formule (VI) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

9. Composition selon la revendication 2, caractérisée par le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

10. Composition selon la revendication 2, caractérisée par le fait que les bases hétérocycliques sont choisies parmi les dérivés pyridiniques, les dérivés pyrimidiniques, les dérivés pyrazoliques, et leurs sels d'addition avec un acide.

11. Composition selon l'une quelconque des revendications précédentes, caractérisée par le fait que les sels d'addition avec un acide sont choisis parmi les chlorhydrates, les bromhydrates, les sulfates et les tartrates.

12. Composition selon l'une quelconque des revendications précédentes, caractérisée par le fait que les colorants directs cationiques de formule (I) sont choisis parmi les composés répondant aux structures (I1) à (I52) suivantes :

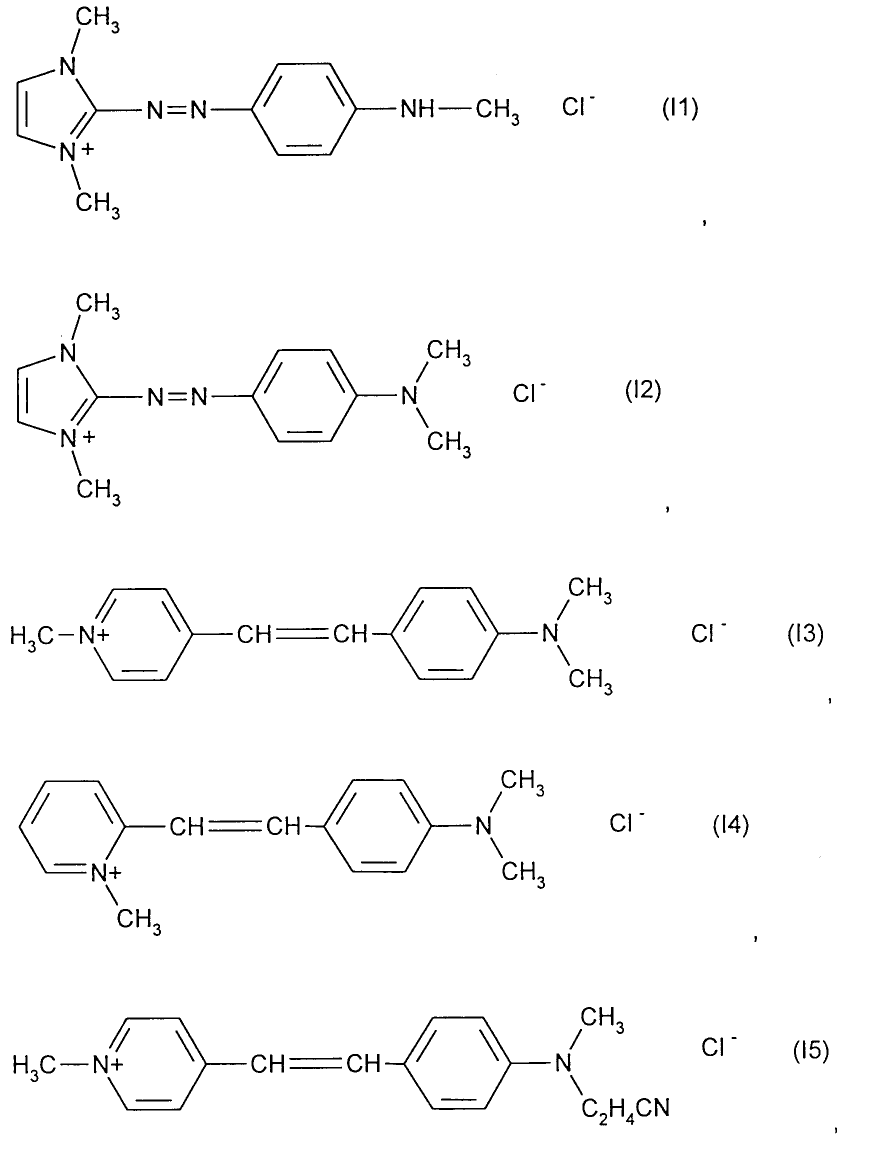

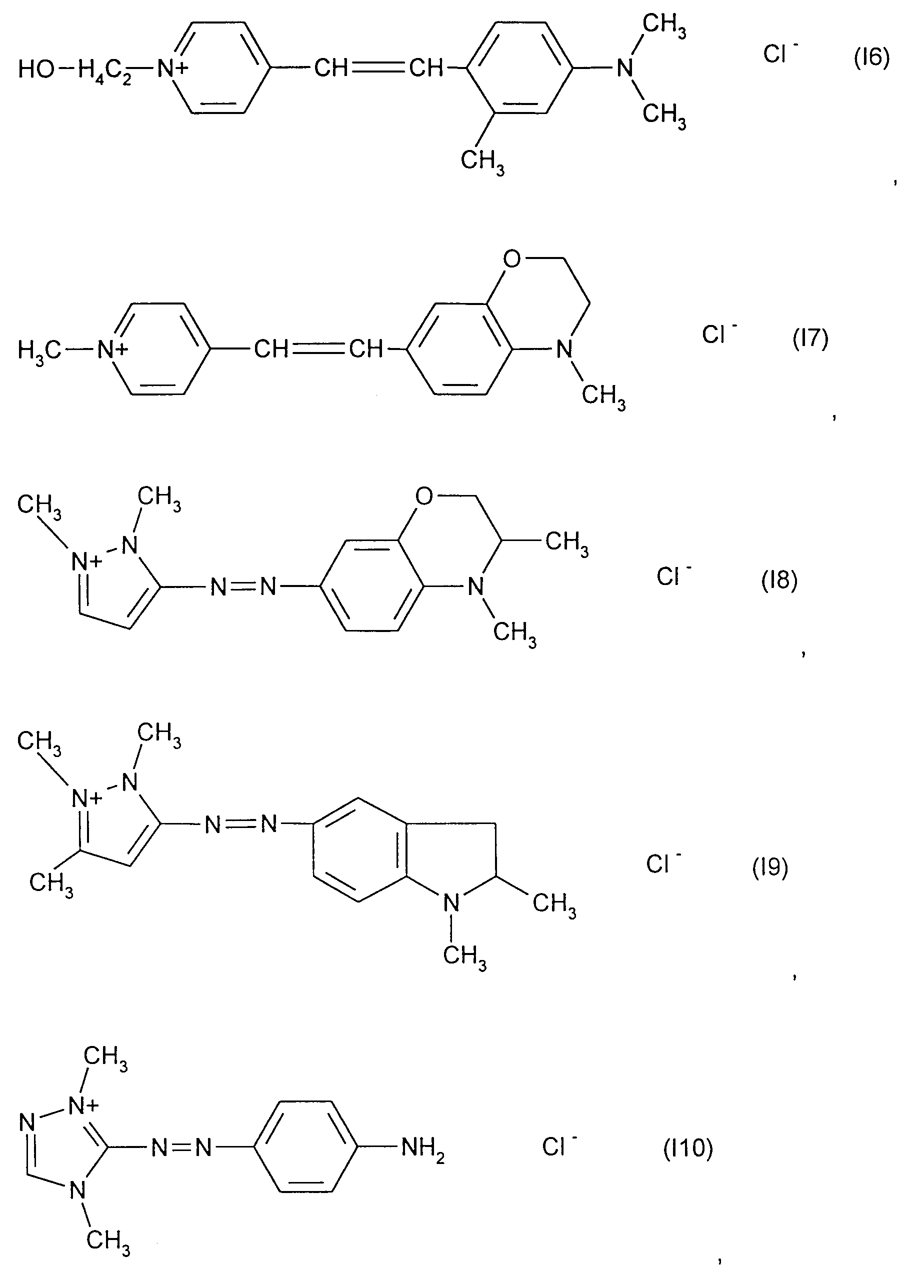

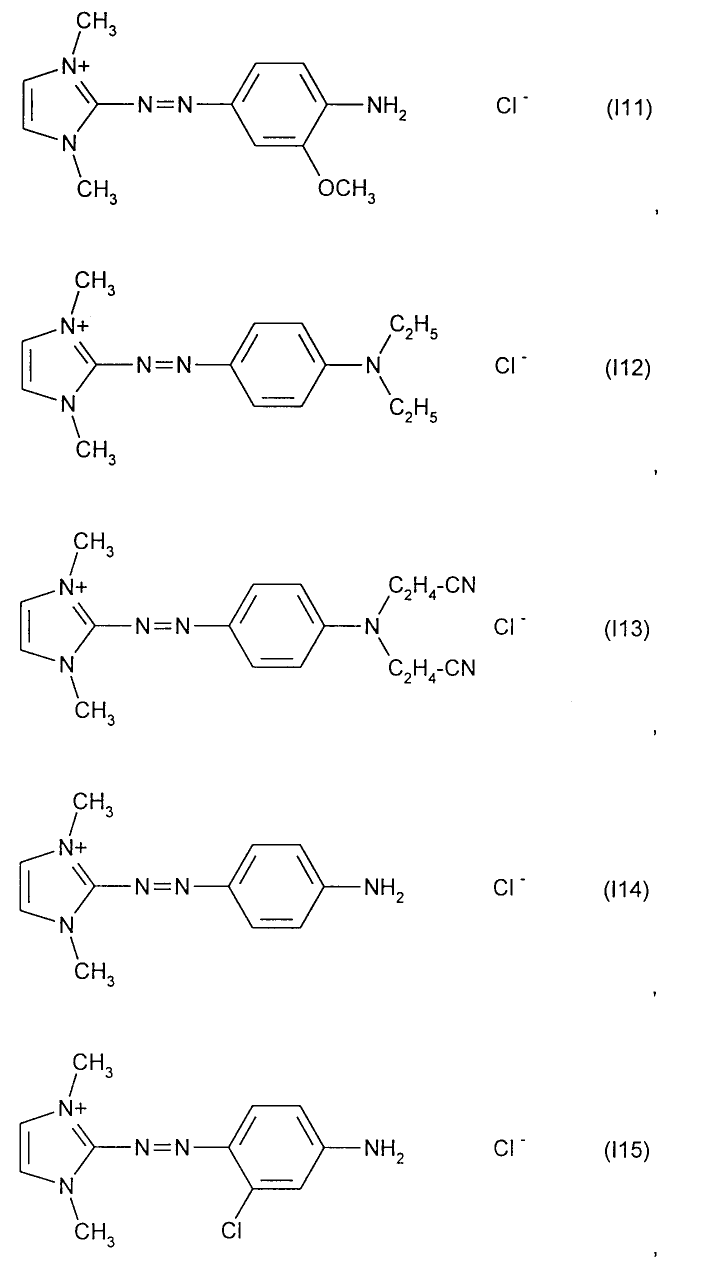

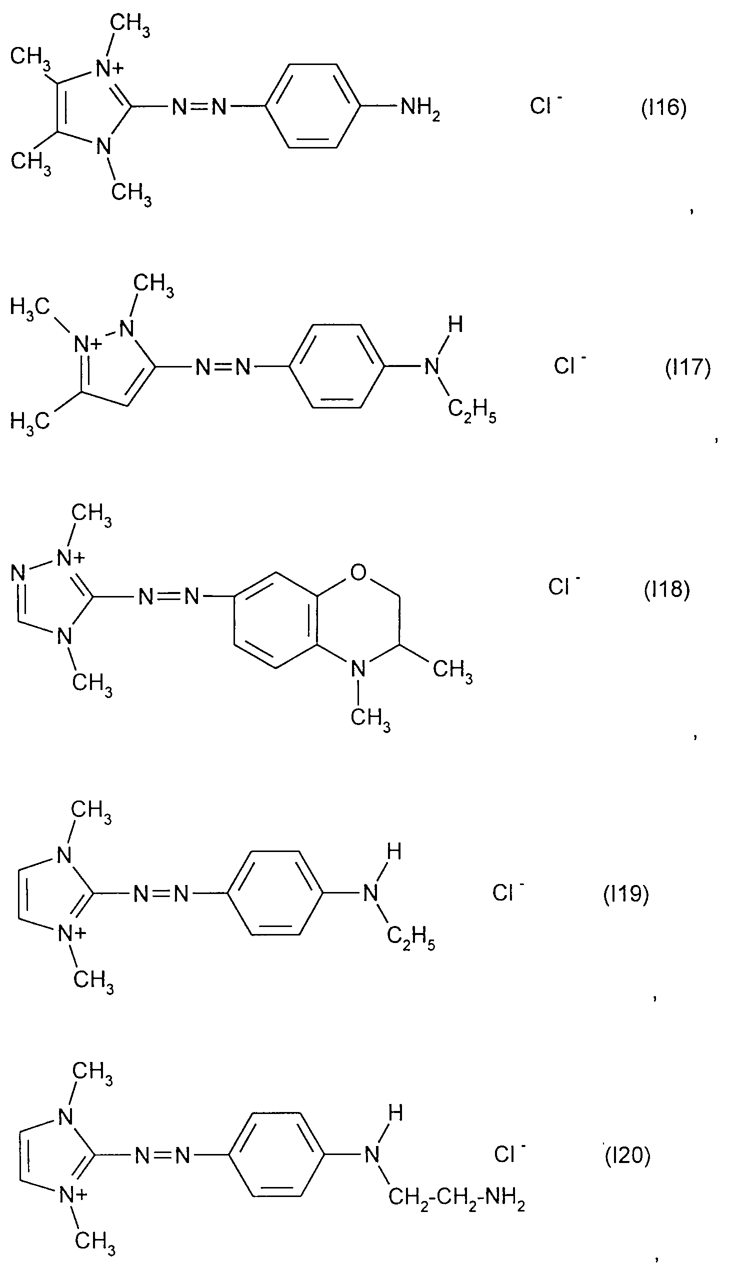

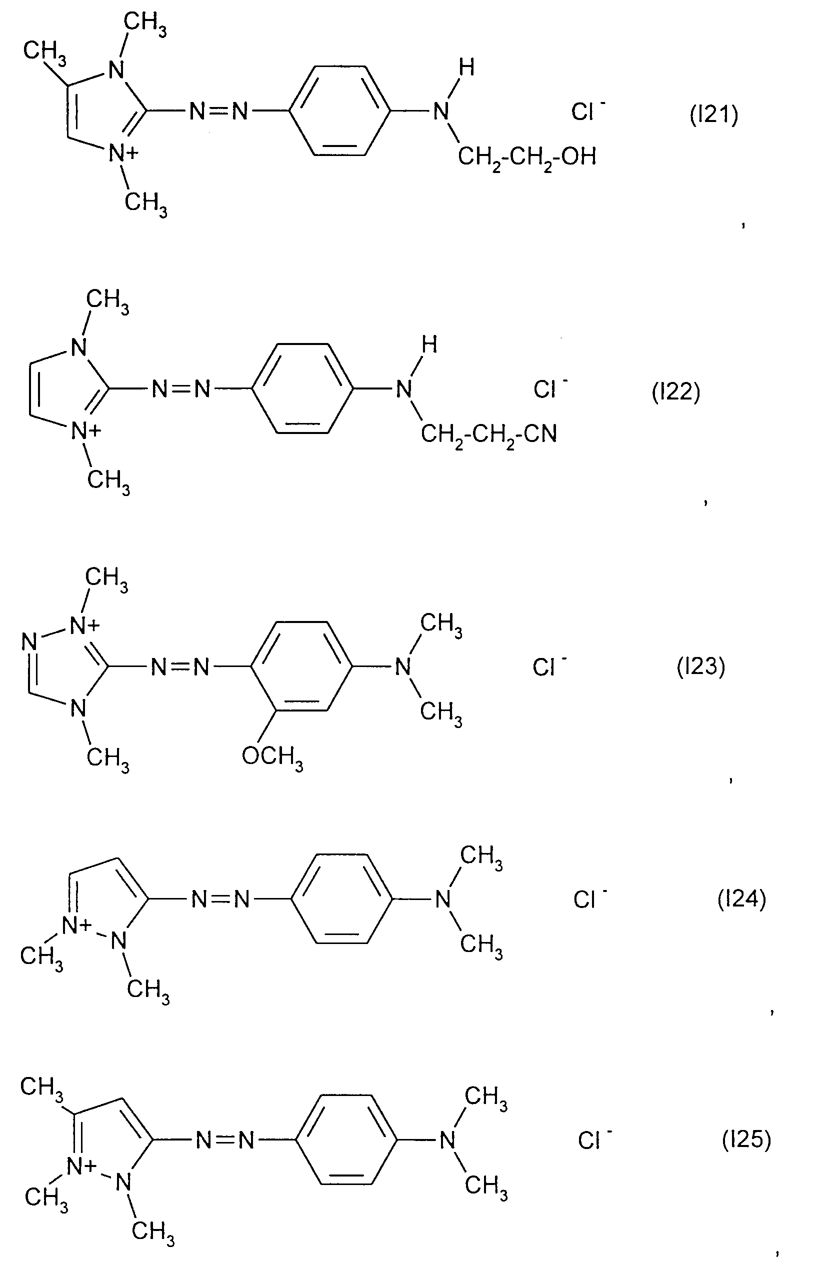

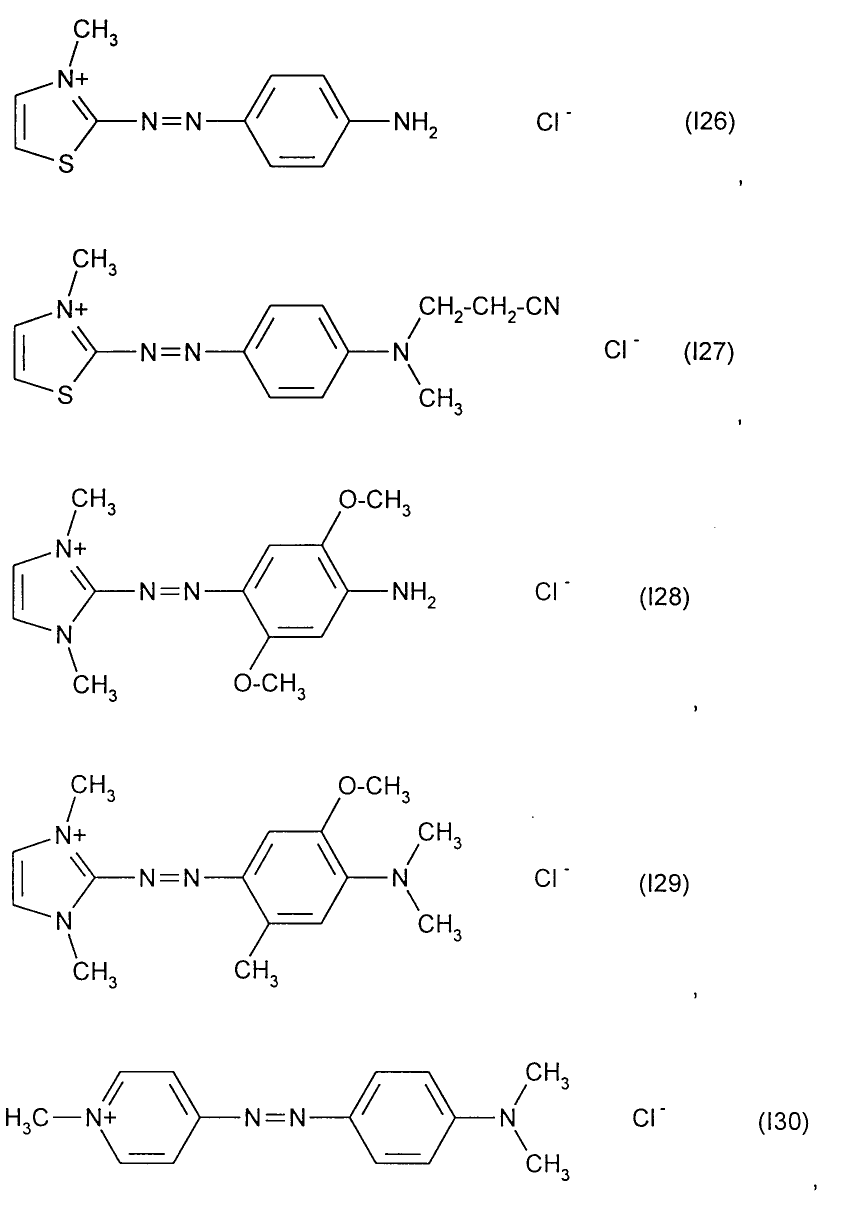

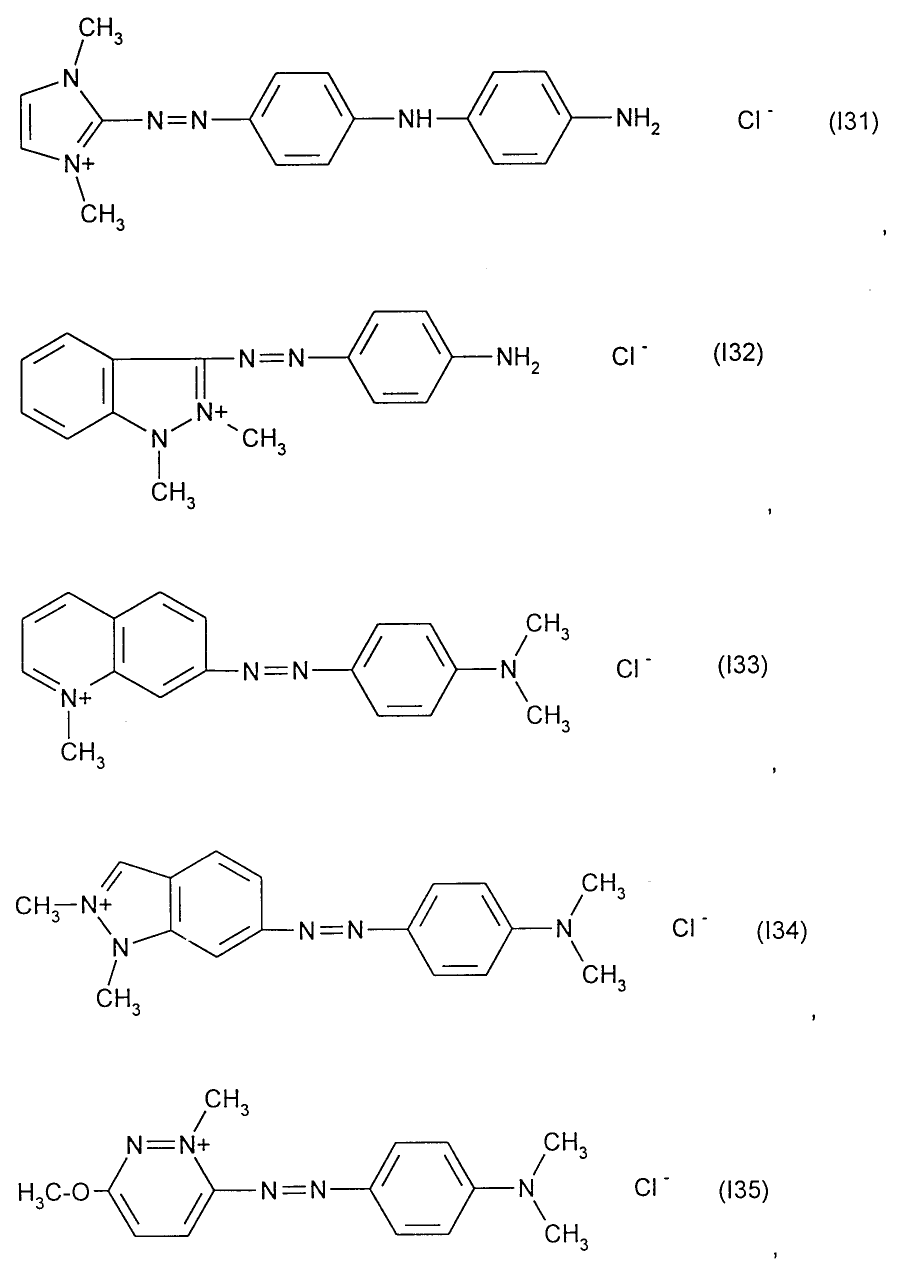

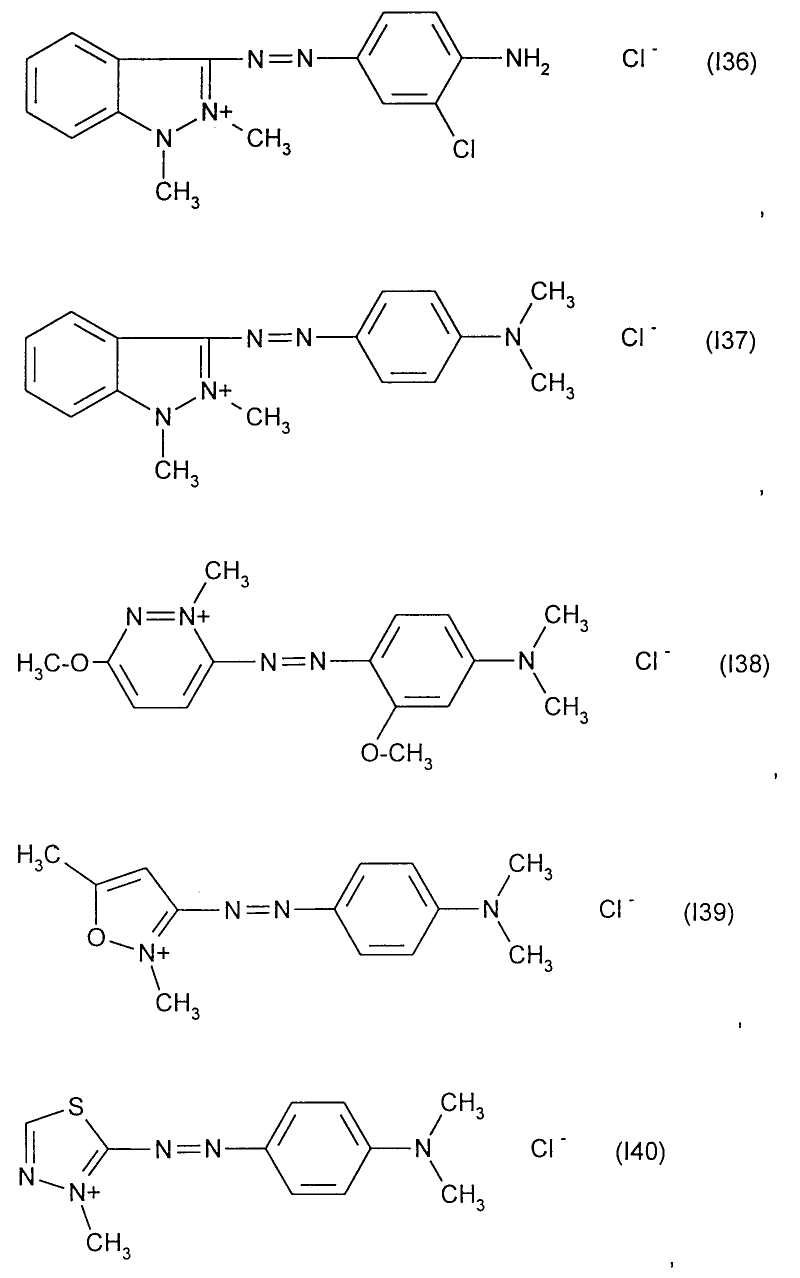

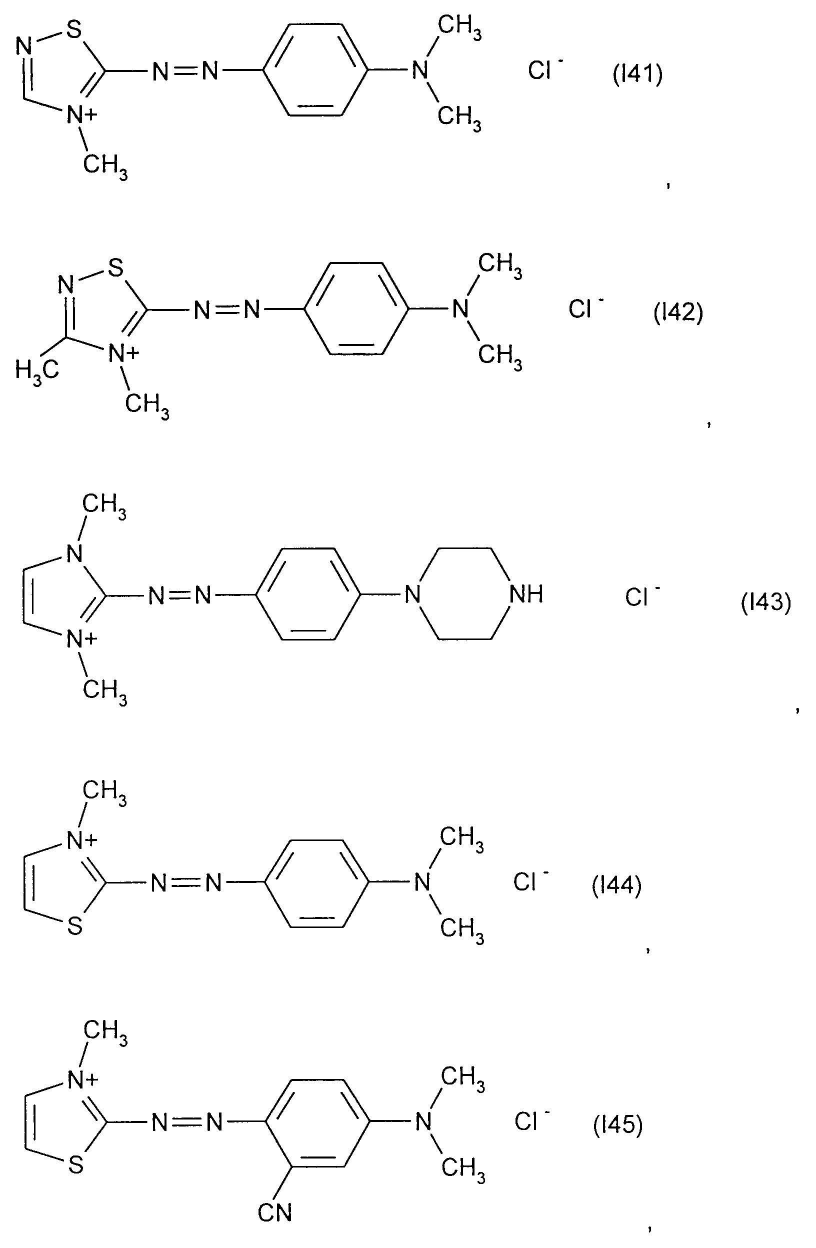

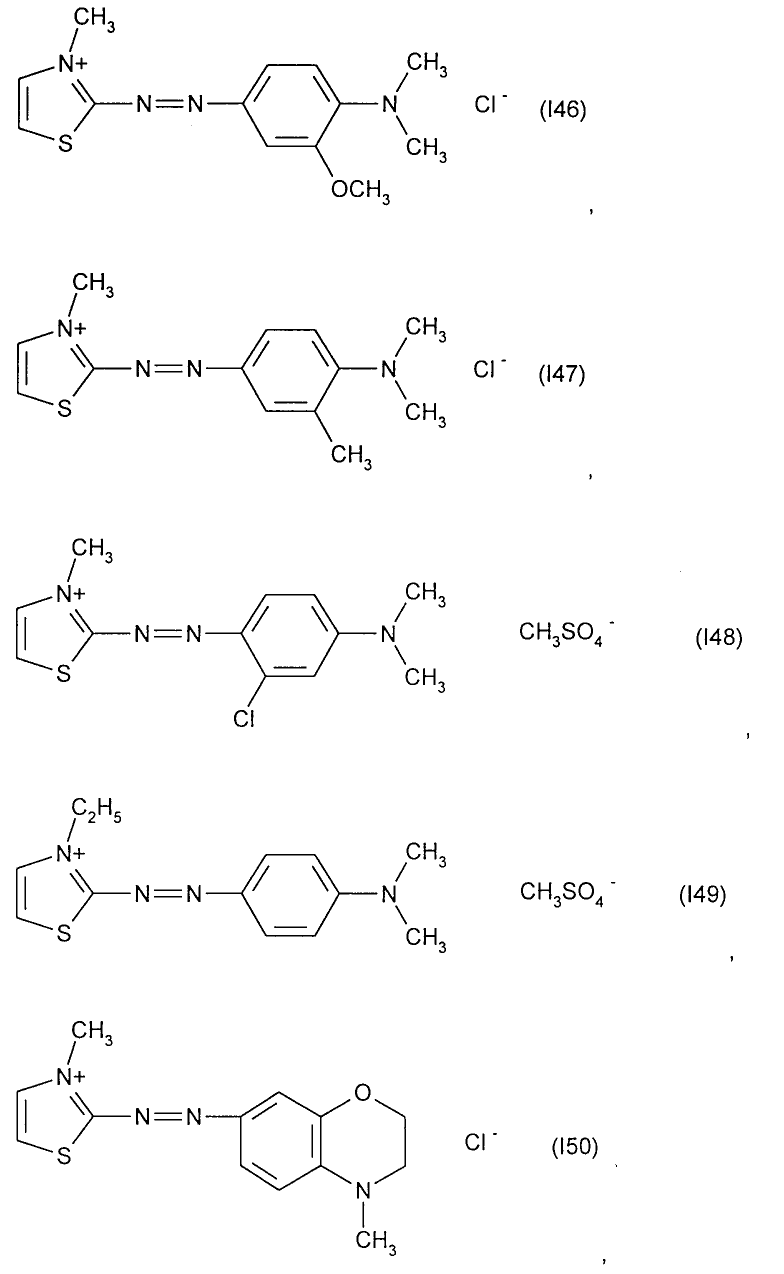

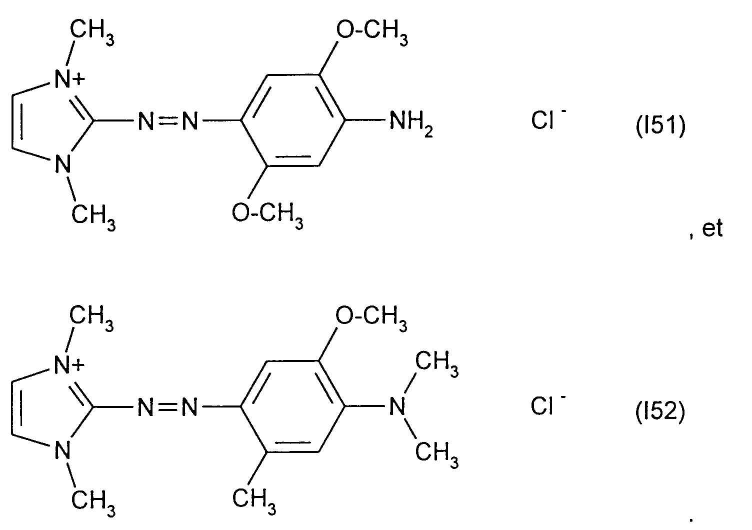

13. Composition selon l'une quelconque des revendications 1 à 11, caractérisée par le fait que les colorants directs cationiques de formule (II) sont choisis parmi les composés répondant aux structures (II1) à (II12) suivantes :

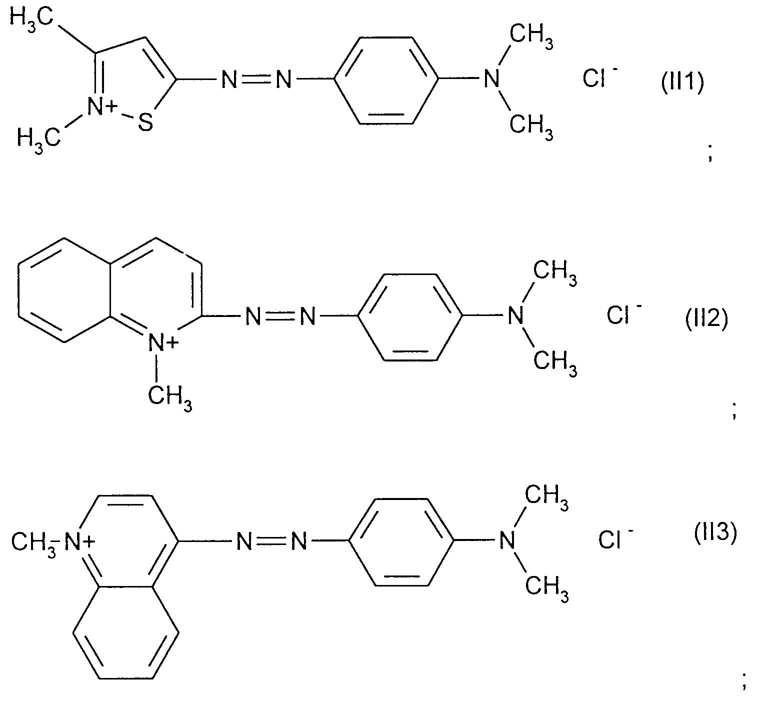

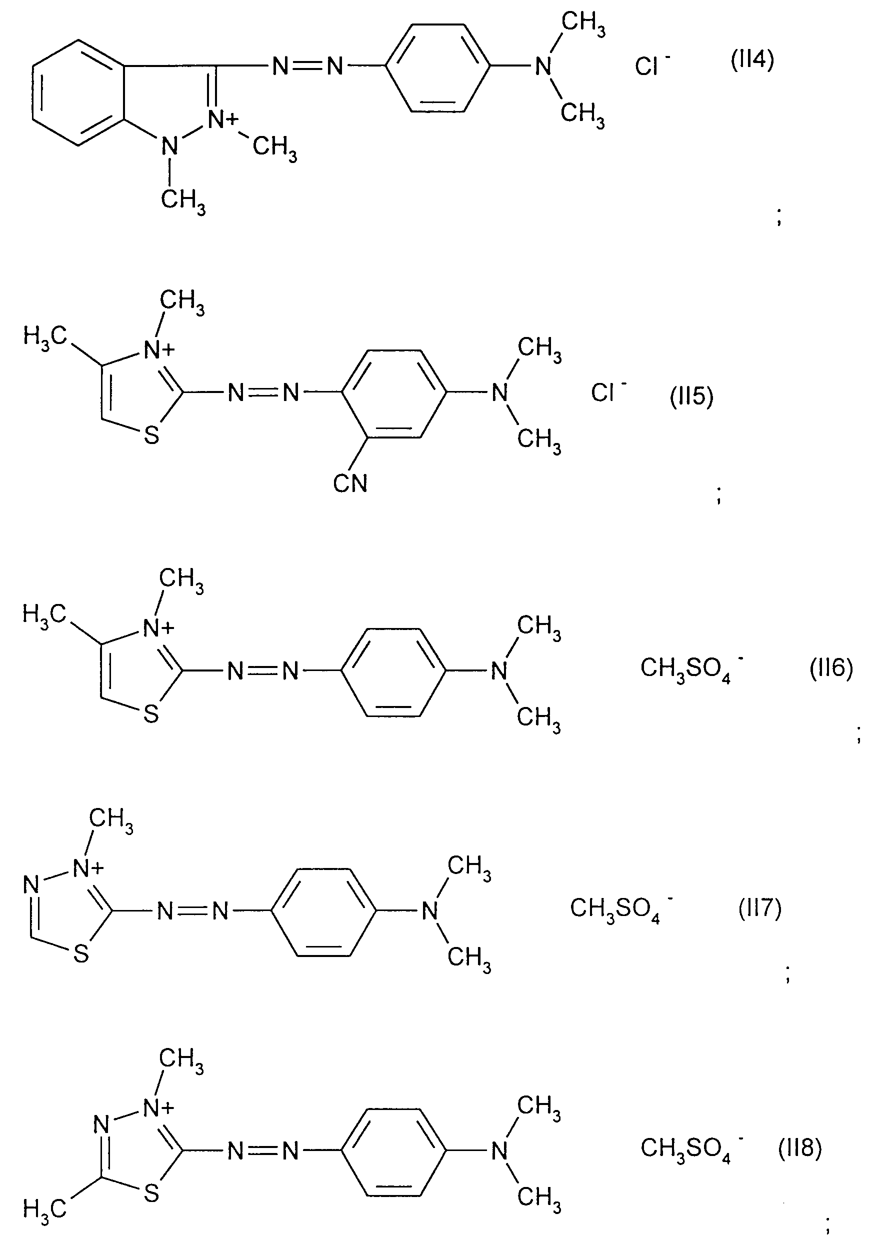

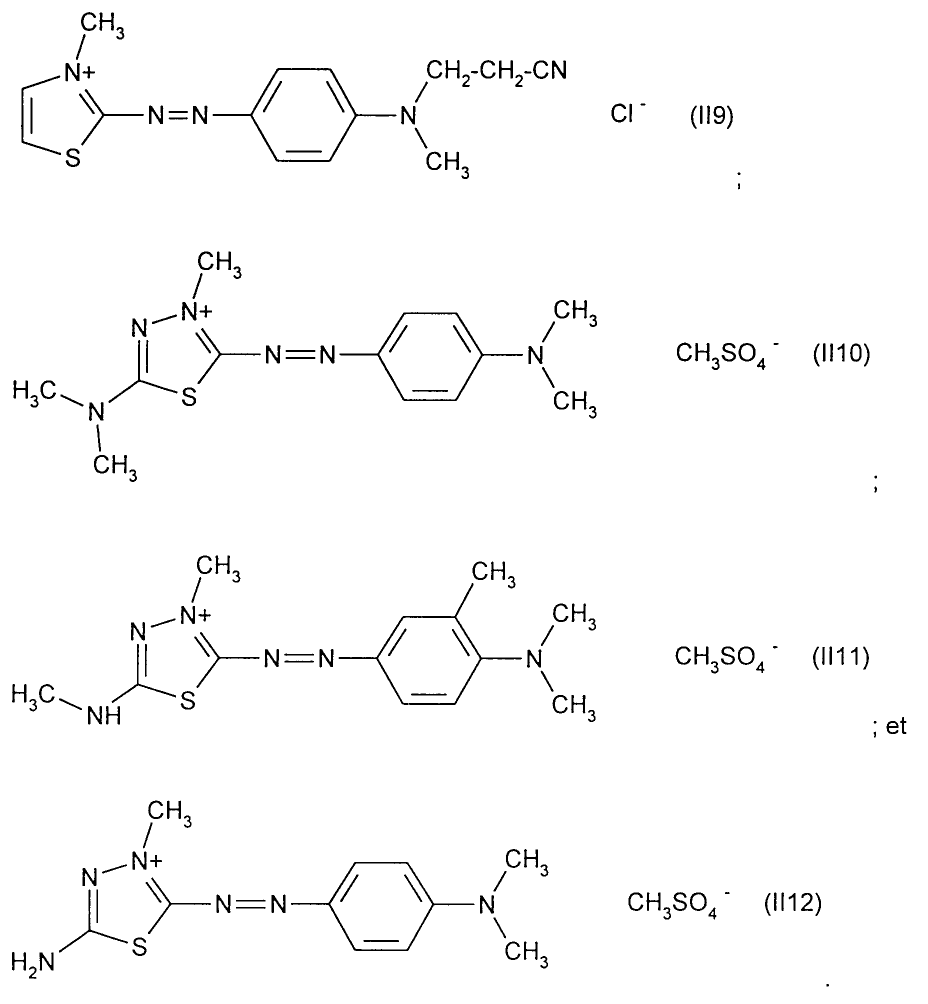

14. Composition selon l'une quelconque des revendications 1 à 11, caractérisée par le fait que les colorants directs cationiques de formule (III) sont choisis parmi les composés répondant aux structures (III1) à (III18) suivantes :

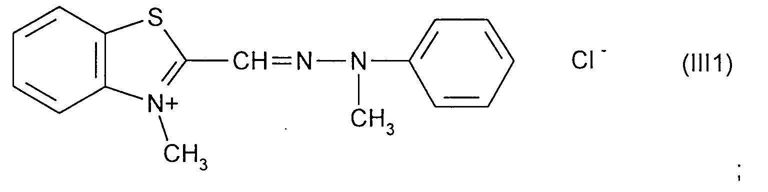

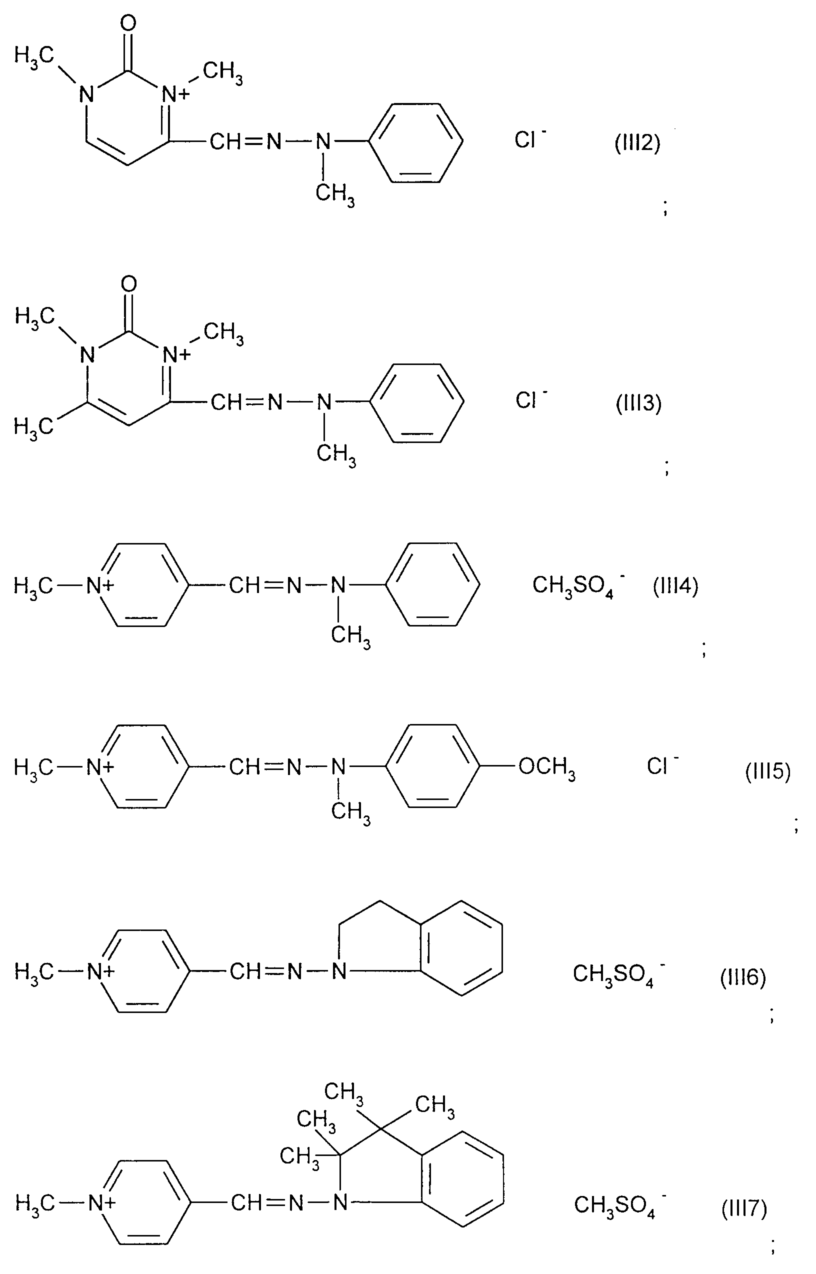

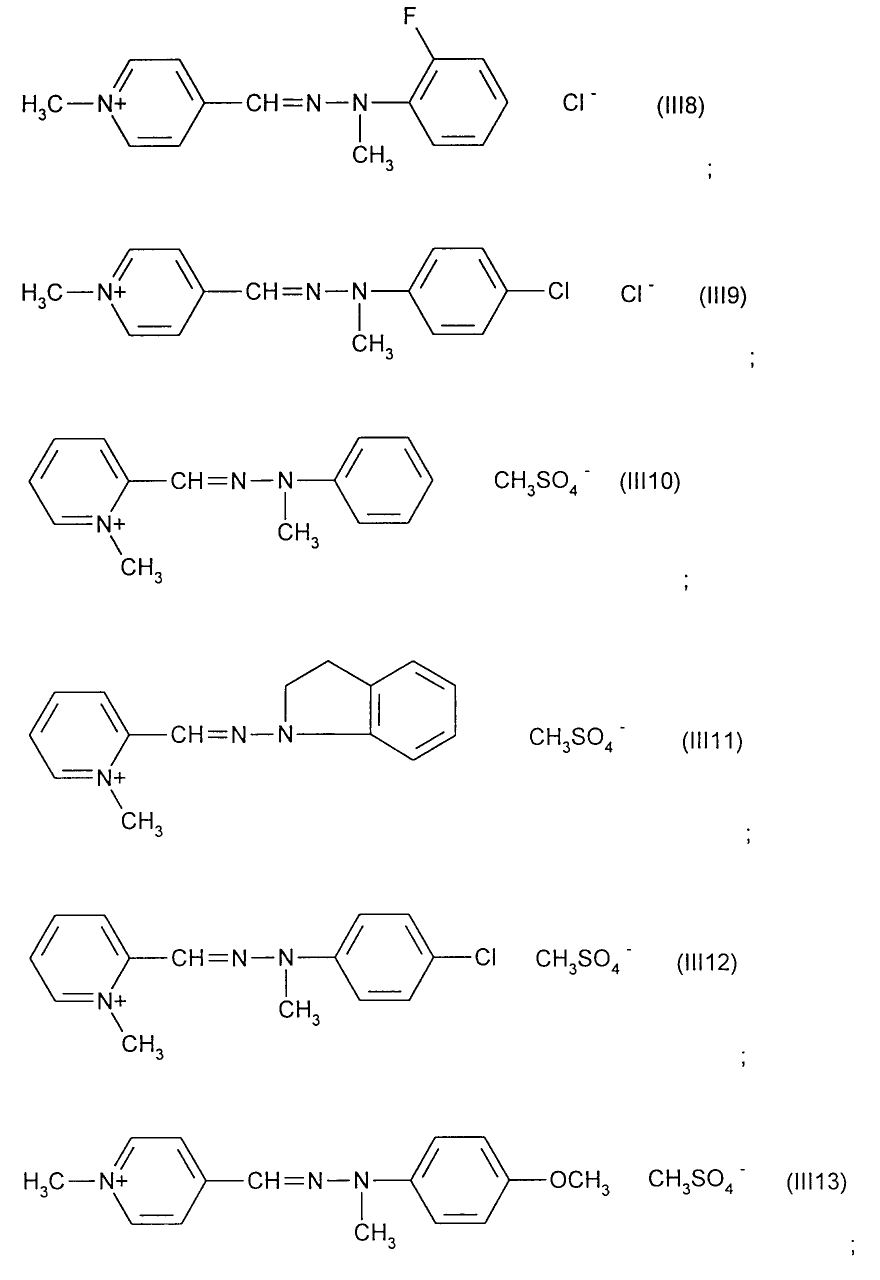

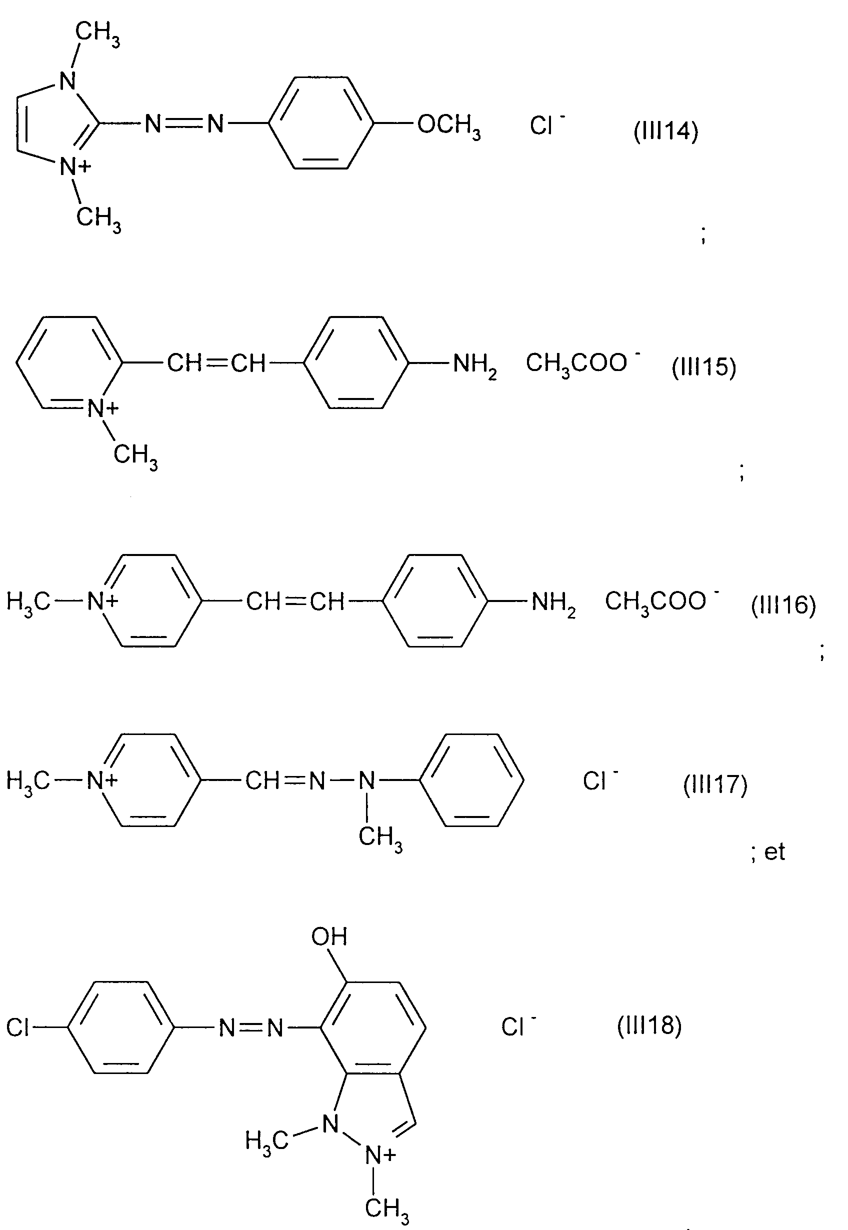

15. Composition selon l'une quelconque des revendications 1 à 11, caractérisée par le fait que les colorants directs cationiques de formule (III') sont choisis parmi les composés répondant aux structures (III'2) à (III'3) suivantes :

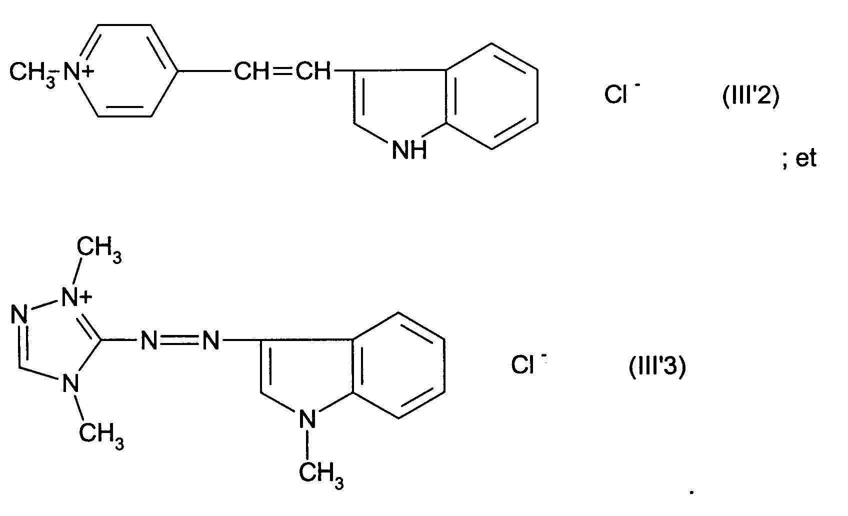

16. Composition selon l'une quelconque des revendications précédentes, caractérisée par le fait que l'agent oxydant est choisi parmi le peroxyde d'hydrogène, le peroxyde d'urée, les bromates de métaux alcalins, les persels tels que les perborates et persulfates.

17. Composition selon l'une quelconque des revendications précédentes, caractérisée par le fait que la ou les bases d'oxydation représentent de 0,0001 à 10 % en poids du poids total de la composition tinctoriale prête à l'emploi.

18. Composition selon l'une quelconque des revendications précédentes, caractérisée par le fait que le ou les colorants directs cationiques de formules (I) et/ou (II) et/ou (III) et/ou (III') représentent de 0,001 à 10 % en poids du poids total de la composition tinctoriale prête à l'emploi.

19. Composition selon l'une quelconque des revendications précédentes, caractérisée par le fait qu'elle présente un pH compris entre 5 et 12.

20. Composition selon l'une quelconque des revendications précédentes, caractérisée par le fait que le milieu approprié pour la teinture est constitué par de l'eau ou par un mélange d'eau et d'au moins un solvant organique.

21. Procédé de teinture des fibres kératiniques et en particulier des fibres kératiniques humaines telles que les cheveux caractérisé par le fait que l'on applique sur ces fibres au moins une composition tinctoriale telle que définie dans l'une quelconque des revendications 1 à 20.

22. Procédé selon la revendication 21, caractérisé par le fait qu'il comporte une étape préliminaire consistant à stocker sous forme séparée, d'une part, une composition (A) comprenant, dans un milieu approprié pour la teinture, au moins une base d'oxydation et au moins un colorant direct cationique choisi parmi les composés de formules (I), (II), (III) et (III') tels que définis dans la revendication 1, et d'autre part, une composition (B) renfermant, dans un milieu approprié pour la teinture, au moins un agent oxydant, et à procéder à leur mélange au moment de l'emploi avant d'appliquer ce mélange sur les fibres kératiniques.

23. Procédé de teinture selon la revendication 21, caractérisé par le fait qu'il comporte une étape préliminaire consistant à stocker sous forme séparée, d'une part, une composition (A) comprenant, dans un milieu approprié pour la teinture au moins une base d'oxydation ; d'autre part une composition (A') comprenant, dans un milieu approprié pour la teinture, au moins un colorant direct cationique choisi parmi les composés de formules (I), (II), (III) et (III') tels que définis dans la revendication 1 ; et enfin, une composition (B) renfermant, dans un milieu approprié pour la teinture, au moins un agent oxydant et à procéder à leur mélange au moment de l'emploi avant d'appliquer ce mélange sur les fibres kératiniques.

24. Procédé selon la revendication 23, caractérisé par le fait que la composition (A') se présente sous forme de poudre.

25. Dispositif à plusieurs compartiments ou "kit" de teinture, caractérisé par le fait qu'un premier compartiment renferme la composition (A) telle que définie à la revendication 22 et un second compartiment renferme une composition oxydante (B).

26. Dispositif à plusieurs compartiments ou "kit" de teinture, caractérisé par le fait qu'un premier compartiment renferme une composition (A) telle que définie à la revendication 23, un second compartiment referme une composition (A') telle que définie à la revendication 23 ou 24 et un troisième compartiment renferme une composition oxydante (B).

## Claims

1. Ready-to-use composition for the oxidation dyeing of keratin fibres, and in particular human keratin fibres such as the hair, characterized in that it comprises, in a medium which is suitable for dyeing:
- at least one oxidation base,
- at least one cationic direct dye chosen from:
a) the compounds of formula (I) below: 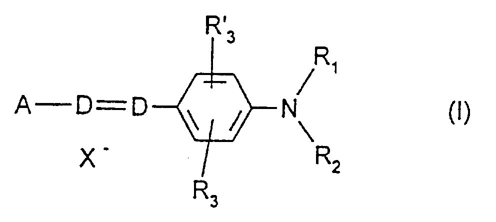 in which:
D represents a nitrogen atom or the -CH group,
R₁ and R₂, which may be identical or different, represent a hydrogen atom; a C₁-C₄ alkyl radical which may be substituted with a -CN, -OH or -NH₂ radical, or form, with a carbon atom of the benzene ring, a heterocycle which is optionally oxygenated or nitrogenous, which may be substituted with one or more C₁-C₄ alkyl radicals; a 4'-aminophenyl radical,
R₃ and R'₃, which may be identical or different, represent a hydrogen or halogen atom chosen from chlorine, bromine, iodine and fluorine, or a cyano, C₁-C₄ alkoxy or acetyloxy radical,
X⁻ represents an anion preferably chosen from chloride, methylsulphate and acetate,
A represents a group chosen from the structures A1 to A19 below: 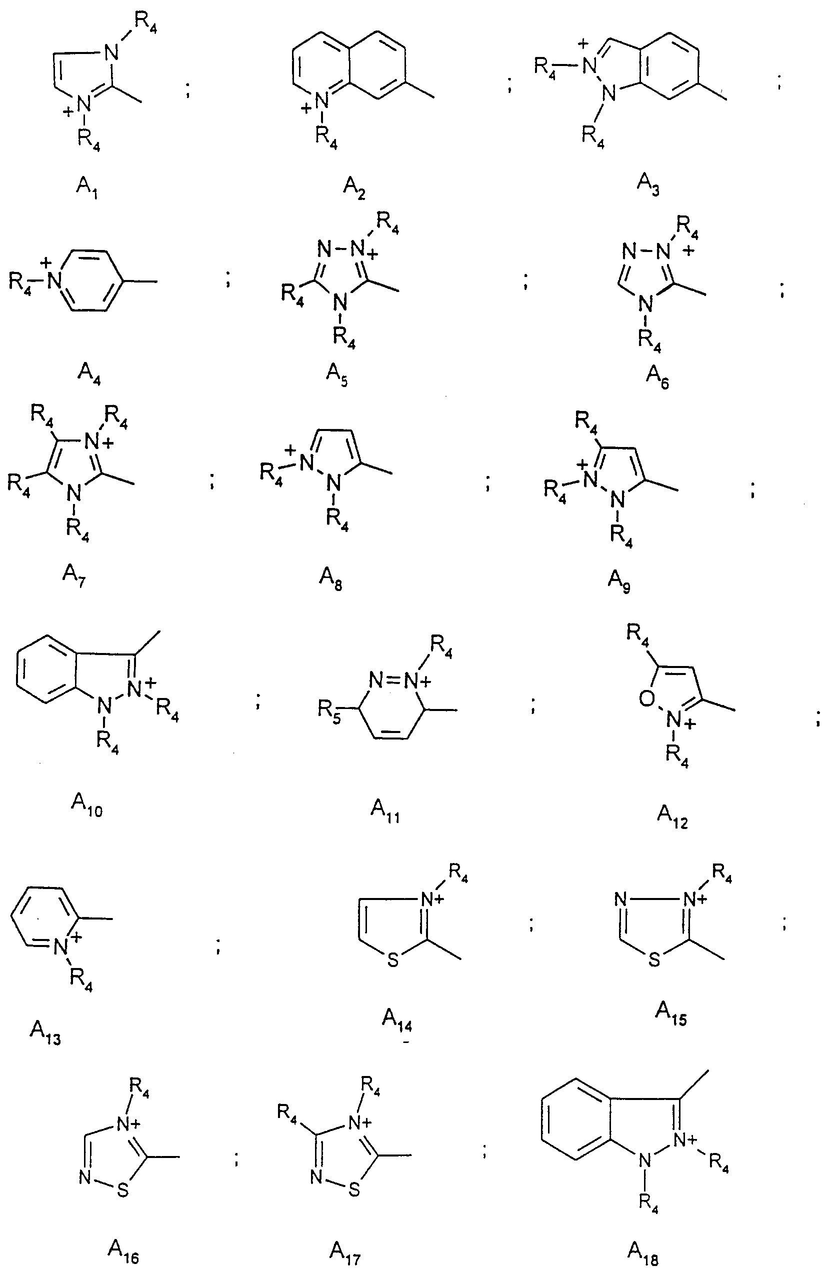 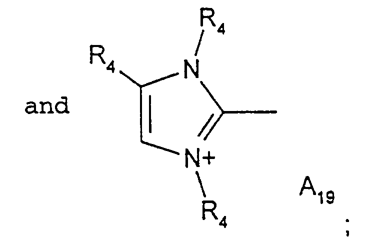
in which R₄ represents a C₁-C₄ alkyl radical which may be substituted with a hydroxyl radical and R₅ represents a C₁-C₄ alkoxy radical, with the proviso that when D represents -CH, A represents A₄ or A₁₃ and R₃ is other than an alkoxy radical, then R₁ and R₂ do not simultaneously denote a hydrogen atom;
b) the compounds of formula (II) below: 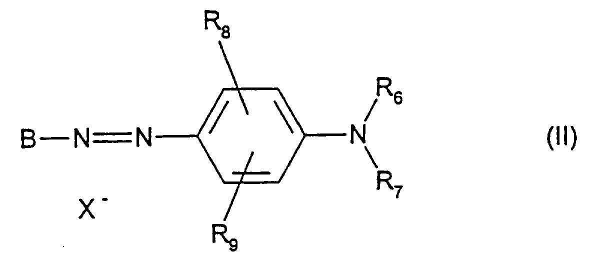 in which:
R₆ represents a hydrogen atom or a C₁-C₄ alkyl radical,
R₇ represents a hydrogen atom, an alkyl radical which may be substituted with a -CN radical or with an amino group, a 4'-aminophenyl radical, or forms with R₆ a heterocycle which is optionally oxygenated and/or nitrogenous, which may be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methylsulphate and acetate,
B represents a group chosen from the structures B1 to B6 below: 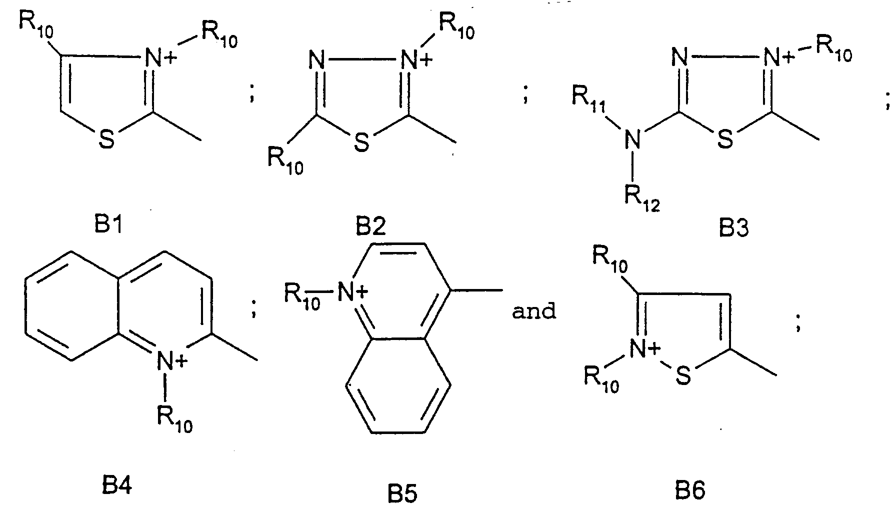
in which R₁₀ represents a C₁-C₄ alkyl radical, R₁₁ and R₁₂, which may be identical or different, represent a hydrogen atom or a C₁-C₄ alkyl radical;
c) the compound of formula (III'1) below: 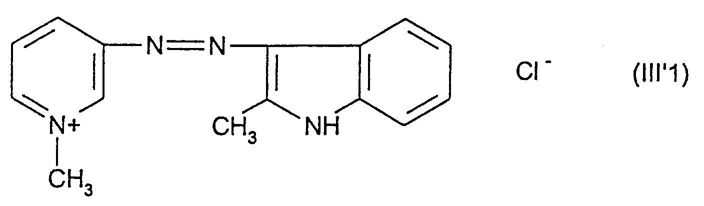 and the compounds of formulae (III) and (III') below: 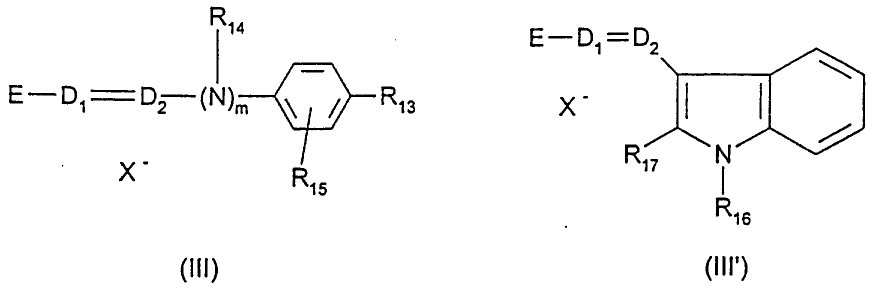 in which:
R₁₃ represents a hydrogen atom, a C₁-C₄ alkoxy radical, a halogen atom such as bromine, chlorine, iodine or fluorine, or an amino radical,
R₁₄ represents a hydrogen atom, a C₁-C₄ alkyl radical or forms, with a carbon atom of the benzene ring, a heterocycle which is optionally oxygenated and/or substituted with one or more C₁-C₄ alkyl groups,
R₁₅ represents a hydrogen or halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methylsulphate and acetate,
E represents a group chosen from the structures E1 to E7 below: 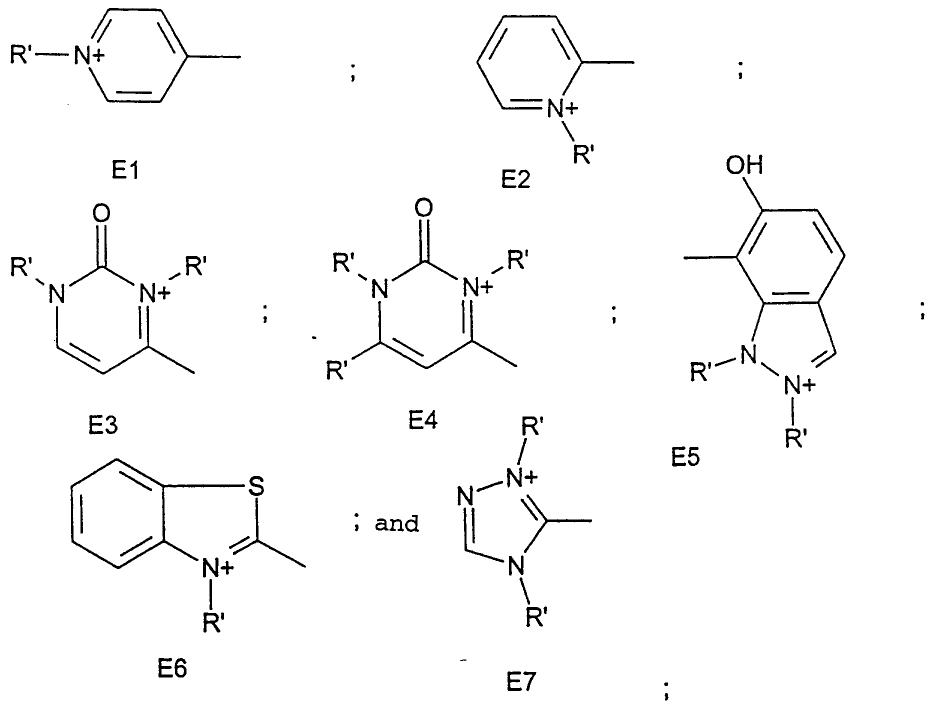
in which R' represents a C₁-C₄ alkyl radical;
when m = 0 and D₁ represents a nitrogen atom, then E can also denote a group of structure E8 below: 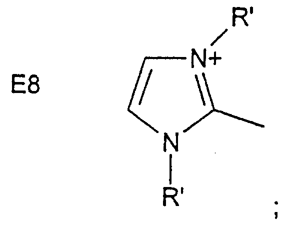
in which R' represents a C₁-C₄ alkyl radical; and
- at least one oxidizing agent.

2. Composition according to Claim 1, characterized in that the oxidation base(s) is (are) chosen from para-phenylenediamines, bis(phenyl)alkylenediamines, para-aminophenols, ortho-aminophenols and heterocyclic oxidation bases.

3. Composition according to Claim 2, characterized in that the para-phenylenediamines are chosen from the compounds of formula (IV) below, and the addition salts thereof with an acid: 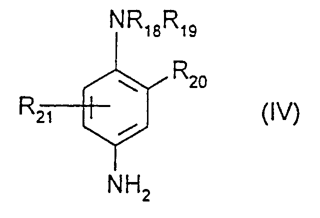 in which:
R₁₈ represents a hydrogen atom or a C₁-C₄ alkyl, C₁-C₄ monohydroxyalkyl, C₂-C₄ polyhydroxyalkyl, phenyl, 4'-aminophenyl or (C₁-C₄)alkoxy(C₁-C₄)alkyl radical,
R₁₉ represents a hydrogen atom or a C₁-C₄ alkyl, C₁-C₄ monohydroxyalkyl or C₂-C₄ polyhydroxyalkyl radical,
R₂₀ represents a hydrogen atom, a halogen atom such as a chlorine, bromine, iodine or fluorine atom, or a C₁-C₄ alkyl, C₁-C₄ monohydroxyalkyl, C₁-C₄ hydroxyalkoxy, C₁-C₄ acetylaminoalkoxy, C₁-C₄ mesylaminoalkoxy or C₁-C₄ carbamoylaminoalkoxy radical,
R₂₁ represents a hydrogen atom or a C₁-C₄ alkyl radical.

4. Composition according to Claim 3, characterized in that the para-phenylenediamines of formula (IV) are chosen from para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-bis(β-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine and 2-β-acetylaminoethyloxy-para-phenylenediamine, and the addition salts thereof with an acid.

5. Composition according to Claim 2, characterized in that the bis(phenyl)alkylenediamines are chosen from the compounds of formula (V) below, and the addition salts thereof with an acid: 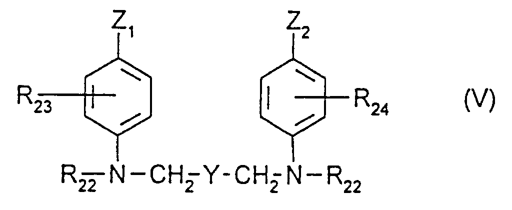 in which:
Z₁ and Z₂, which may be identical or different, represent a hydroxyl radical or NHR₂₅ in which R₂₅ represents a hydrogen atom or a C₁-C₄ alkyl radical,
R₂₂ represents a hydrogen atom or a C₁-C₄ alkyl, C₁-C₄ monohydroxyalkyl or C₂-C₄ polyhydroxyalkyl radical or a C₁-C₄ aminoalkyl radical in which the amino residue can be substituted,
R₂₃ and R₂₄, which may be identical or different, represent a hydrogen or halogen atom or a C₁-C₄ alkyl radical,
Y represents a radical taken from the group consisting of the following radicals:
-(CH₂)ₙ- ; -(CH₂)ₘ-O-(CH₂)ₘ; -(CH₂)ₘ-CHOH-(CH₂)ₘ and 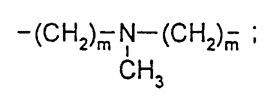
in which n is an integer between 0 and 8 inclusive and m is an integer between 0 and 4 inclusive.

6. Composition according to Claim 5, characterized in that the bis(phenyl)alkylenediamines of formula (V) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine, and the addition salts thereof with an acid.

7. Composition according to Claim 2, characterized in that the para-aminophenols are chosen from the compounds of formula (VI) below, and the addition salts thereof with an acid: 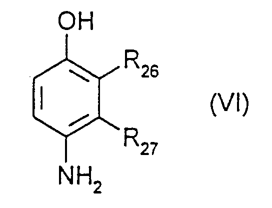 in which:
R₂₆ represents a hydrogen or fluorine atom or a C₁-C₄ alkyl, C₁-C₄ monohydroxyalkyl, (C₁-C₄)alkoxy(C₁-C₄)alkyl, C₁-C₄ aminoalkyl or hydroxy(C₁-C₄)alkylamino(C₁-C₄)alkyl radical,
R₂₇ represents a hydrogen or fluorine atom or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical,
it being understood that at least one of the radicals R₂₆ or R₂₇ represents a hydrogen atom.

8. Composition according to Claim 7, characterized in that the para-aminophenols of formula (VI) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

9. Composition according to Claim 2, characterized in that the ortho-aminophenols are chosen from 2-aminophenol, 2-amino-5-methylphenol, 2-amino-6-methylphenol and 5-acetamido-2-aminophenol, and the addition salts thereof with an acid.

10. Composition according to Claim 2, characterized in that the heterocyclic bases are chosen from pyridine derivatives, pyrimidine derivatives and pyrazole derivatives, and the addition salts thereof with an acid.

11. Composition according to any one of the preceding claims, characterized in that the addition salts with an acid are chosen from the hydrochlorides, hydrobromides, sulphates and tartrates.

12. Composition according to any one of the preceding claims, characterized in that the cationic direct dyes of formula (I) are chosen from the compounds corresponding to the structures (I1) to (I52) below:

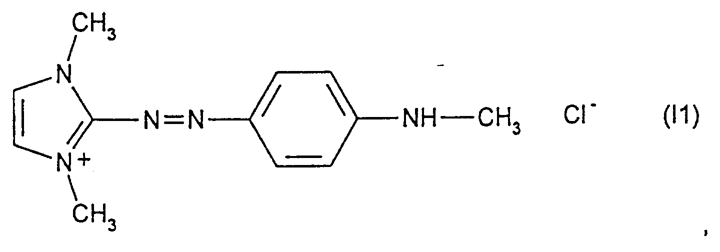

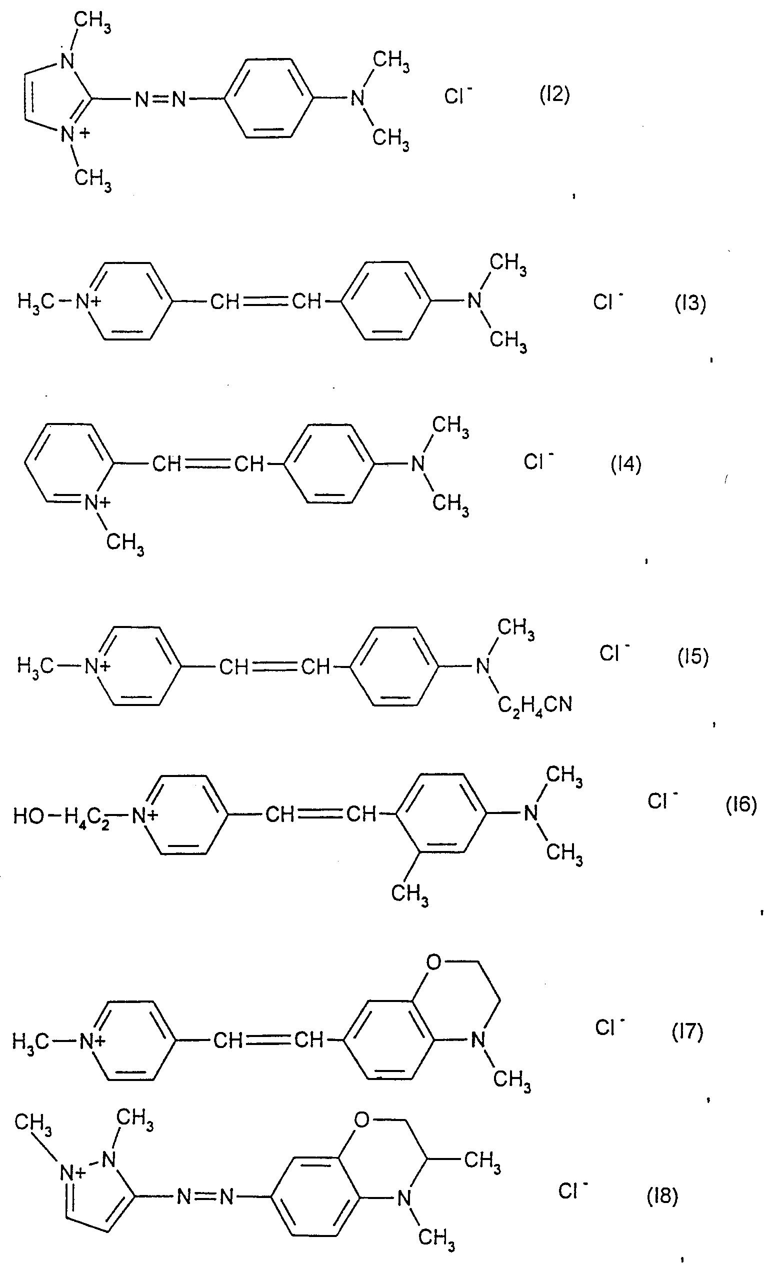

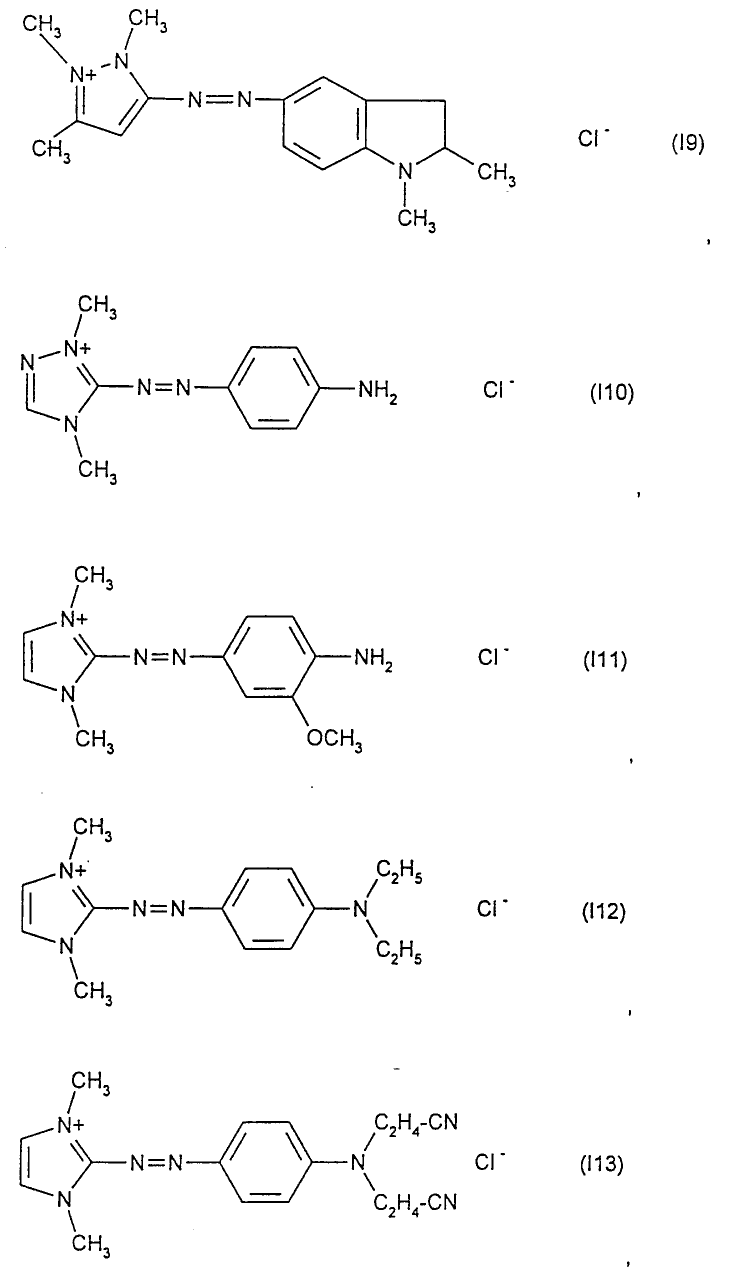

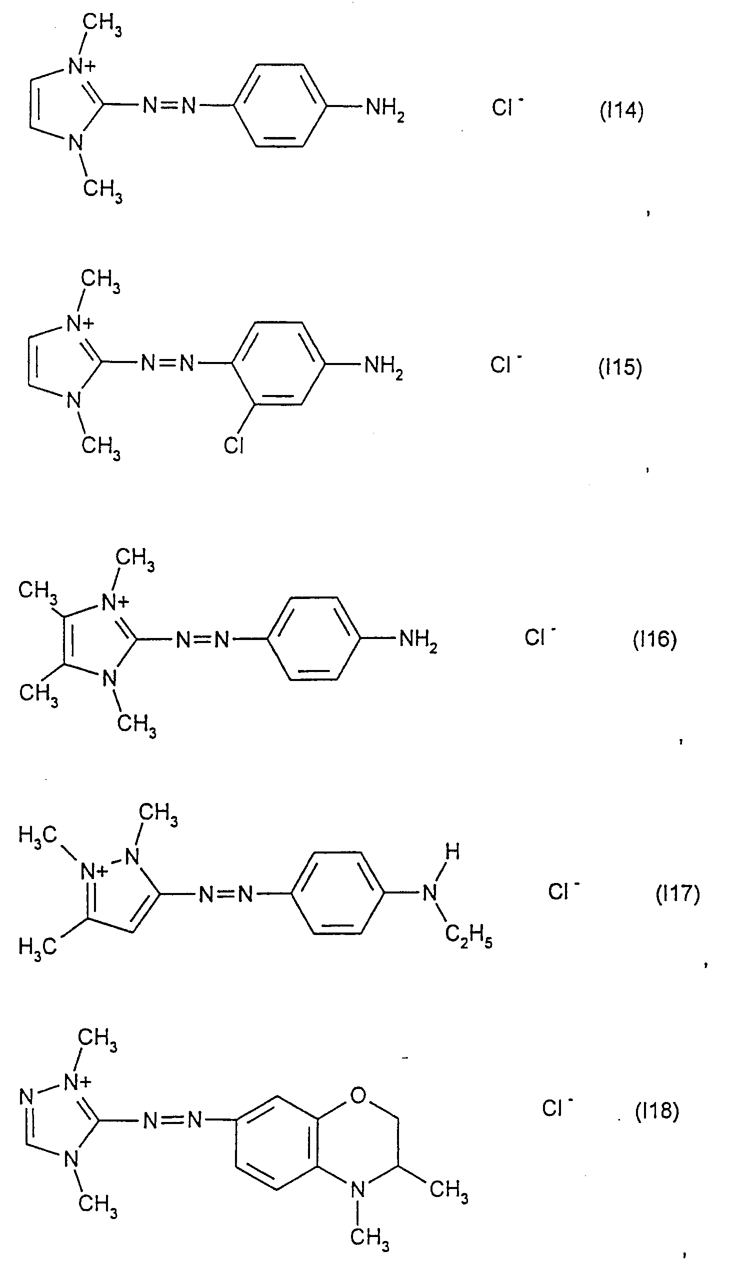

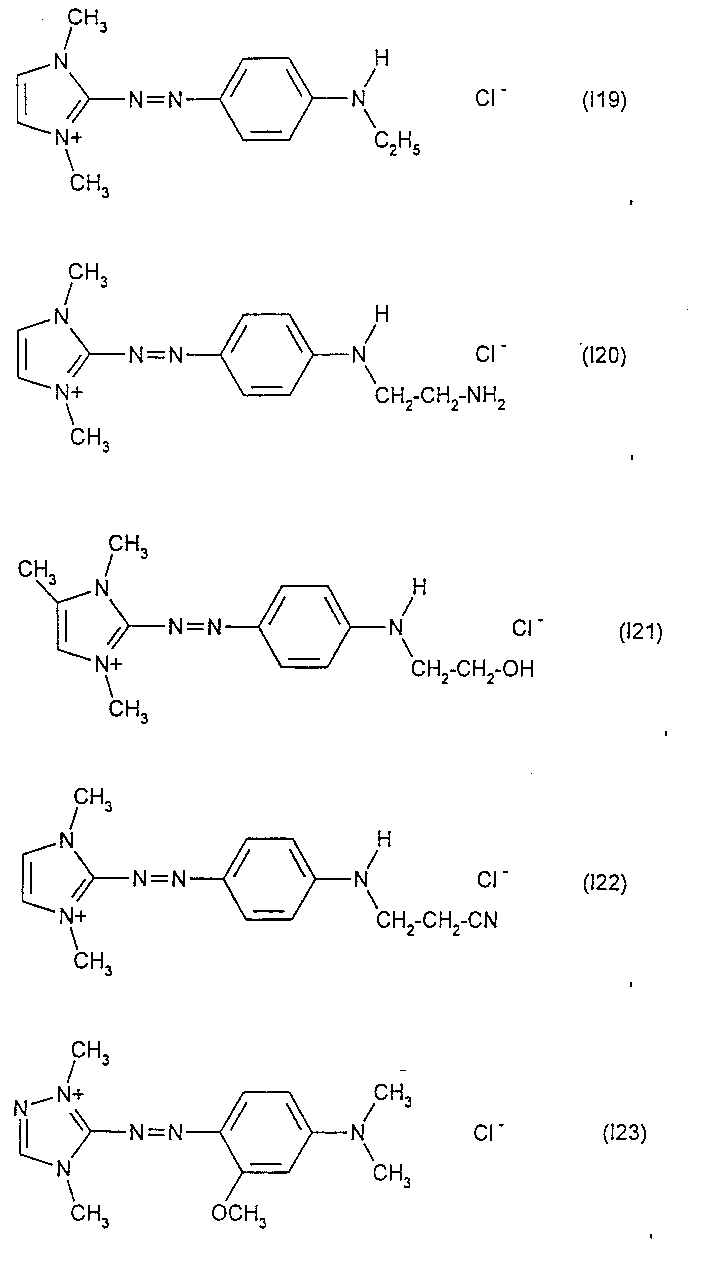

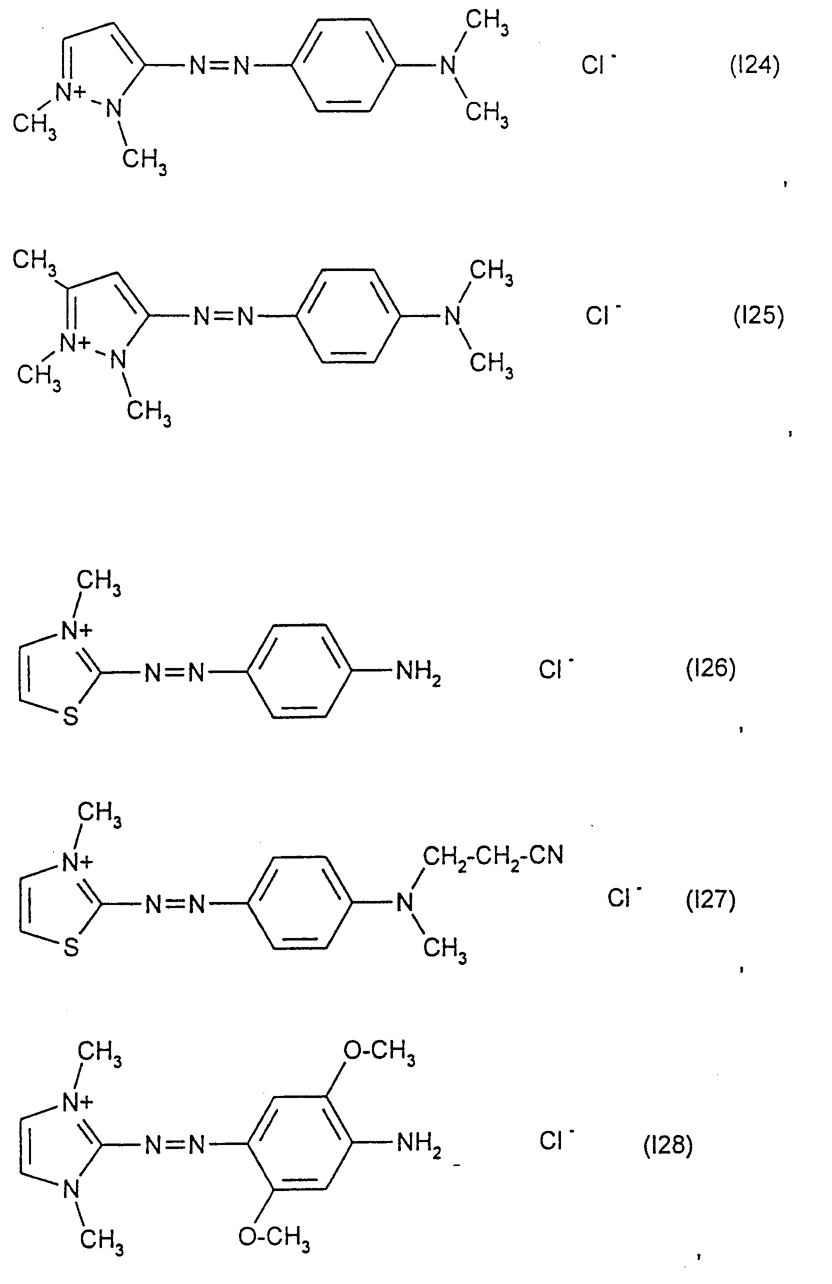

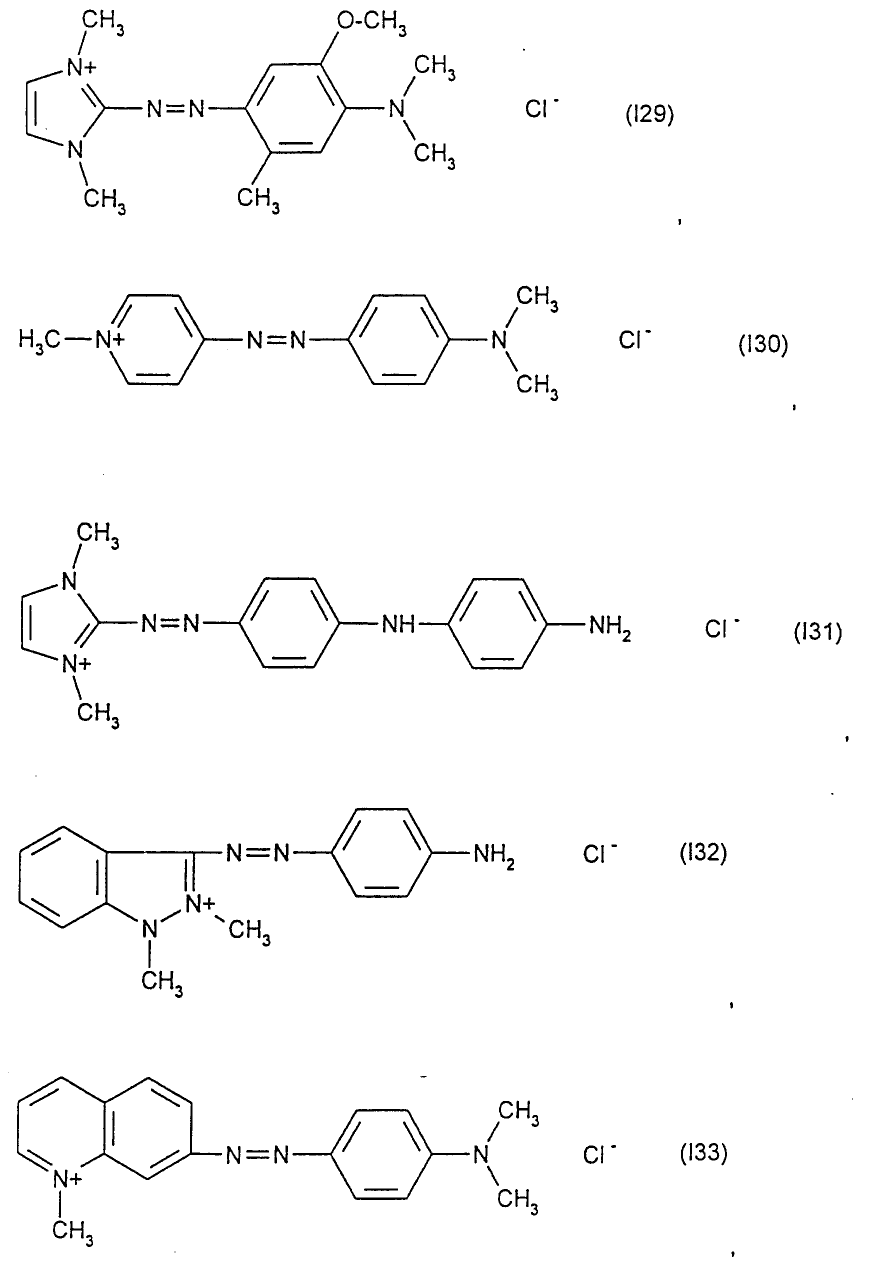

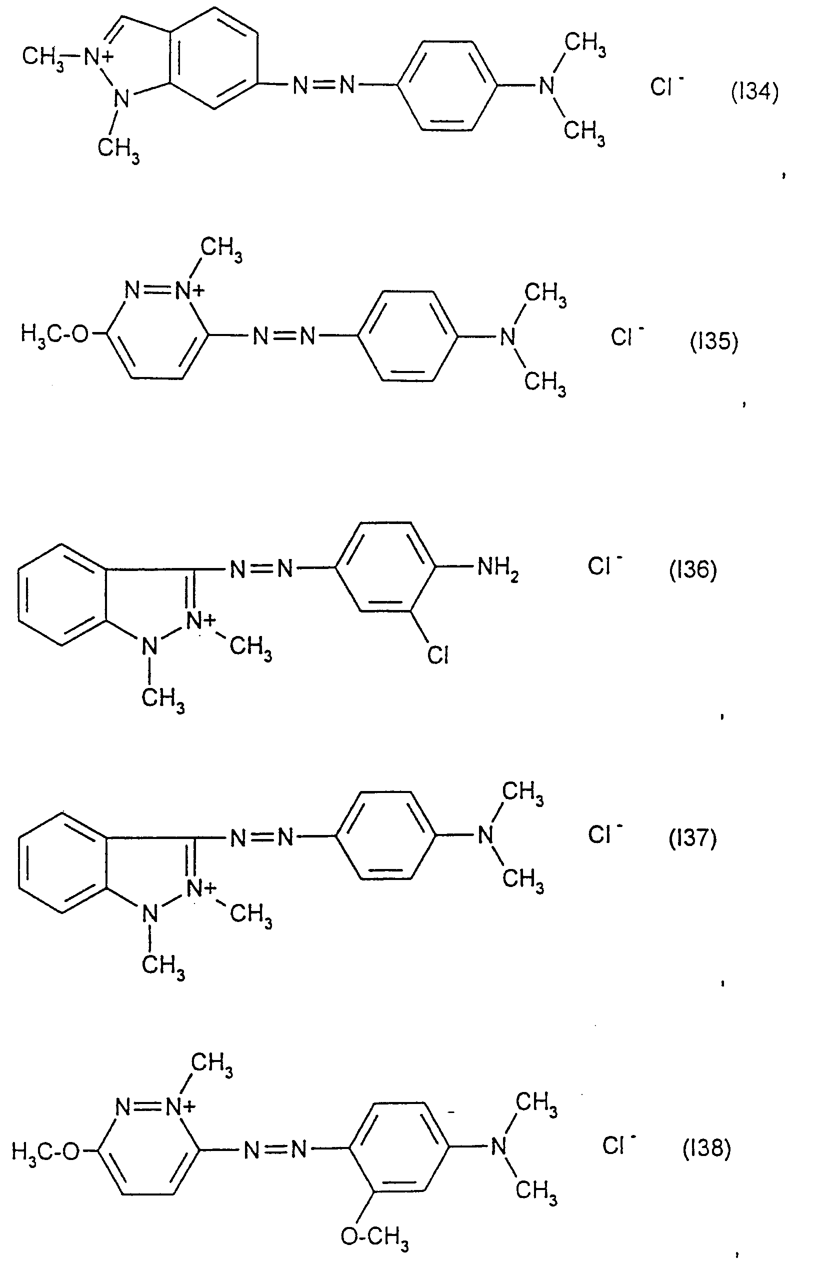

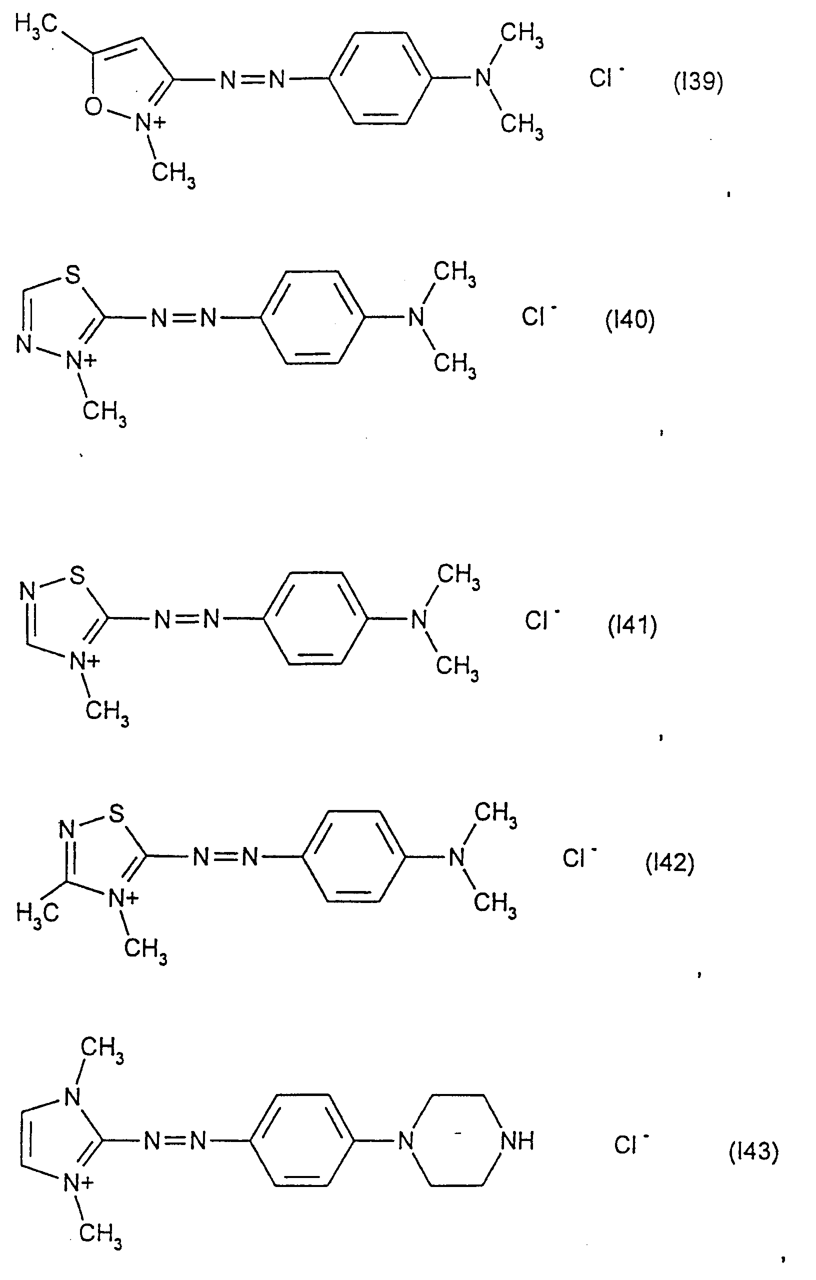

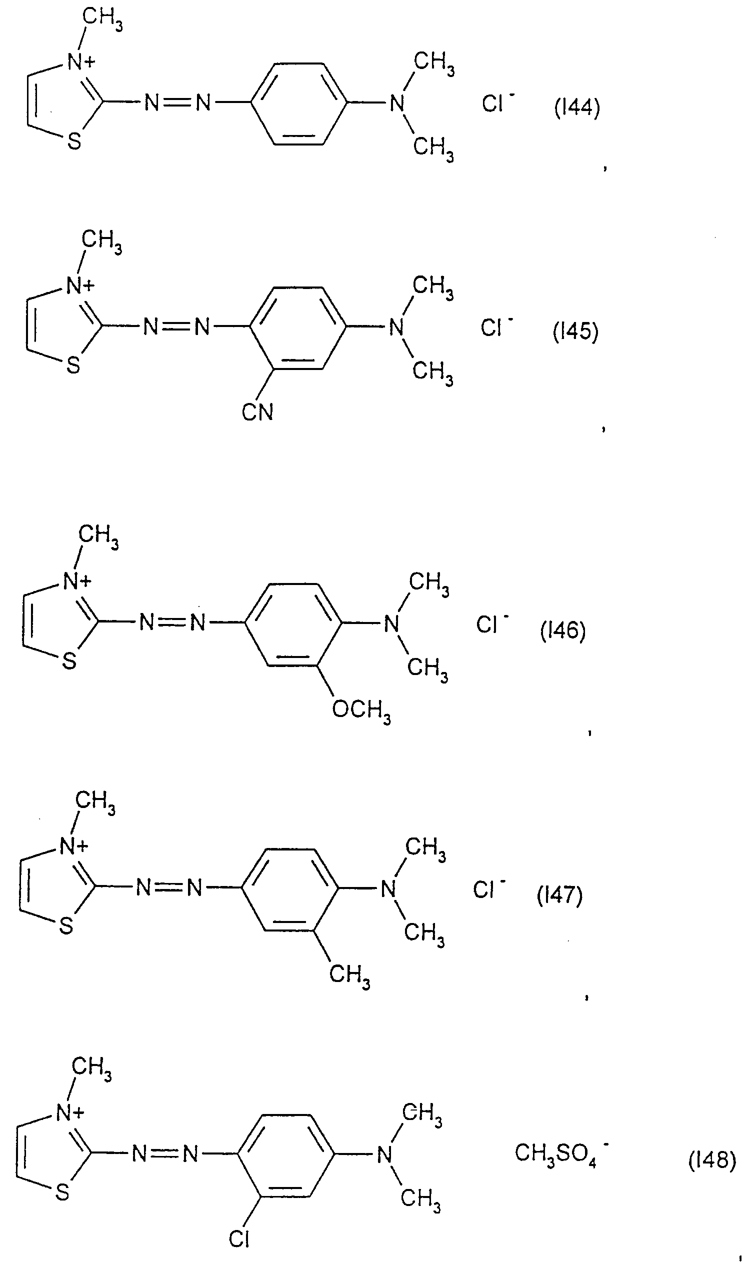

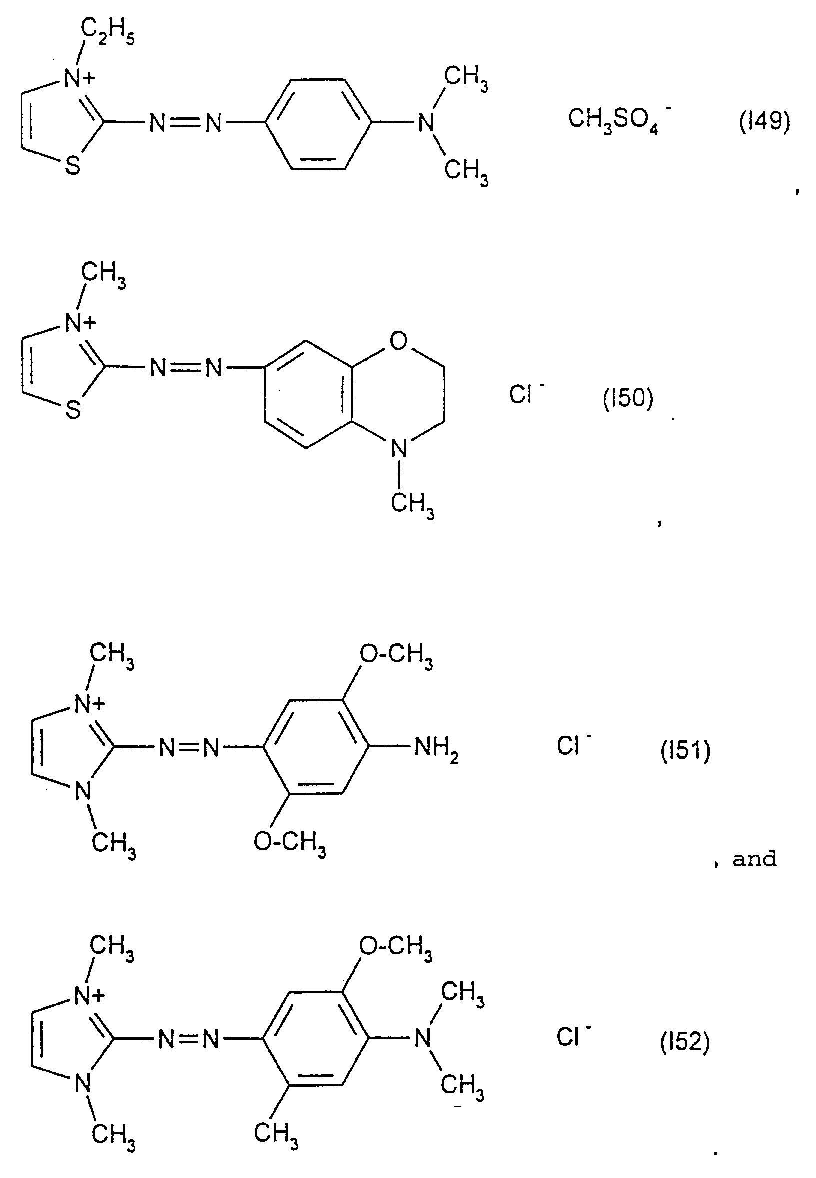

13. Composition according to any one of Claims 1 to 11, characterized in that the cationic direct dyes of formula (II) are chosen from the compounds corresponding to the structures (II1) to (II12) below:

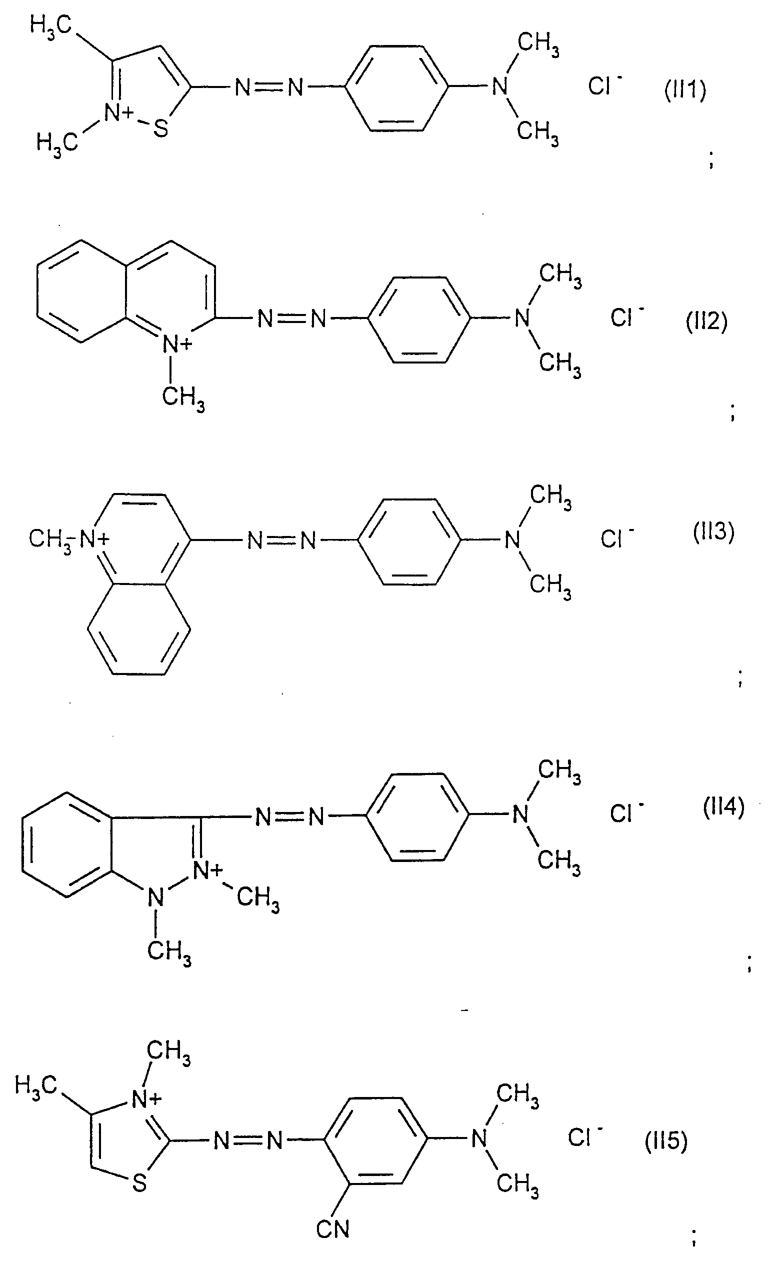

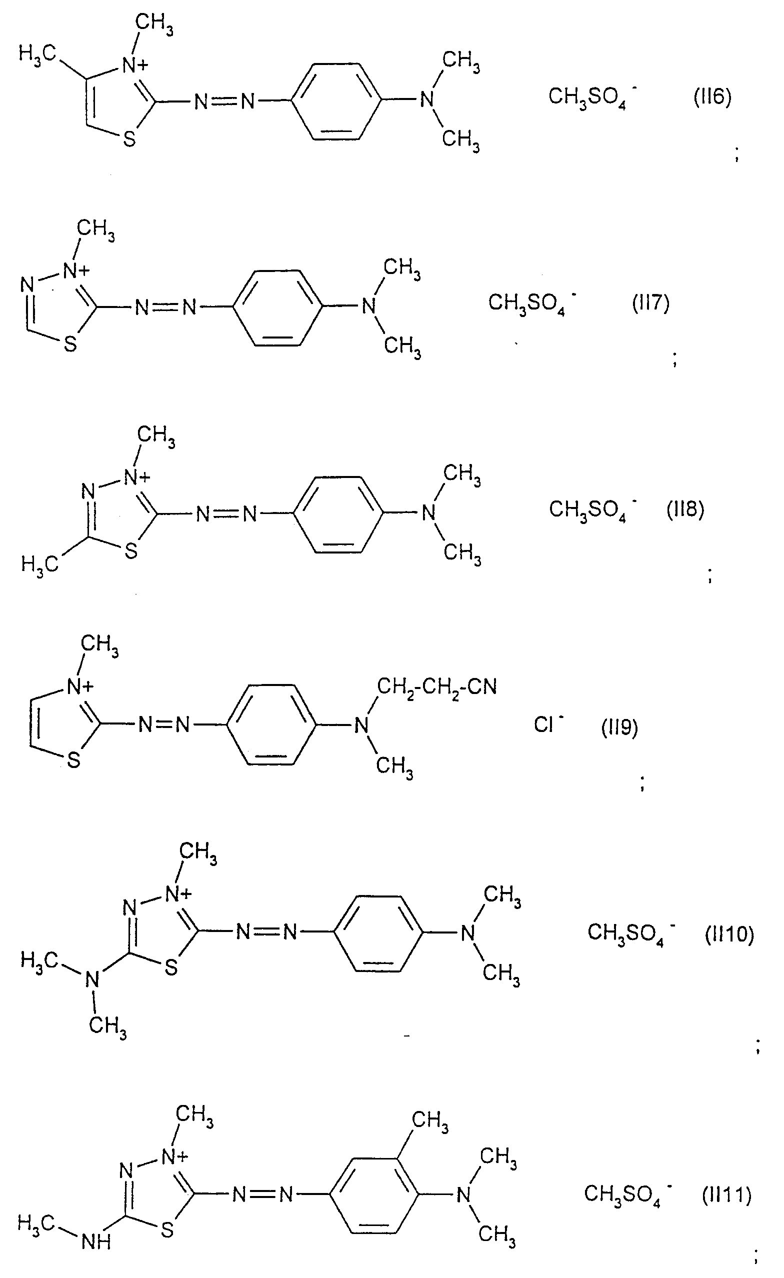

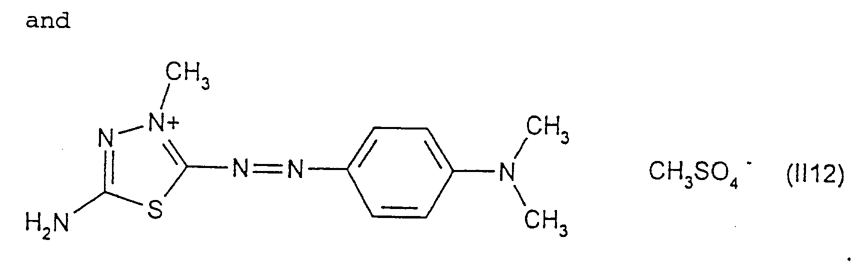

14. Composition according to any one of Claims 1 to 11, characterized in that the cationic direct dyes of formula (III) are chosen from the compounds corresponding to the structures (III1) to (III18) below:

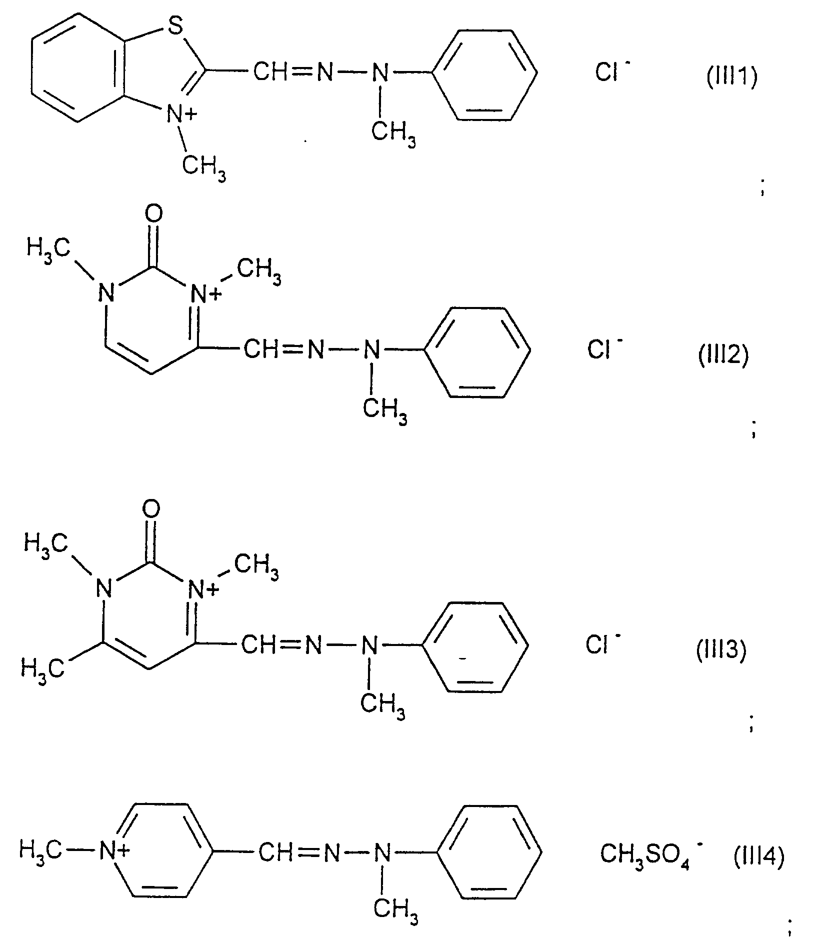

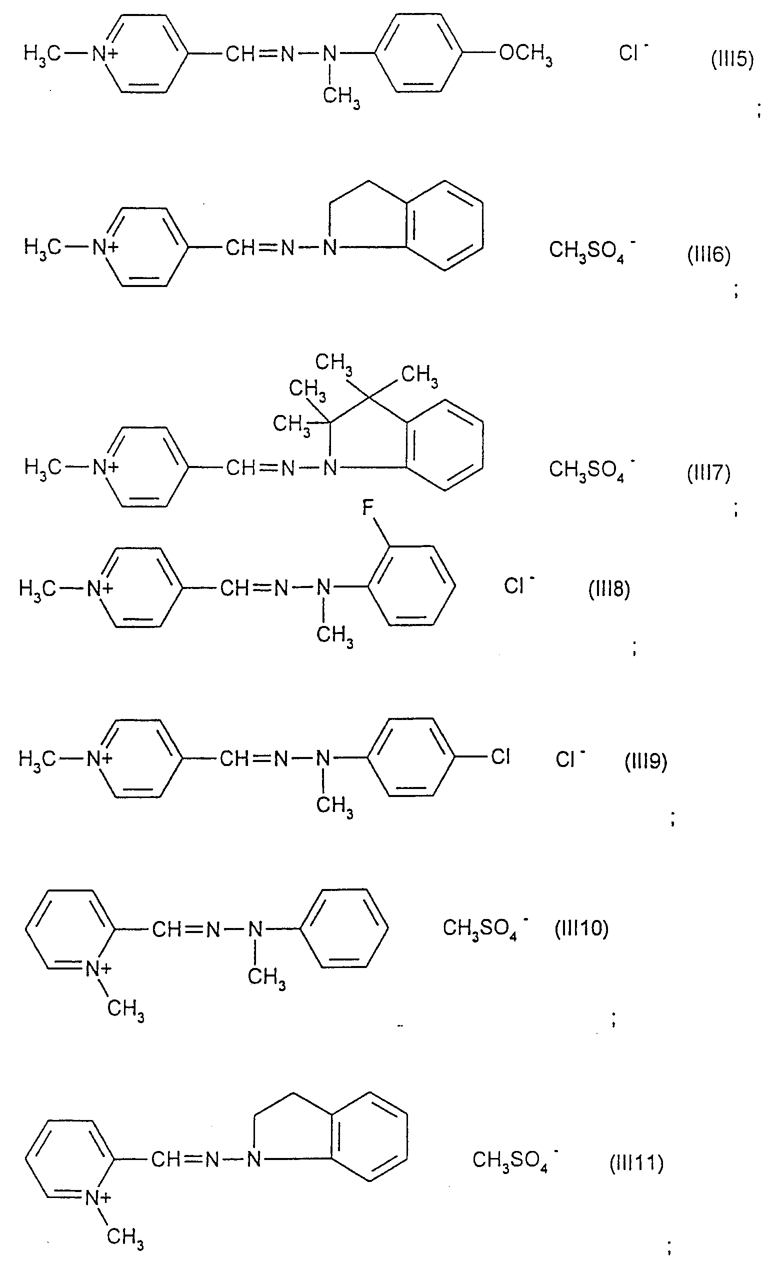

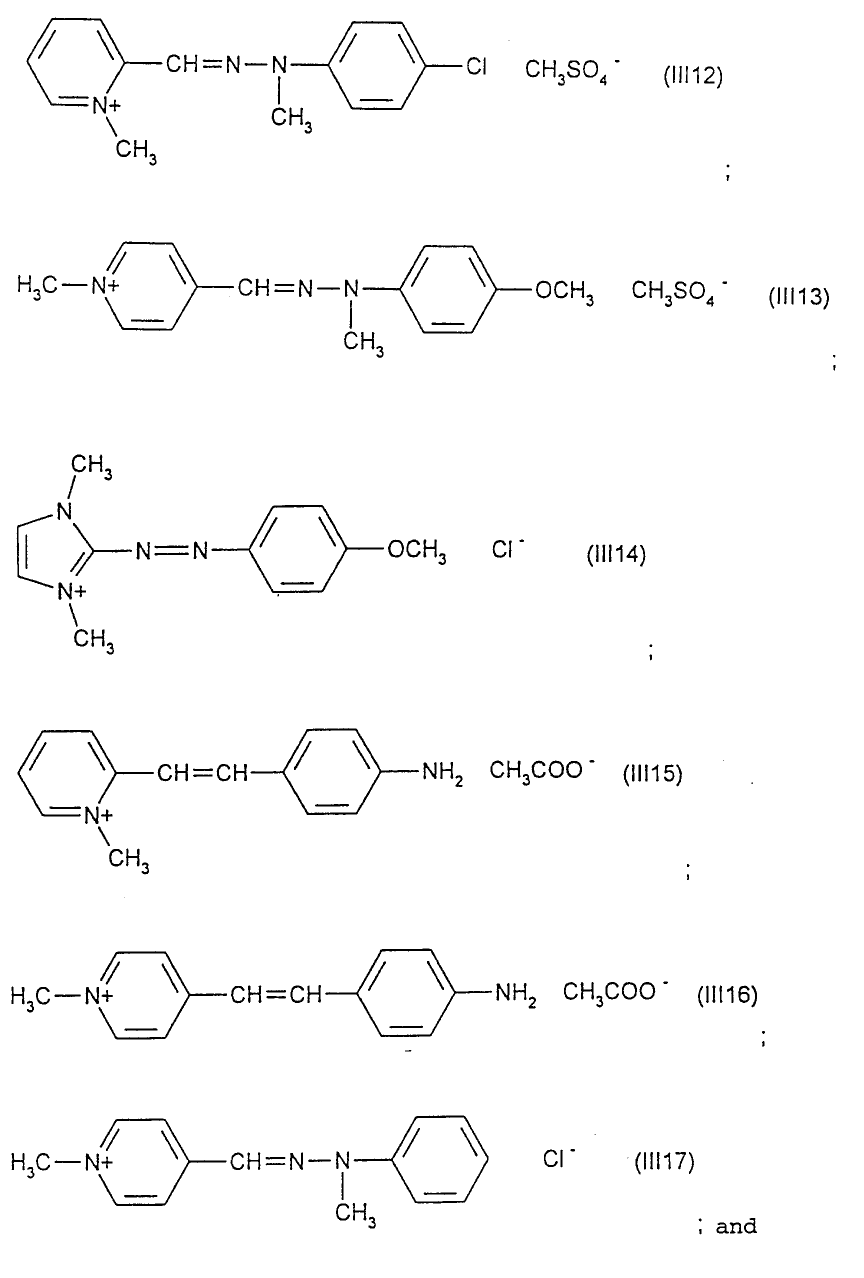

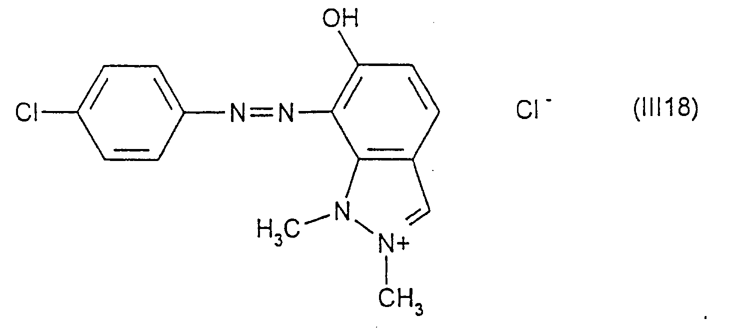

15. Composition according to any one of Claims 1 to 11, characterized in that the cationic direct dyes of formula (III') are chosen from the compounds corresponding to the structures (III'2) to (III'3) below:

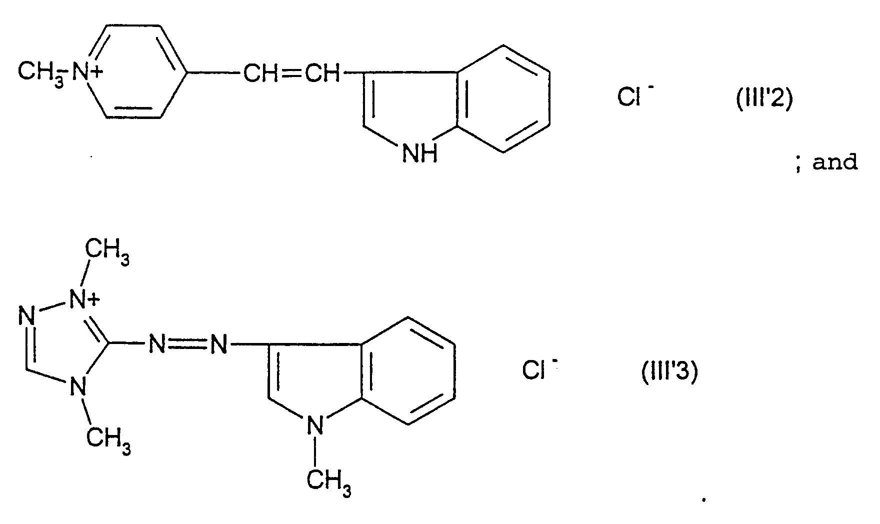

16. Composition according to any one of the preceding claims, characterized in that the oxidizing agent is chosen from hydrogen peroxide, urea peroxide, alkali metal bromates and persalts such as perborates and persulphates.

17. Composition according to any one of the preceding claims, characterized in that the oxidation base(s) represent from 0.0001 to 10% by weight relative to the total weight of the ready-to-use dye composition.

18. Composition according to any one of the preceding claims, characterized in that the cationic direct dye(s) of formulae (I) and/or (II) and/or (III) and/or (III') represent from 0.001 to 10% by weight relative to the total weight of the ready-to-use dye composition.

19. Composition according to any one of the preceding claims, characterized in that it has a pH of between 5 and 12.

20. Composition according to any one of the preceding claims, characterized in that the medium which is suitable for dyeing consists of water or of a mixture of water and at least one organic solvent.

21. Process for dyeing keratin fibres, and in particular human keratin fibres such as the hair, characterized in that at least one dye composition as defined in any one of Claims 1 to 20 is applied to these fibres.

22. Process according to Claim 21, characterized in that it includes a preliminary step which consists in separately storing, on the one hand, a composition (A) comprising, in a medium which is suitable for dyeing, at least one oxidation base and at least one cationic direct dye chosen from the compounds of formulae (I), (II), (III) and (III') as defined in Claim 1, and, on the other hand, a composition (B) containing, in a medium which is suitable for dyeing, at least one oxidizing agent, and in mixing them together at the time of use before applying this mixture to the keratin fibres.

23. Dyeing process according to Claim 21, characterized in that it includes a preliminary step which consists in separately storing, on the one hand, a composition (A) comprising, in a medium which is suitable for dyeing, at least one oxidation base; on the other hand, a composition (A') comprising, in a medium which is suitable for dyeing, at least one cationic direct dye chosen from the compounds of- formulae (I), (II), (III) and (III') as defined in Claim 1, and, lastly, a composition (B) containing, in a medium which is suitable for dyeing, at least one oxidizing agent, and in mixing them together at the time of use before applying this mixture to the keratin fibres.

24. Process according to Claim 23, characterized in that the composition (A') is in powder form.

25. Multi-compartment dyeing "kit" or device, characterized in that a first compartment contains the composition (A) as defined in Claim 22 and a second compartment contains an oxidizing composition (B).

26. Multi-compartment dyeing "kit" or device, characterized in that a first compartment contains a composition (A) as defined in Claim 23, a second compartment contains a composition (A') as defined in Claim 23 or 24, and a third compartment contains an oxidizing composition (B).

## Patentansprüche

1. Gebrauchsfertige Zusammensetzung für die oxidative Färbung von Keratinfasern und insbesondere von menschlichen Keratinfasern wie den Haaren, dadurch gekennzeichnet ist, daß sie in einem für die Färbung geeigneten Medium enthält:
- mindestens eine Oxidationsbase,
- mindestens einen kationischen Direktfarbstoff, der ausgewählt ist unter:
a) den Verbindungen der folgenden Formel (I), in der bedeuten: 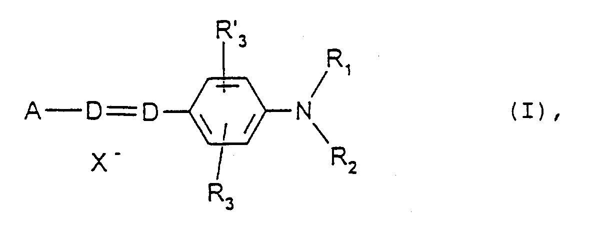
- D ein Stickstoffatom oder die Gruppe -CH,
- R₁ und R₂, die gleich oder verschieden sind, Wasserstoff, C₁-C₄-Alkyl, das mit einer Gruppe -CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁-C₄-Alkylgruppen substituiert sein kann, oder 4'-Aminophenyl,
- R₃ und R'₃, die gleich oder verschieden sind, Wasserstoff oder ein Halogenatom, das unter Chlor, Brom, Iod und Fluor ausgewählt ist, Cyano, C₁-C₄-Alkoxy oder Acetyloxy,
- X⁻ ein Anion, das vorzugsweise unter Chlorid, Methylsulfat und Acetat ausgewählt ist,
- A eine Gruppe, die unter den folgenden Strukturen A₁ bis A₁₉ ausgewählt ist: 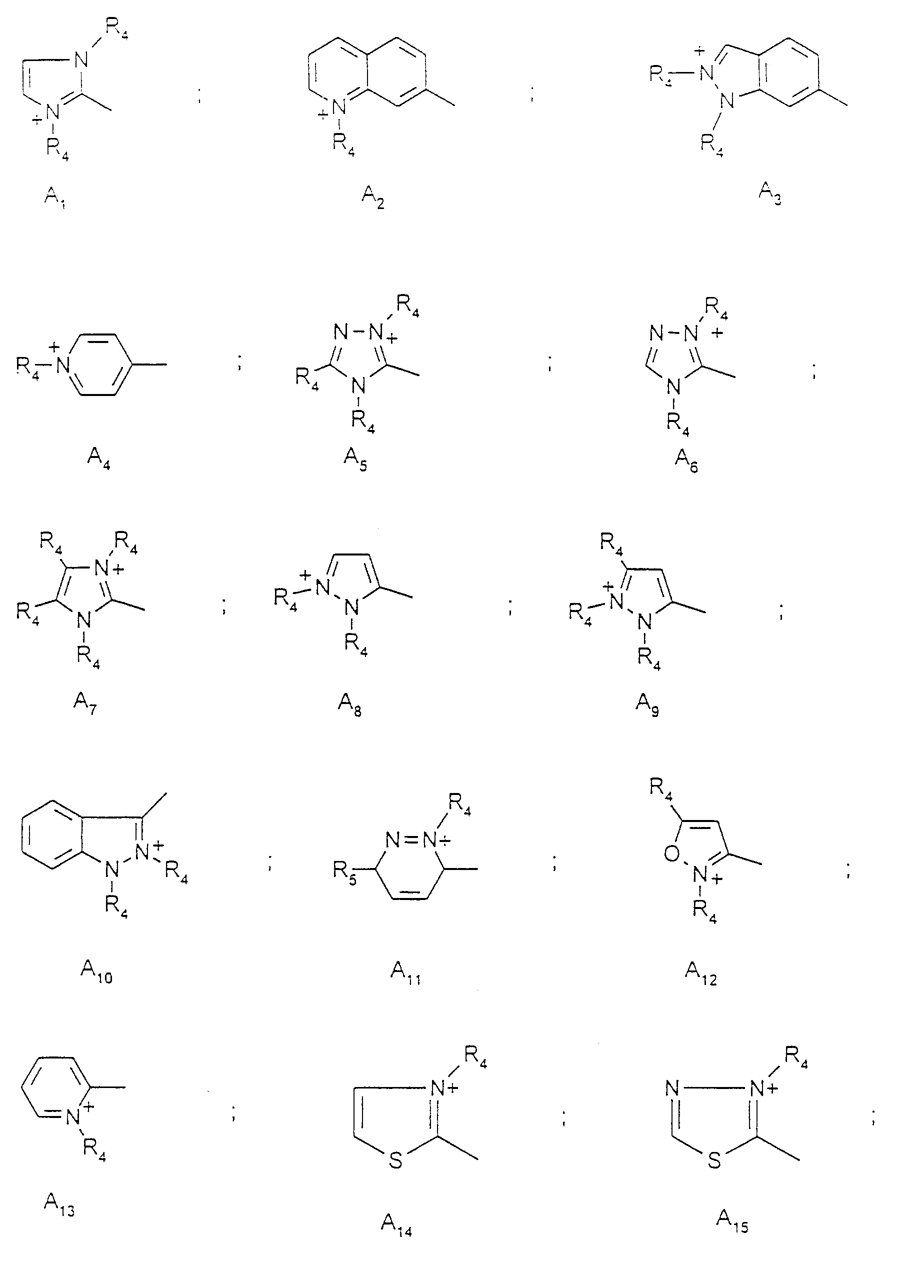 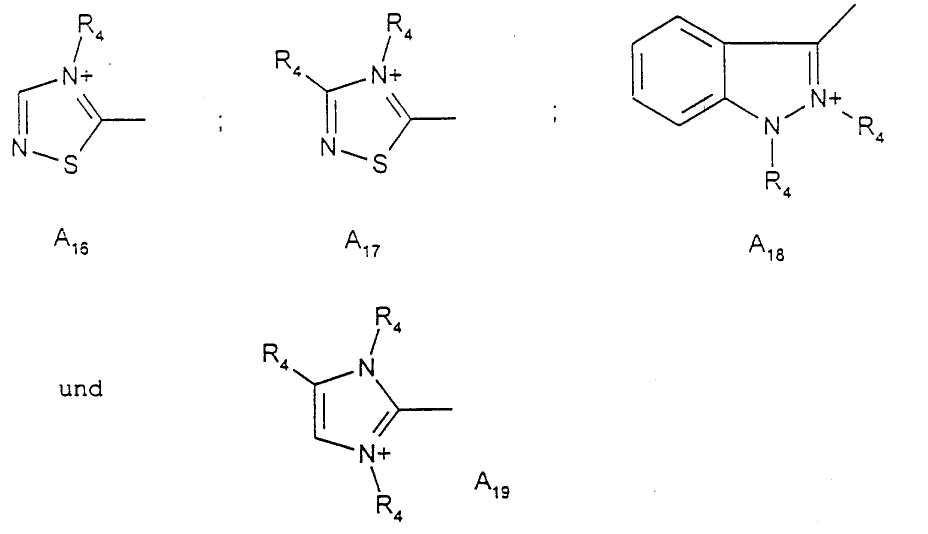
worin R₄ C₁-C₄-Alkyl, das mit einer Hydroxygruppe substituiert sein kann, und R₅ C₁-C₄-Alkoxy bedeutet mit der Maßgabe, daß R₁ und R₂ nicht gleichzeitig Wasserstoff bedeuten, wenn D -CH, A die Gruppe A₄ oder A₁₃ bedeutet und wenn R₃ verschieden von Alkoxy ist;
b) den Verbindungen der folgenden Formel (II) 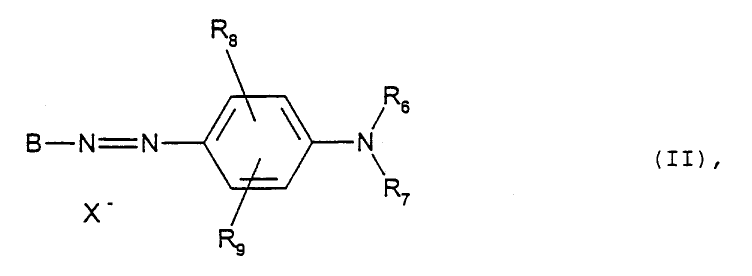 in der bedeuten:
- R₆ Wasserstoff oder C₁-C₄-Alkyl,
- R₇ Wasserstoff, Alkyl, das mit einer Gruppe -CN oder einer Aminogruppe substituiert sein kann, 4-Aminophenyl oder einen Rest, der mit R₆ einen gegebenenfalls sauerstoff- und/oder stickstoffhaltigen Heterocyclus bildet, der mit C₁-C₄-Alkyl substituiert sein kann,
- R₈ und R₉, die gleich oder verschieden sind, Wasserstoff, Halogen, wie z.B. Brom, Chlor, Iod oder Fluor, C₁-C₄-Alkyl oder C₁-C₄-Alkoxy, -CN,
- X⁻ ein Anion, das vorzugsweise unter Chlorid, Methylsulfat und Acetat ausgewählt ist,
- B eine Gruppe, die unter den folgenden Strukturen B1 bis B6 ausgewählt ist: 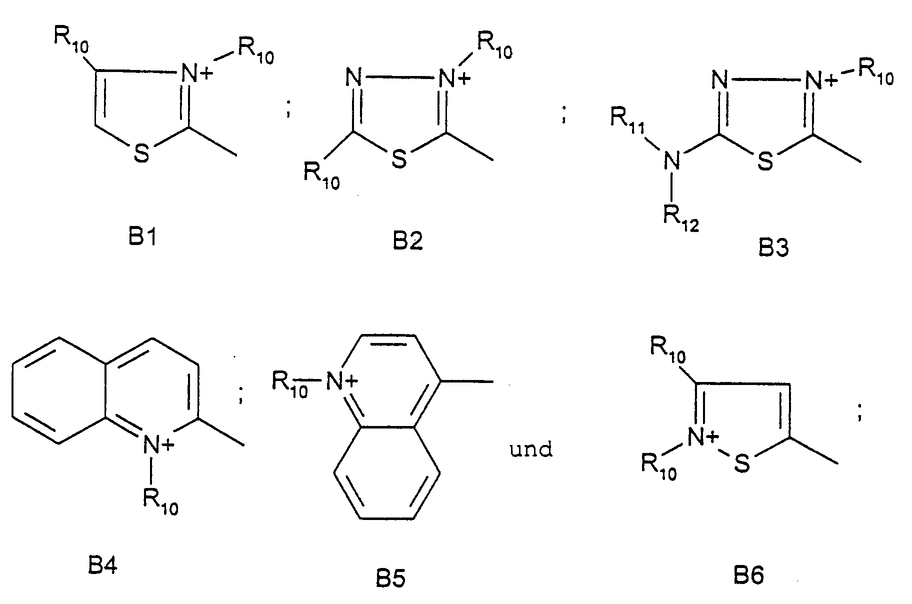
worin R₁₀ C₁-C₄-Alkyl und R₁₁ und R₁₂, die gleich oder verschieden sind, Wasserstoff oder C₁-C₄-Alkyl bedeuten;
c) der Verbindung der folgenden Formel (III'1) 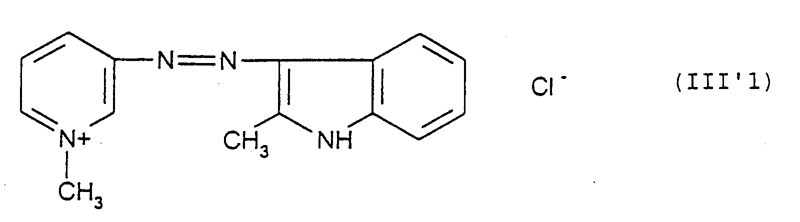 und den Verbindungen der folgenden Formeln (III) und (III') 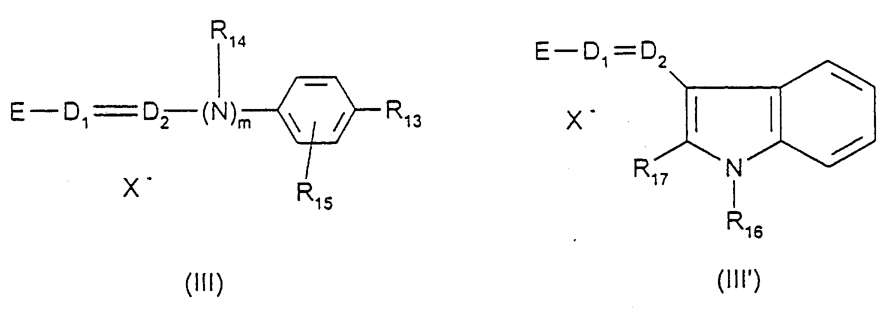 in denen bedeuten:
R₁₃ Wasserstoff, C₁-C₄-Alkoxy, ein Halogenatom, wie z.B. Brom, Chlor, Iod oder Fluor, oder Amino,
R₁₄ Wasserstoff, C₁-C₄-Alkyl, oder eine Gruppe, die mit einem Kohlenstoffatom des Benzolrings einen gegebenenfalls sauerstoffhaltigen und/oder mit einer oder mehreren C₁-C₄-Alkylgruppen substituierten Heterocyclus bildet,
R₁₅ Wasserstoff oder ein Halogenatom, wie z.B. Brom, Chlor, Iod oder Fluor,
R₁₆ und R₁₇, die gleich oder verschieden sind, Wasserstoff oder C₁-C₄-Alkyl,
D₁ und D₂, die gleich oder verschieden sind, ein Stickstoff- atom oder die Gruppe -CH,
m = 0 oder 1,
wobei D₁ und D₂ gleichzeitig eine Gruppe -CH bedeuten und m gleich 0 ist, wenn R₁₃ eine nicht substituierte Aminogruppe darstellt,
X⁻ ein Anion, das vorzugsweise unter Chlorid, Methylsulfat und Acetat ausgewählt ist,
E eine Gruppe, die unter den folgenden Strukturen E1 bis E7 ausgewählt ist 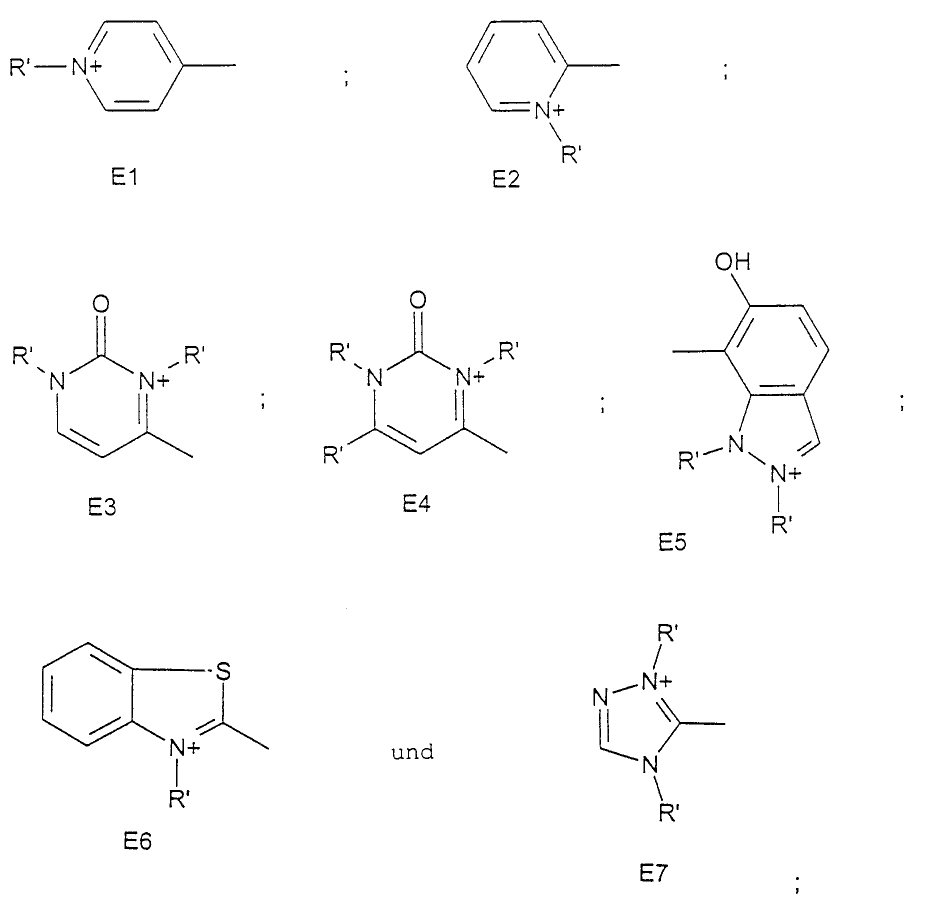
in denen R' C₁-C₄-Alkyl bedeutet,
wobei E außerdem eine Gruppe der folgenden Struktur E8 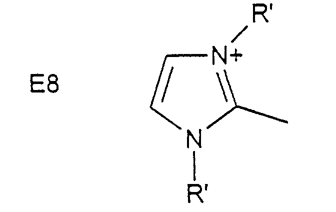
in der R' C₁-C₄-Alkyl bedeutet, sein kann, wenn m gleich 0 ist und D₁ ein Stickstoffatom darstellt;
und
- mindestens ein Oxidationsmittel.

2. Zusammensetzung nach Anspruch 1, dadurch gekennzeichnet, daß die Oxidationsbase(n) unter p-Phenylendiaminen, Bis-phenylalkylendiaminen, p-Aminophenolen, o-Aminophenolen und heterocyclischen Oxidationsbasen ausgewählt ist/sind.

3. Zusammensetzung nach Anspruch 2, dadurch gekennzeichnet, daß die p-Phenylendiamine unter den Verbindungen der folgenden Formel (IV) 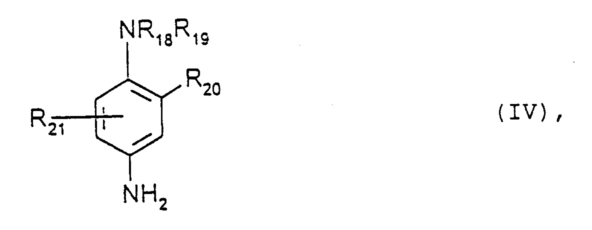 und ihren Additionssalzen mit einer Säure ausgewählt sind, worin bedeuten:
R₁₈ Wasserstoff, C₁-C₄-Alkyl, C₁-C₄-Monohydroxyalkyl, C₂-C₄-Polyhydroxyalkyl, Phenyl, 4'-Aminophenyl oder (C₁-C₄)-Alkoxy-(C₁-₄)-alkyl,
R₁₉ Wasserstoff, C₁-C₄-Alkyl, C₁-C₄-Monohydroxyalkyl oder C₂-C₄-Polyhydroxyalkyl,
R₂₀ Wasserstoff, ein Halogenatom, wie z.B. Chlor, Brom, Iod oder Fluor, C₁-C₄-Alkyl, C₁-C₄-Monohydroxyalkyl, C₁-C₄-Hydroxyalkoxy, Acetylamino-C₁-C₄-alkoxy, Mesylamino-C₁-C₄-alkoxy oder Carbamoylamino-C₁-C₄-alkoxy,
R₂₁ Wasserstoff oder C₁-C₄-Alkyl.

4. Zusammensetzung nach Anspruch 3, dadurch gekennzeichnet, daß die p-Phenylendiamine der Formel (IV) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-Amino-N,N-bis(β-hydroxyethyl)-3-methylanilin, 4-Amino-3-chlor-N,N-bis(β-hydroxyethyl)-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin und ihren Additionssalzen mit einer Säure ausgewählt sind.

5. Zusammensetzung nach Anspruch 2, dadurch gekennzeichnet, daß die Bisphenylalkylendiaminen unter den Verbindungen der folgenden Formel (V) 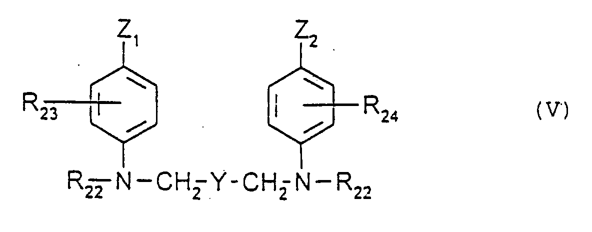 und ihren Additionssalzen mit einer Säure ausgewählt sind, in der bedeuten:
Z₁ und Z₂, die gleich oder verschieden sind, Hydroxy oder -NHR₂₅, worin R₂₅ Wasserstoff oder C₁-C₄-Alkyl bedeutet,
R₂₂ Wasserstoff, C₁-C₄-Alkyl, C₁-C₄-Monohydroxyalkyl, C₂-C₄-Polyhydroxyalkyl oder C₁-C₄-Aminoalkyl, wobei die Aminogruppe substituiert sein kann,
R₂₃ und R₂₄, die gleich oder verschieden sind, Wasserstoff oder ein Halogenatom oder C₁-C₄-Alkyl,
Y eine Gruppe, die unter den folgenden Gruppen
-(CH₂)ₙ-; -(CH₂)ₘ-; -(CH₂)ₘ-CHOH-(CH₂)ₘ-und 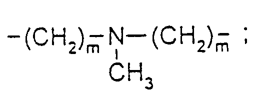
worin n eine ganze Zahl von 0 bis 8 einschließlich 0 und 8 und m eine ganze Zahl von 0 bis 4 einschließlich 0 und 4 bedeutet, ausgewählt ist.

6. Zusammensetzung nach Anspruch 5, dadurch gekennzeichnet, daß die Bis-phenylalkylendiamine der (V) unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)tetramethylendiamin, N,N'-Bis(β-hydroxyethyl)-N,N'-bis(4'-aminophenyl)tetramethylendiamin, N,N'-Bis(4-methylaminophenyl)tetramethylendiamin, N,N'-Bis(ethyl)-N,N'-bis(4'-amino-3'-methylphenyl)ethylendiamin und ihren Additionssalzen mit einer Säure ausgewählt sind.

7. Zusammensetzung nach Anspruch 2, dadurch gekennzeichnet, daß die p-Aminophenole unter den Verbindungen der folgenden Formel (VI) 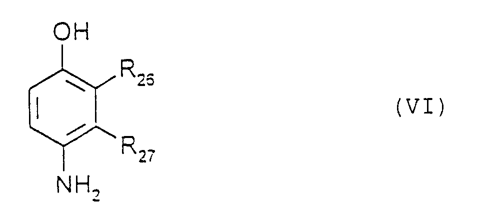 und ihren Additionssalzen mit einer Säure ausgewählt sind, worin bedeuten:
R₂₆ Wasserstoff oder Fluor, C₁-C₄-Alkyl, C₁-C₄-Monohydroxyalkyl, (C₁-C₄)-Alkoxy-(C₁-C₄)-alkyl, C₁-C₄-Aminoalkyl oder (C₁-C₄)-Hydroxyalkyl-(C₁-C₄)-aminoalkyl,
R₂₇ Wasserstoff oder Fluor, C₁-C₄-Alkyl, C₁-C₄-Monohydroxyalkyl, C₂-C₄-Polyhydroxyalkyl, C₁-C₄-Aminoalkyl, C₁-C₄-Cyanoalkyl, (C₁-C₄)-Alkoxy-(C₁-C₄)-alkyl,
wobei mindestens einer der Reste R₂₆ und R₂₇ Wasserstoff bedeutet.

8. Zusammensetzung nach Anspruch 7, dadurch gekennzeichnet, daß die p-Aminophenole der (VI) unter p-Aminophenol, 4-Amino-3-methylphenol, 4-Amino-3-fluorphenol, 4-Amino-3-(hydroxymethyl)-phenol, 4-Amino-2-methylphenol, 4-Amino-(2-hydroxymethyl)-phenol, 4-Amino-(2-methoxymethyl)-phenol, 4-Amino-(2-aminomethyl)-phenol, 4-Amino-2-(β-hydroxyethyl-aminomethyl)-phenol, 4-Amino-2-fluorphenol und ihren Additionssalzen mit einer Säure ausgewählt sind.

9. Zusammensetzung nach Anspruch 2, dadurch gekennzeichnet, daß die o-Aminophenole unter 2-Aminophenol, 2-Amino-5-methylphenol, 2-Amino-6-methylphenol, 5-Acetamido-2-aminophenol und ihren Additionssalzen mit einer Säure ausgewählt sind.

10. Zusammensetzung nach Anspruch 2, dadurch gekennzeichnet, daß die heterocyclischen Basen unter Pyridin-Derivaten, Pyrimidin-Derivaten, Pyrazol-Derivaten und ihren Additionssalzen mit einer Säure ausgewählt sind.

11. Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

12. Zusammensetzung nach einem der vorhergehenden Ansprüche, dadurch gekennzeichnet, daß die kationischen Direktfarbstoffe der Formel (I) unter den Verbindungen mit den folgenden Strukturen (I1) bis (I52) ausgewählt sind:

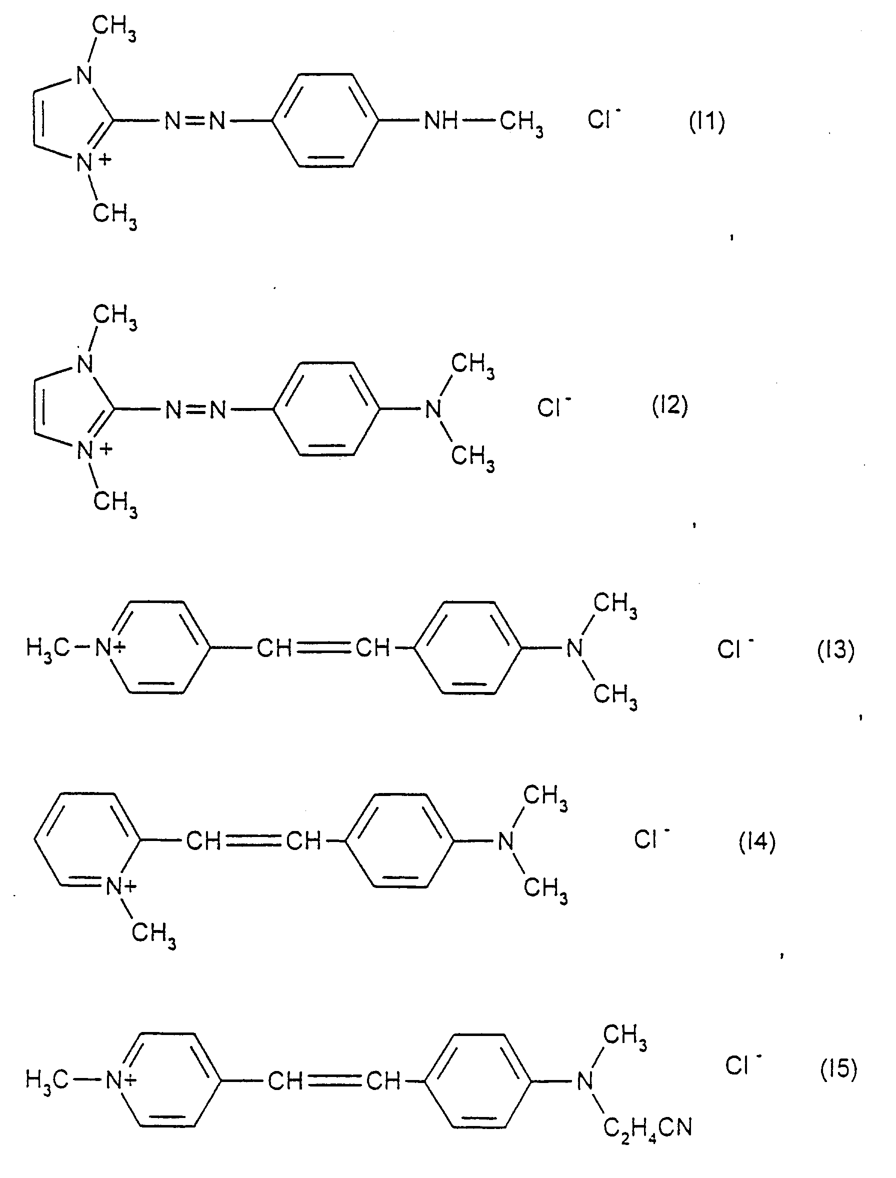

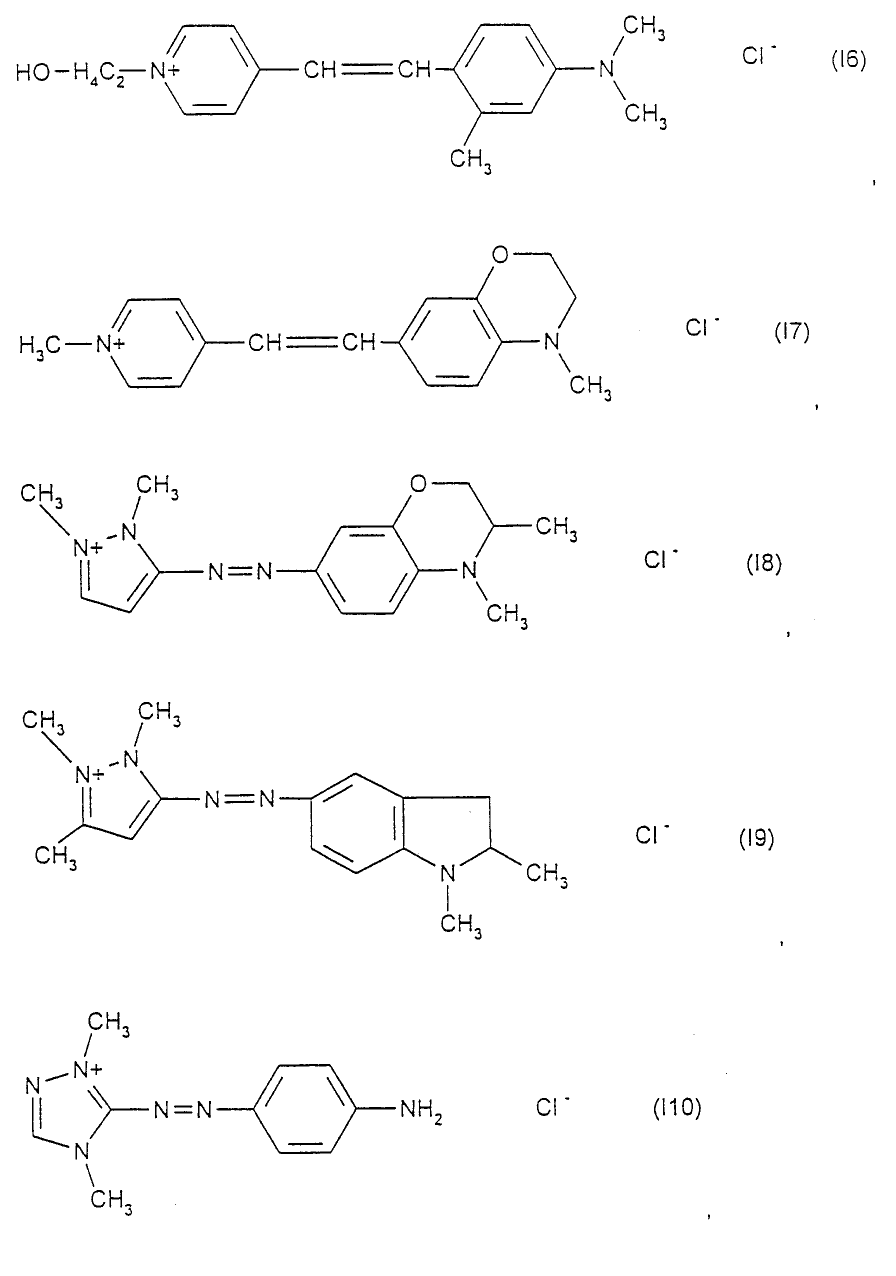

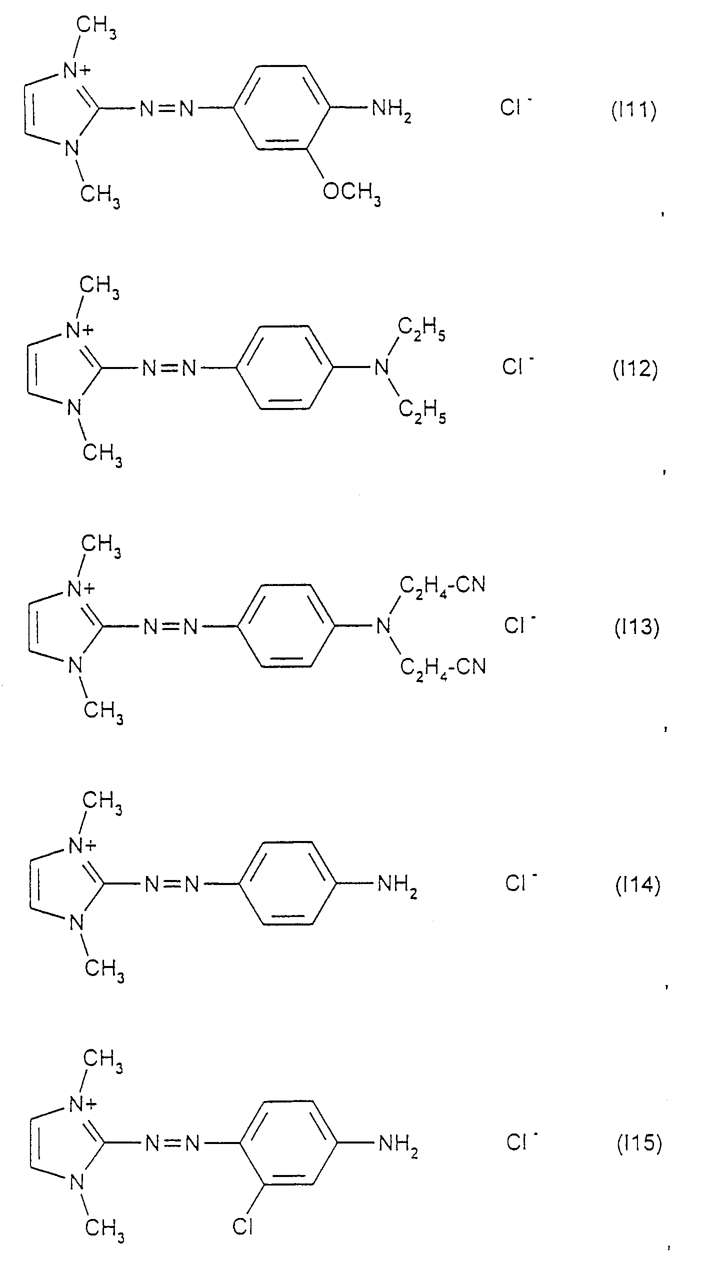

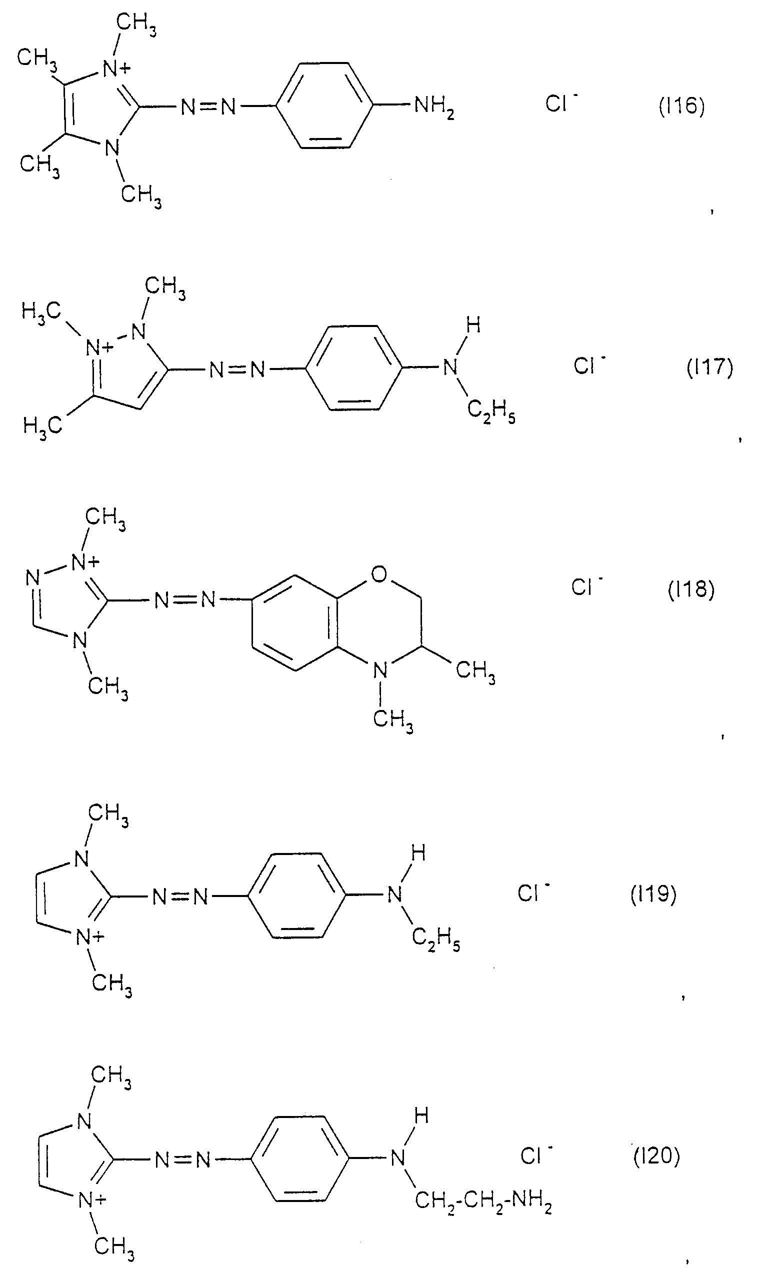

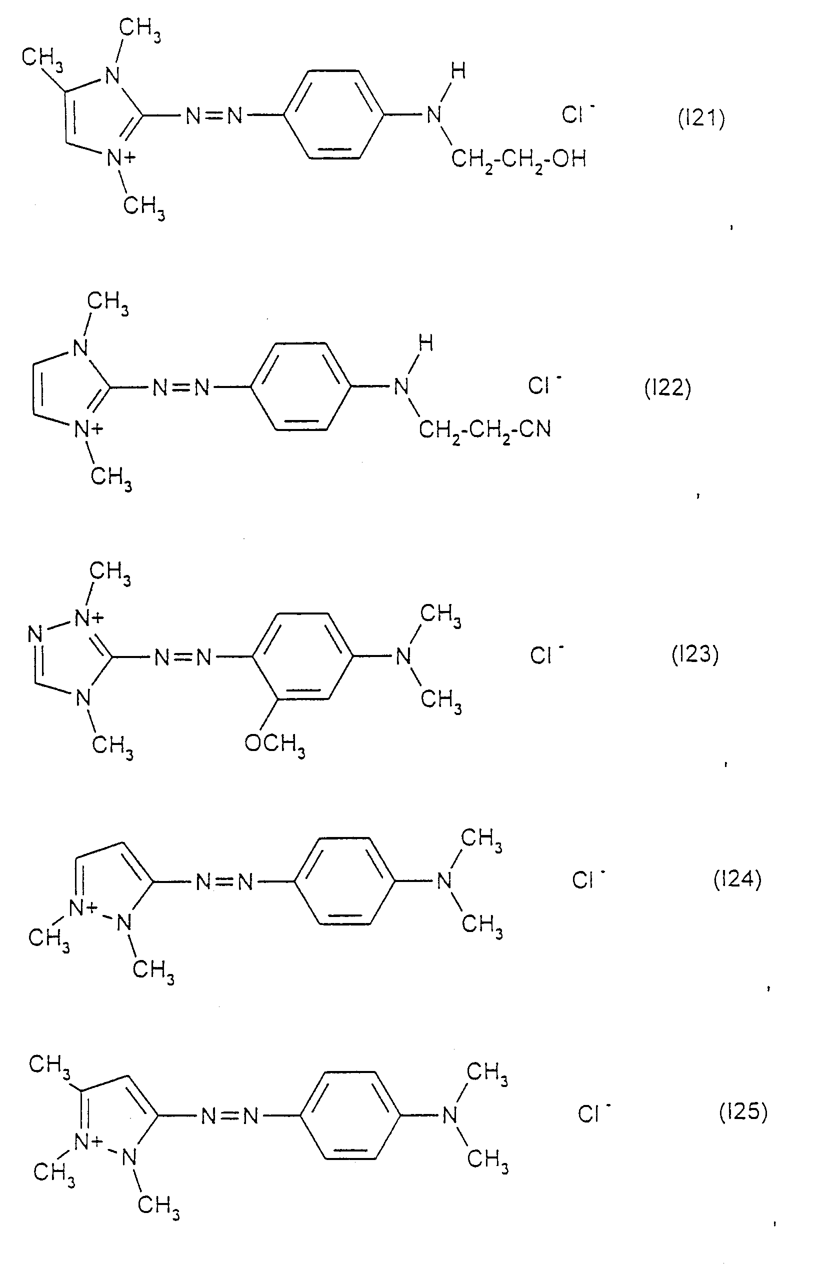

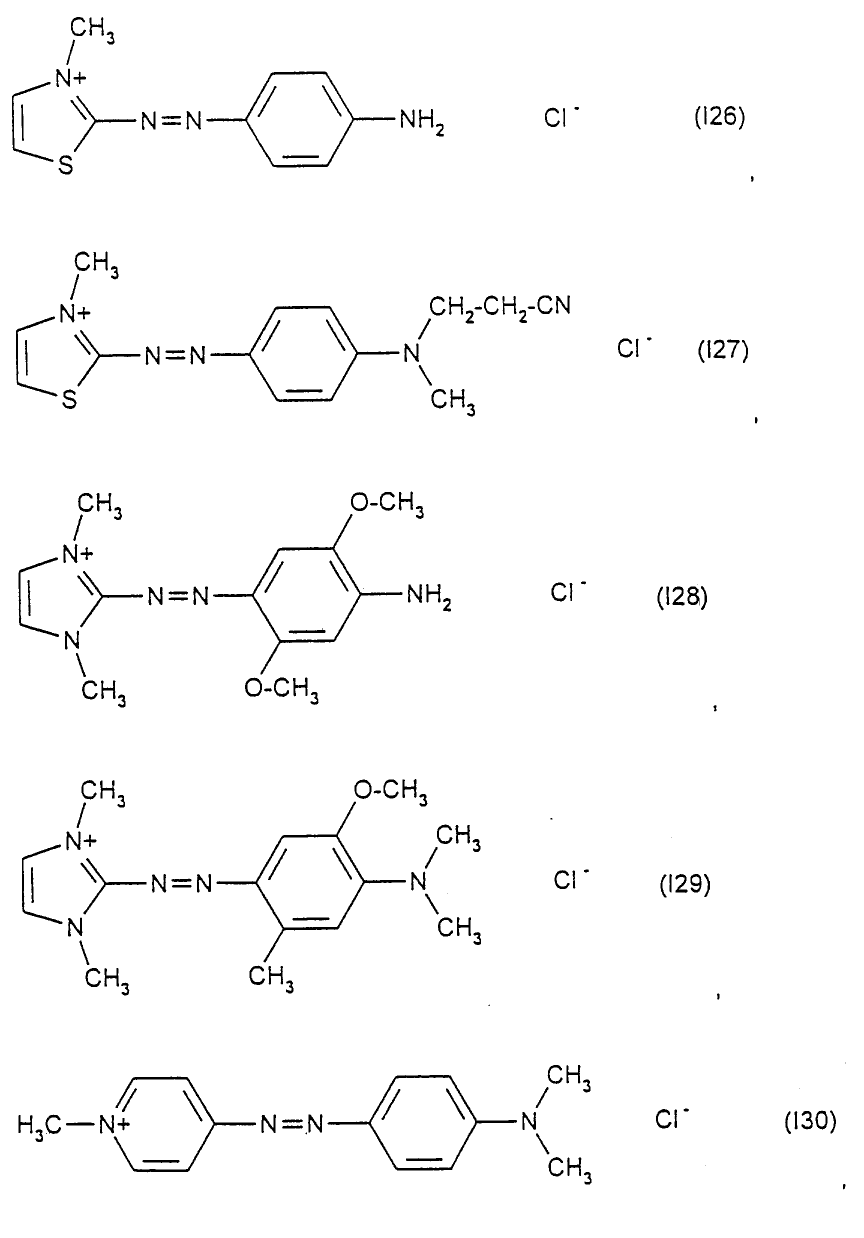

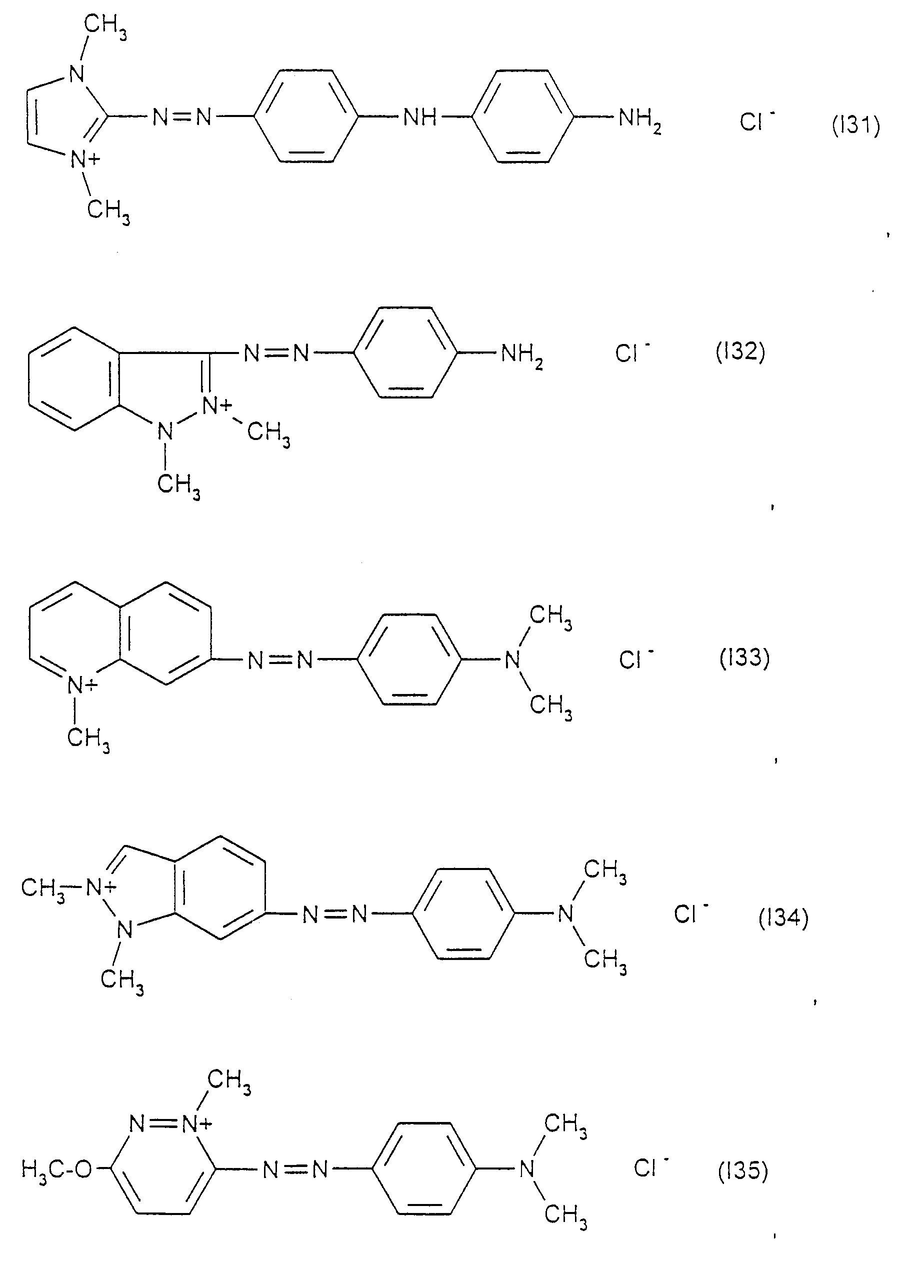

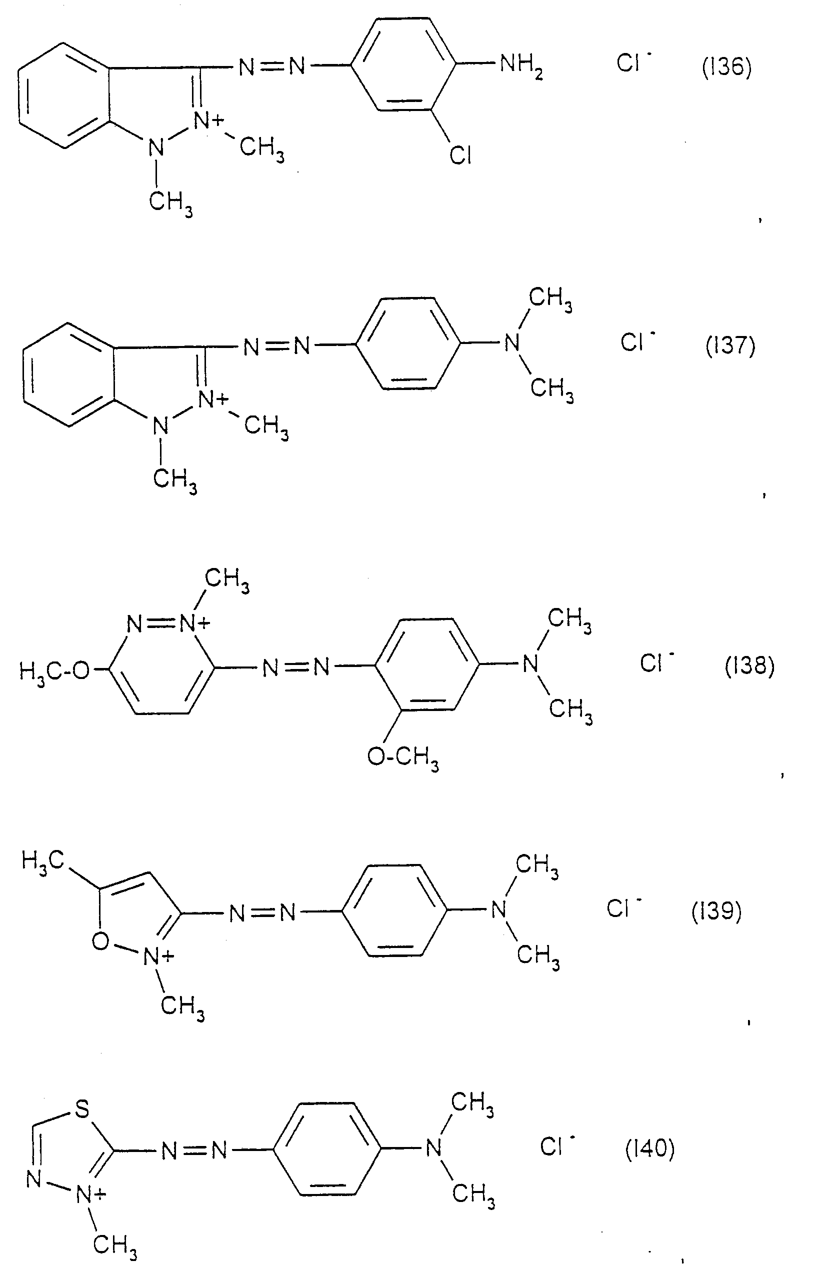

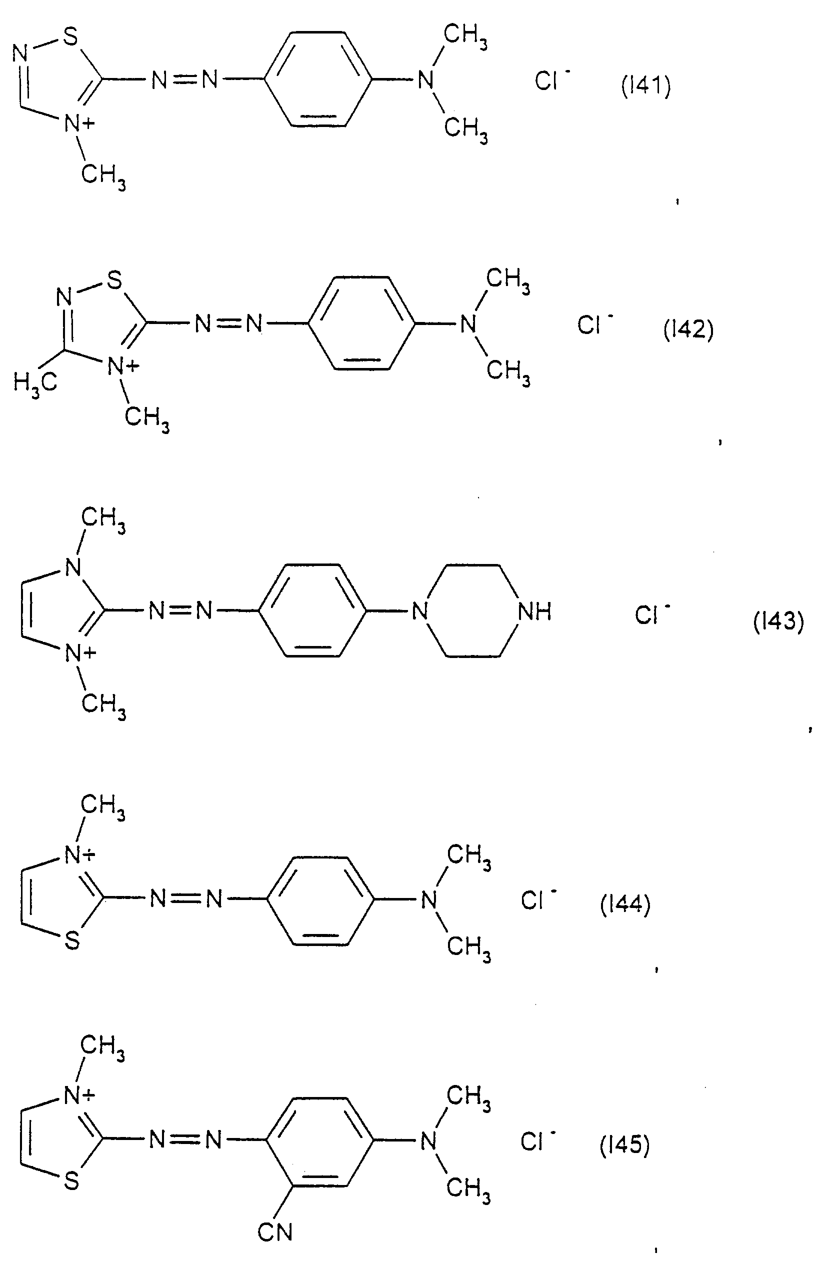

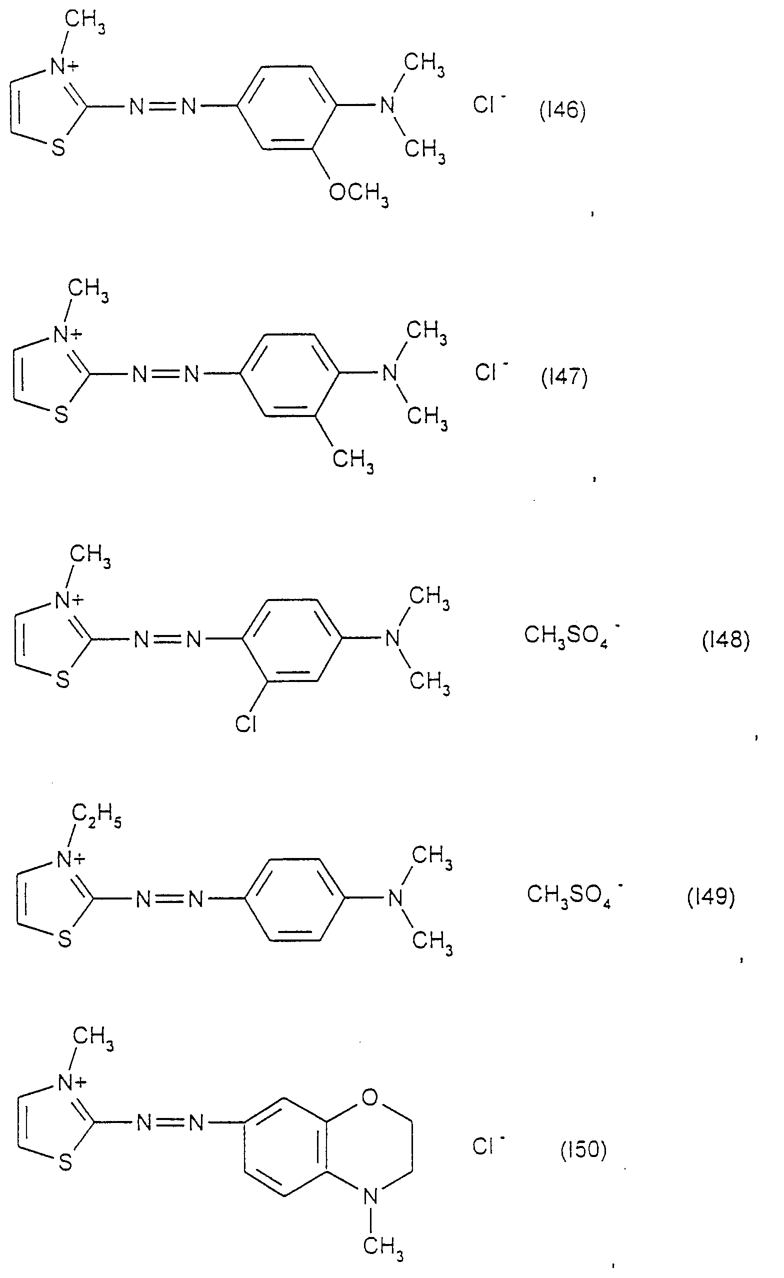

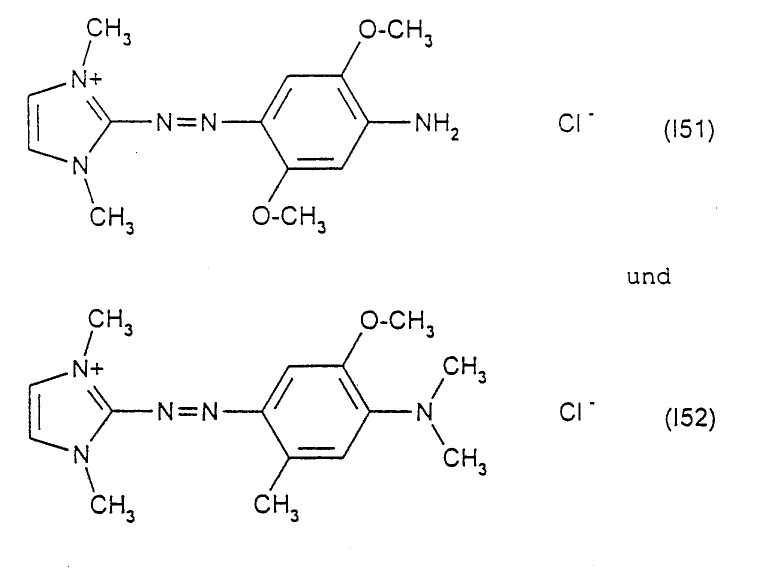

13. Zusammensetzung nach einem der Ansprüche 1 bis 11, dadurch gekennzeichnet, daß die kationischen Direktfarbstoffe der Formel (II) unter den Verbindungen mit den folgenden Strukturen (II1) bis (I12) ausgewählt sind:

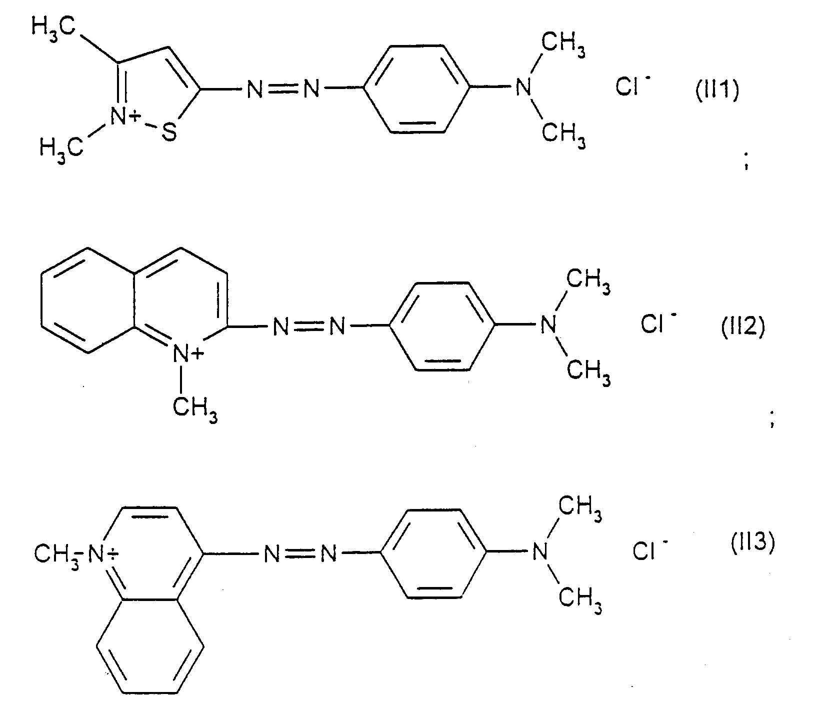

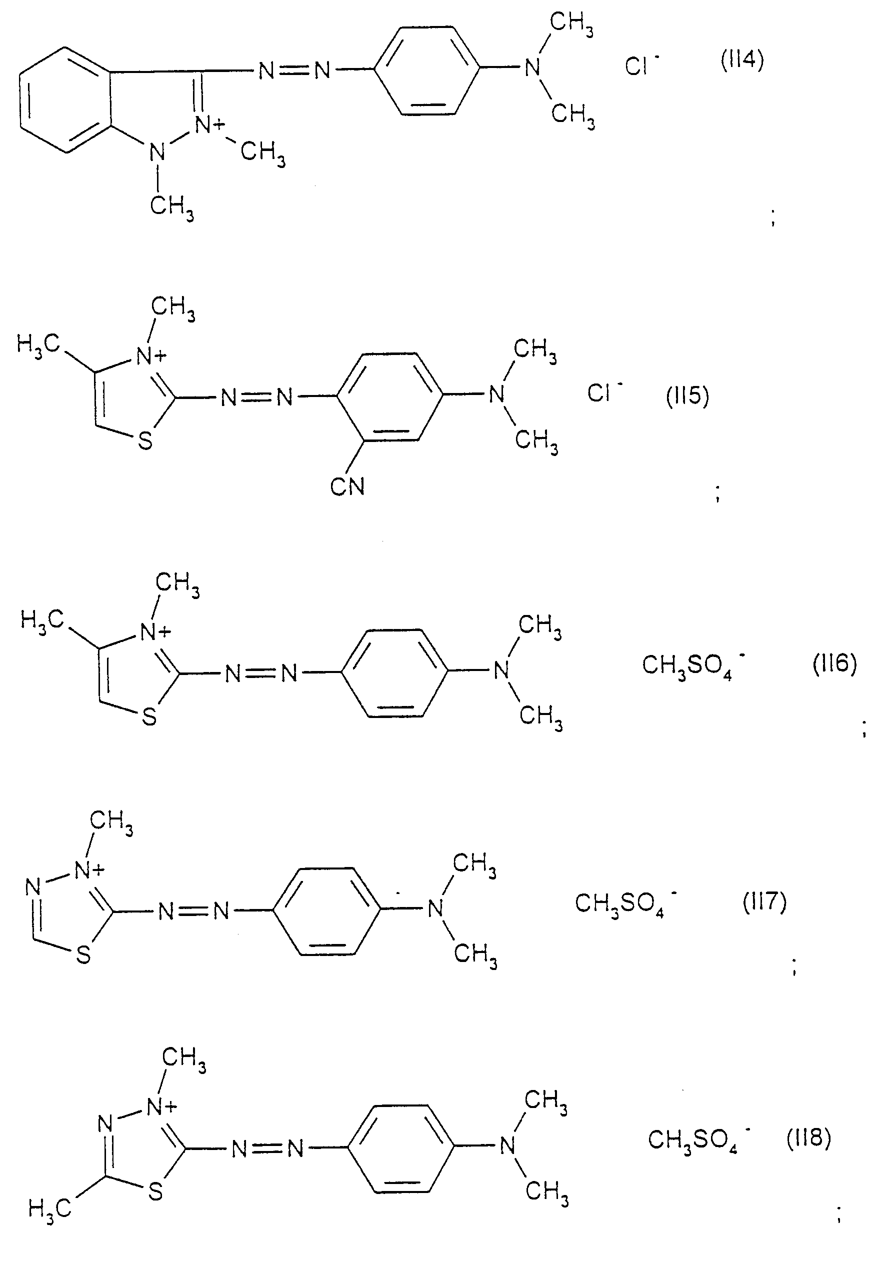

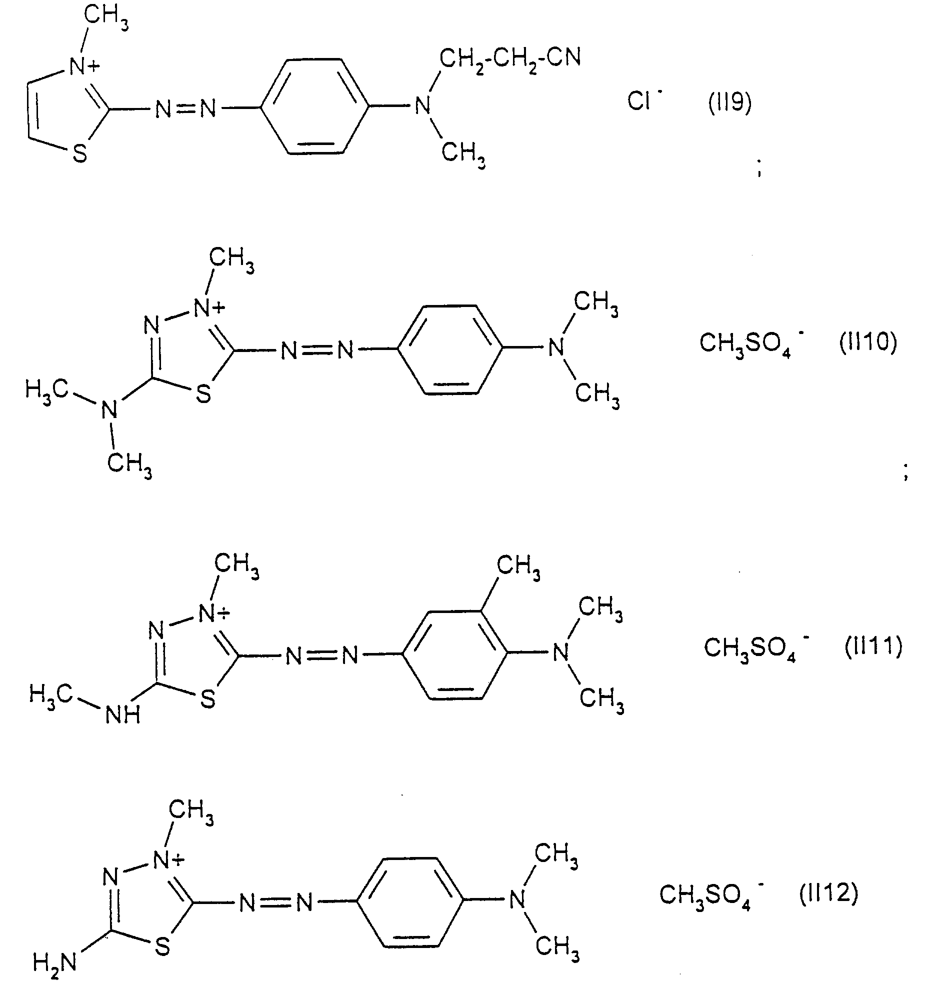

14. Zusammensetzung nach einem der Ansprüche 1 bis 11, dadurch gekennzeichnet, daß die kationischen Direktfarbstoffe der Formel (III) unter den Verbindungen mit den folgenden Strukturen (III1) bis (III18) ausgewählt sind:

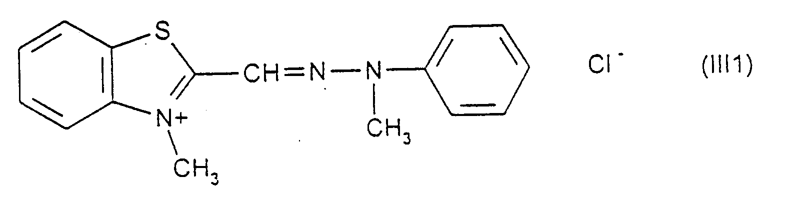

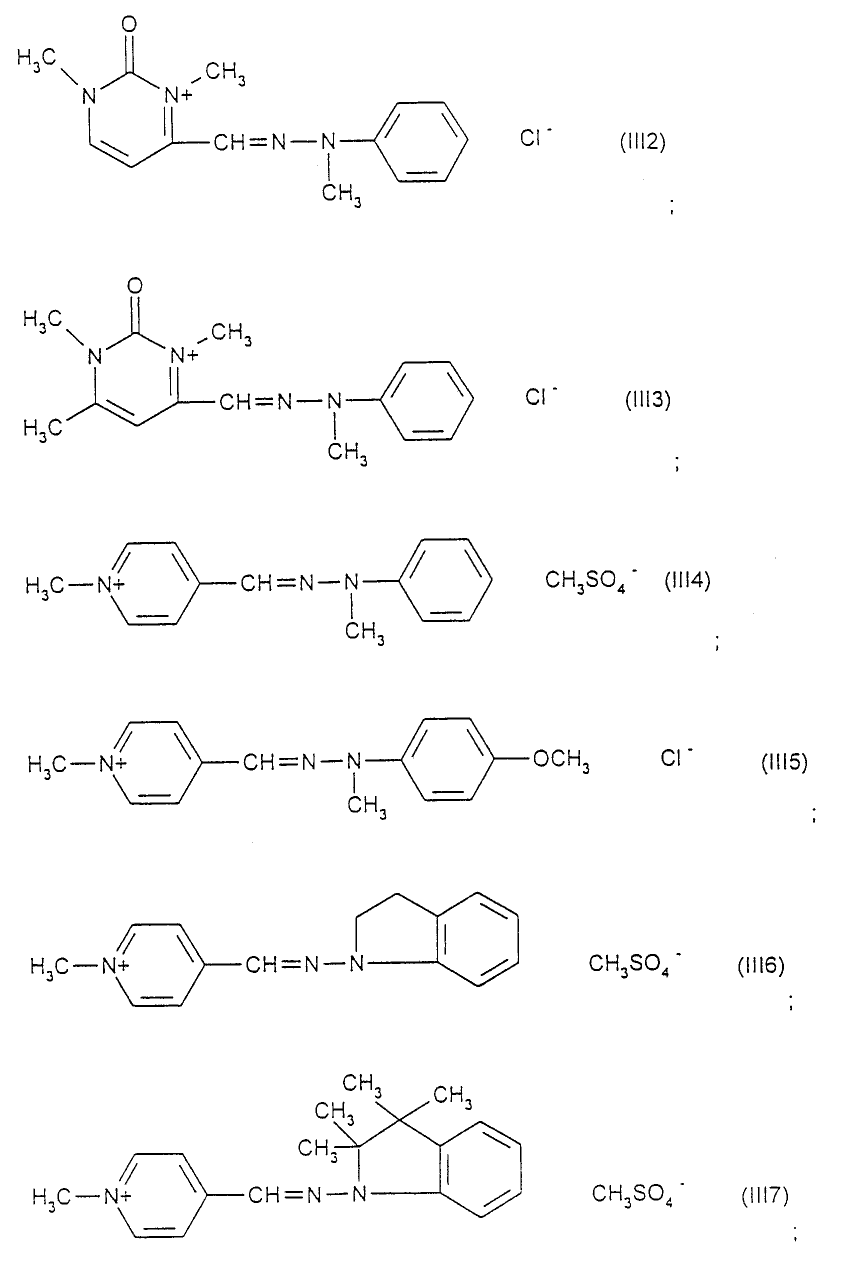

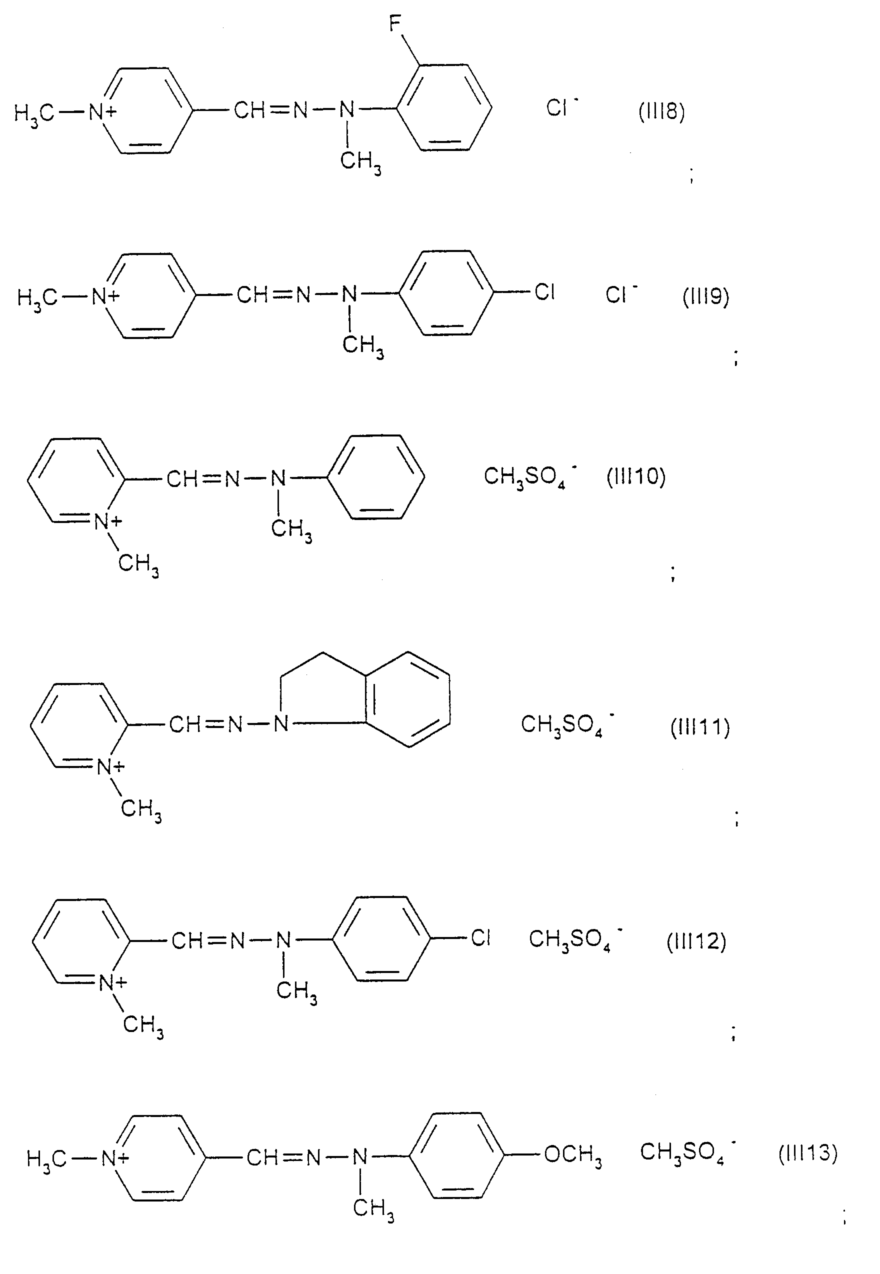

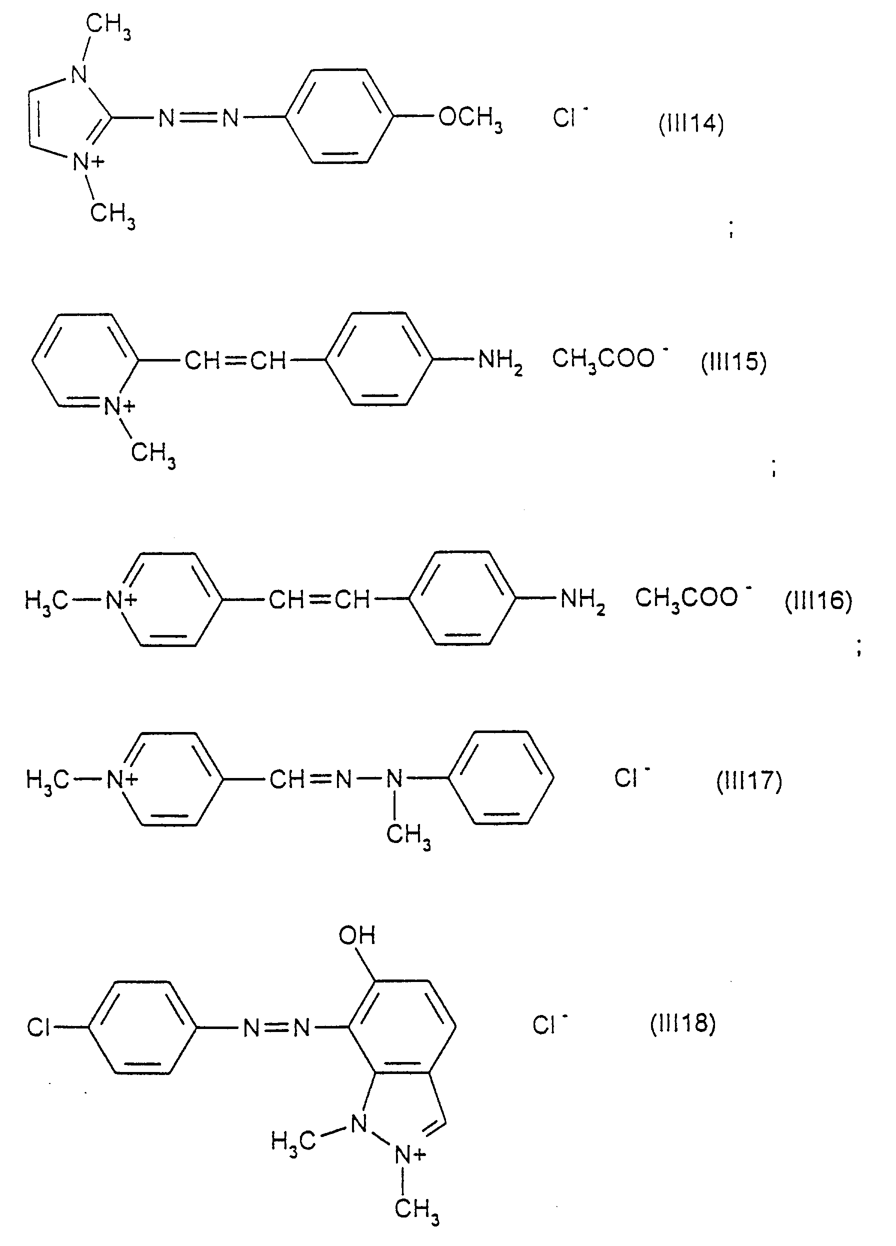

15. Zusammensetzung nach einem der Ansprüche 1 bis 11, dadurch gekennzeichnet, daß die kationischen Direktfarbstoffe der Formel (III') unter den Verbindungen mit den folgenden Strukturen (III'2) und (III'3) 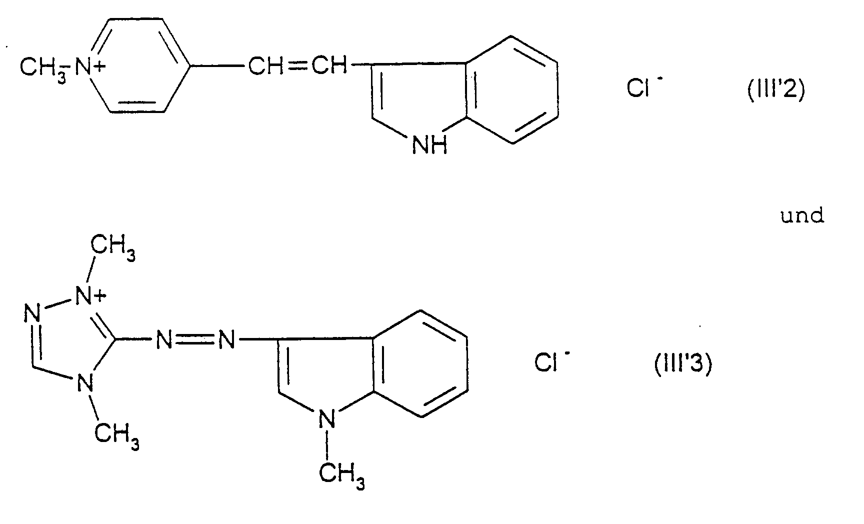 ausgewählt sind.

16. Zusammensetzung nach einem der vorhergehenden Ansprüche, dadurch gekennzeichnet, daß das Oxidationsmittel unter Wasserstoffperoxid, Harnstoffperoxid, Alkalimetallbromaten, Persalzen wie Perboraten und Persulfaten ausgewählt ist.

17. Zusammensetzung nach einem der vorhergehenden Ansprüche, dadurch gekennzeichnet, daß der Anteil der Oxidationsbase(n) 0,0001 bis 10 Gew.-% des Gesamtgewichts der gebrauchsfertigen Färbemittelzusammensetzung beträgt.

18. Zusammensetzung nach einem der vorhergehenden Ansprüche, dadurch gekennzeichnet, daß der Anteil des oder der erfindungsgemäßen kationischen Direktfarbstoffe der Formeln (I) und/oder (II) und/oder (III) und/oder (III') 0,001 bis 10 Gew.-% des Gesamtgewichts der gebrauchsfertigen Färbemittelzusammensetzung beträgt.

19. Zusammensetzung nach einem der vorhergehenden Ansprüche, dadurch gekennzeichnet, daß sie einen pH-Wert im Bereich von 5 bis 12 aufweist.

20. Zusammensetzung nach einem der vorhergehenden Ansprüche, dadurch gekennzeichnet, daß das für die Färbung geeignete Medium aus Wasser oder einem Gemisch aus Wasser und mindestens einem organischen Lösungsmittel besteht.

21. Verfahren zum Färben von Keratinfasern und insbesondere von menschlichen Keratinfasern wie den Haaren, dadurch gekennzeichnet, daß mindestens eine wie in einem der Ansprüche 1 bis 20 definierte gebrauchsfertige Färbemittelzusammensetzung aufgetragen wird.

22. Verfahren nach Anspruch 21, dadurch gekennzeichnet, daß es einen vorbereitenden Schritt umfaßt, der darin besteht, eine Zusammensetzung (A), die in einem für die Färbung geeigneten Medium mindestens eine Oxidationsbase und mindestens einen kationischen Direktfarbstoff enthält, der unter den Verbindungen der wie weiter oben definierten Formeln (I), (II), (III) und (III') ausgewählt wird, und eine Zusammensetzung (B), die in einem für die Färbung geeigneten Medium mindestens ein wie weiter oben definiertes Oxidationsmittel enthält, zu lagern und daß die Zusammensetzungen zum Zeitpunkt ihrer Verwendung vermischt werden, bevor dieses Gemisch auf die Keratinfasern aufgetragen wird.

23. Verfahren nach Anspruch 21, dadurch gekennzeichnet, daß es einen vorbereitenden Schritt umfaßt, der darin besteht, eine eine Zusammensetzung (A), die in einem für die Färbung geeigneten Medium mindestens eine Oxidationsbase enthält, eine Zusammensetzung (A'), die in einem für die Färbung geeigneten Medium mindestens einen kationischen Direktfarbstoff enthält, der unter den Verbindungen der wie weiter oben definierten Formeln (I), (II), (III) und (III') ausgewählt wird, und eine Zusammensetzung (B), die in einem für die Färbung geeigneten Medium mindestens ein wie weiter oben definiertes Oxidationsmittel enthält, zu lagern, und daß diese Zusammensetzungen zum Zeltpunkt ihrer Verwendung vermischt werden, bevor dieses Gemisch auf die Keratinfasern aufgetragen wird.

24. Verfahren nach Anspruch 23, dadurch gekennzeichnet, daß die Zusammensetzung (A') in Form eines Pulvers vorliegt

25. Vorrichtung mit mehreren Abteilungen oder "Kit" zum Färben, dadurch gekennzeichnet, daß eine erste Abteilung die wie in Anspruch 22 definierte Zusammensetzung (A) und eine zweite Abteilung eine oxidierende Zusammensetzung (B) enthält.

26. Vorrichtung mit mehreren Abteilungen oder "Kit" zum Färben, dadurch gekennzeichnet, daß eine erste Abteilung eine wie in Anspruch 23 definierte Zusammensetzung (A), eine zweite Abteilung eine wie in Anspruch 23 oder 24 definierte Zusammensetzung (A') und eine dritte Abteilung eine oxidierende Zusammensetzung (B) enthält.
